# EUROPEAN PATENT SPECIFICATION

(11) **EP 3 785 727 B1**
(45) Date of publication and mention of the grant of the patent: **04.11.2026**
(21) Application number: 20186437.8
(22) Date of filing: 28.09.2018
(51) Int. Cl.: A61K 38/39, C07K 14/78

(54) **RECOMBINANT COLLAGEN AND ELASTIN MOLECULES AND USES THEREOF**
REKOMBINANTE KOLLAGEN- UND ELASTINMOLEKÜLE UND VERWENDUNGEN DAVON
COLLAGÈNE RECOMBINANT ET MOLÉCULES D'ÉLASTINE, AINSI QUE LEURS UTILISATIONS

(30) Priority: 28.09.2017 US 201762564964 P; 13.04.2018 US 201862657591 P; 27.09.2018 US 201816144914
(43) Date of publication of application: 03.03.2021
(62) Document number(s) of the earlier application(s) in accordance with Art. 76 EPC: 18796161.0 / 3 687 564
(73) Proprietor: Geltor, Inc., San Leandro, CA 94577 (US)
(72) Inventor: OUZOUNOV, Nikolay, San Leandro, CA California 94577 (US); LORESTANI, Alexander, San Leandro, CA California 94577 (US); BHATIA, Monica, San Leandro, CA California 94577 (US)
(74) Representative: Mewburn Ellis LLP

(56) References cited:
- WO-A2-2015/012682
- ANONYMOUS: "colF1 - Fibrillar collagen - Podocoryna carnea (Hydrozoan) - colF1 gene & protein", 1 January 1998 (1998-01-01), XP055541622, Retrieved from the Internet <URL:https://www.uniprot.org/uniprot/O76966#entry_information> [retrieved on 20190114]

## Description

This application claims priority to U.S. Patent Application No. 16/144,914, filed September 27, 2018, titled, "RECOMBINANT COLLAGEN AND ELASTIN MOLECULES AND USES THEREOF," which claims the benefit under 35 USC 119(e) of U.S. Provisional Patent Application 62/564,964, filed September 28, 2017, and U.S. Provisional Patent Application 62/657,591, filed April 13, 2018, both titled, "RECOMBINANT COLLAGEN AND ELASTIN MOLECULES AND USES THEREOF".

### FIELD

The present disclosure relates to non-naturally occurring truncated collagen molecules and uses thereof.

### BACKGROUND

Collagens and similar proteins are the most abundant proteins in the biosphere. Collagens and elastins are structural proteins found in the skin, connective tissue and bone of animals and other tissues. In humans, the amount of collagen present in the body is approximately one third of the total proteins and accounts for about three fourths of the dry weight of skin. Elastin is a highly elastic protein found in connective tissue and other types of tissue.

The structure of collagen is a triple helix in which three polypeptide strands together form a helical coil. The individual polypeptide strands are composed of repeating triplet amino acid sequences designated as GLY-X-Y. X and Y can be any amino acid and the third amino acid is glycine. The amino acids proline and hydroxyproline are found in high concentrations in collagen. The most common triplet is proline-hydroxyproline-glycine (Gly-Pro-Hyp) accounting for approximately 10.5% of the triplets in collagen.

Gelatin is a product obtained by partial hydrolysis of collagen. Typically, gelatin is produced by acid hydrolysis, alkaline hydrolysis, and enzymatic hydrolysis or by exposing collagen to heat in an aqueous solution (e.g., boiling the bones and skins of animal, boiling fish scales, etc.).

Gelatin is used in many products including cosmetics, foods, pharmaceuticals, medical devices, photographic films, adhesives, binders and many others. The physical and chemical properties of gelatin are tuned to the particular application. These physical/chemical properties include gel strength, melting point temperature, viscosity, color, turbidity, pH, isoelectric point and others.

Elastin is an elastic protein that is crucial for the proper functioning of arteries, lung, tendons, ligament, skin and other tissue. Elastin provides the tissues with the ability to stretch and return to its original shape. The protein tropoelastin is the building block of elastin. In contrast to collagen that include a family of genes, there is one tropoelastin gene in humans. When expressed, the single elastin gene is spliced to produce different forms of the tropoelastin protein. Many tropoelastin molecules associate together to form elastin.

L-form bacteria, or L-forms, are bacterial strains derived from parent species (N-forms) that are able to grow as cell wall-deficient (spheroplast type) or as cell wall-less (protoplast type) cells. See, Madoff S (Ed): The Bacterial L-Forms. New York: Marcel Dekker Inc., 1986; Mattmann LH (Ed): Cell Wall Deficient Forms. Boca Raton: CRC Press; 1993; and Gumpert J, Taubeneck U: Characteristic properties and biological significance of stable protoplast type L-forms. In Protoplasts, Lecture Proceedings of the 6th International Protoplast Symposium: Basel. Experientia 1983, 46(suppl):227-241.

Protoplast type L-forms have been cultivated in the cell wall-less state and represent genetically stable mutants showing extreme pleiotropic changes, including the inability to form cell walls, capsules, flagella, pili, spores and mesosomes, altered colony and cell morphology, qualitative and quantitative changes in the lipid and protein components of the cytoplasmic membrane, the absence of extracellular proteolytic activities, resistance against bacteriophages and the incapability to propagate outside laboratory conditions. See, Gumpert and Taubeneck (supra); and Hoischen et al., Lipid and fatty acid composition of cytoplasmic membranes from Streptomyces hygroscopic and its stable protoplast type L-form. J Bacteriol 1997, 179:3430-3436.

### SUMMARY

In one aspect, the invention provides recombinant collagen polypeptides, compositions, and recombinant host cells as defined by the claims.

In another aspect, the non-naturally occurring collagen further comprises amino acid sequences including a secretion tag, a histidine tag, a green fluorescent protein tag, a protease cleavage site, and/or a Beta-lactamase. In one aspect, the secretion tag is DsbA, PelB, OmpA, TolB, MalE, 1pp, TorA, or Hy1A, or a hybrid secretion tag that comprises a portion of one secretion tag fused to a portion of a second secretion tag. An exemplary secretion tag is DsbA.

The compositions can further comprise at least one additional ingredient comprising a topical carrier or a preservative.

Compositions comprising non-naturally occurring collagen are in one aspect topical compositions for applying to skin. The topical compositions are used for decreasing skin damage or promoting the repair of damaged skin.

In another aspect the application of the composition increases the synthesis of procollagen by the fibroblast cells of the subject's skin. In another aspect the topical application of the composition protects skin or keratinocytes against UV damage. In yet another embodiment, thymine-thymine (TT) dimer formation is decreased by the collagens or elastins disclosed herein.

The collagen or elastin as provided increases the viability of keratinocytes and/or fibroblasts is increased upon exposure to UV radiation, urban dust or other damaging stimuli.

Host cells that express the polynucleotides of the invention are disclosed. Host cells can be any host cell including bacterial cells, yeast cells, fungal cells, insect cells, mammalian cells, plant cells and any other cells used to express exogenous polynucleotides.

Bacterial host cells in which the cells have been modified to inhibit cell division and the periplasmic space is increased are provided. An exemplary host cell is *E. coli.*

### BRIEF DESCRIPTION OF THE DRAWINGS

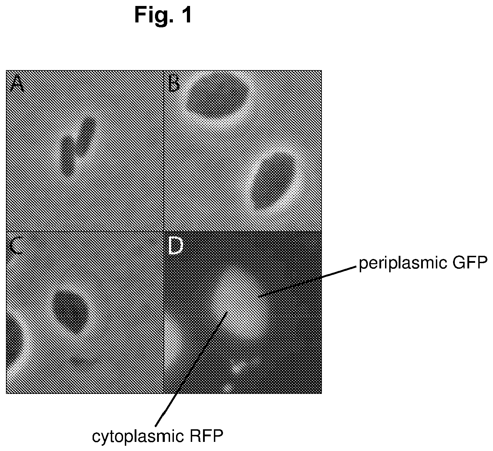

Fig. 1 depicts the physiological state difference between switched and unswitched cells. A) Unswitched Escherichia coli cells. B) Same Escherichia coli population as figure A but has undergone the physiological switch. C) Phase contrast of switched Escherichia coli cell containing cytoplasmic RFP and periplasmic GFP. D) Fluorescent imaging of cell in figure C illustrates targeted protein localization.

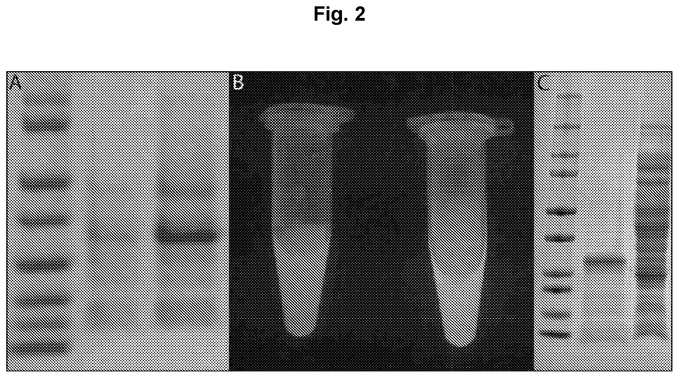

Fig. 2 depicts enhanced protein production in switched cells. A-B) Target protein for T7 inducible protein production is periplasmic expressed GFP, produced in Escherichia coli BL21. The same population of cells was used and induced at OD 1.1. A) Protein ladder (lane 1), IPTG induced protein production (lane 2), IPTG induced protein production with physiological switch (lane 3). B) Two vials of the cell GFP induced cultures with IPTG only on left and IPTG+Switch on right. C) Expression of a 22 kDa collagen using switched cells showing protein ladder (lane 1), supernatant after protein production (lane 2), cell pellet (lane 3).

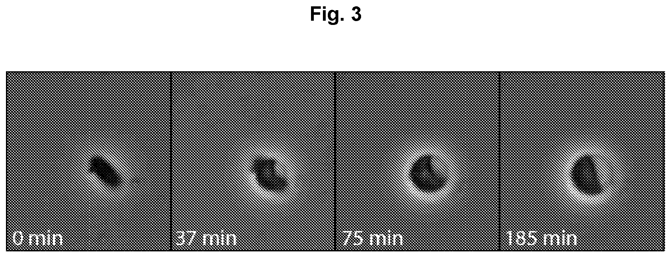

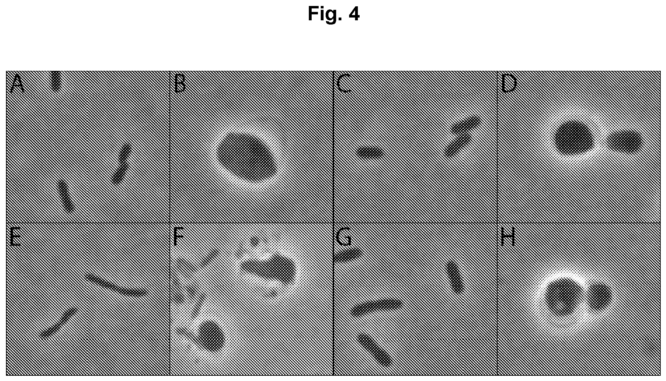

Fig. 3 depicts a time-lapse of Escherichia coli cell switching over time. Fig. 4 illustrates other organisms undergoing the physiological switch. A) *Agrobacterium tumefaciens* normal physiology. *B)Agrobacterium tumefaciens* switched physiology. C) *Pseudomonas aeruginosa* PAO1 normal physiology. D) *Pseudomonas aeruginosa* PAO1 switched physiology. E) *Brevundimonas diminuta* normal physiology. F) *Brevundimonas diminuta* switched physiology. G) *Agrobacterium tumefaciens* normal physiology. H)*Agrobacterium tumefaciens* switched physiology.
Fig. 4 illustrates other organisms undergoing the physiological switch. A) *Agrobacterium tumefaciens* normal physiology. B)*Agrobacterium tumefaciens* switched physiology. C) *Pseudomonas aeruginosa* PAO1 normal physiology. D) *Pseudomonas aeruginosa* PAO1 switched physiology. E) *Brevundimonas diminuta* normal physiology. F) *Brevundimonas diminuta* switched physiology. G) *Agrobacterium tumefaciens* normal physiology. H)*Agrobacterium tumefaciens* switched physiology.

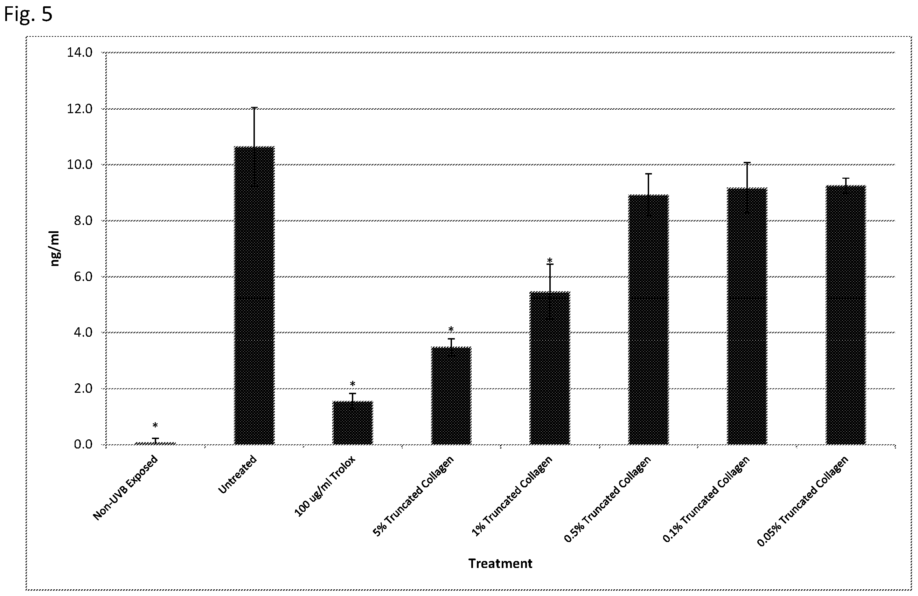

Fig. 5 illustrates the reduction in TT dimer formation by treatment of human keratinocytes with truncated collagen.

### DESCRIPTION

In the following description, certain specific details are set forth in order to provide a thorough understanding of various embodiments of the disclosure. However, one skilled in the art will understand that the disclosure may be practiced without these details.

As used herein the term "about" refers to ± 10 %.

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this disclosure may be presented in a range format. It should be understood that the description in range format is merely for convenience and brevity and should not be construed as an inflexible limitation on the scope of the disclosure. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals there between.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

The term "collagen" or "collagen-like" as used herein refers to a monomeric polypeptide that can associate with one or more collagen or collagen-like polypeptides to form a quaternary structure. Collagen can be treated with acid, base or heat to prepare gelatin. The quaternary structure of natural collagen is a triple helix typically composed of three polypeptides. Of the three polypeptides that form natural collagen, two are usually identical and are designated as the alpha chain. The third polypeptide is designated as the beta chain. Thus a typical natural collagen can be designated as AAB, wherein the collagen is composed of two alpha ("A") strands and one beta ("B") strand. The term "procollagen" as used herein refers to polypeptides produced by cells that can be processed to naturally occurring collagen.

The terms "elastin" as used herein refers to a polypeptide that is elastic and functions to stretch or contract and return to its original shape. Elastin is found naturally in connective tissue.

The term "expression vector" or "vector" as used herein refers to a nucleic acid assembly which is capable of directing the expression of the exogenous gene. The expression vector may include a promoter which is operably linked to the exogenous gene, restriction endonuclease sites, nucleic acids that encode one or more selection markers, and other nucleic acids useful in the practice of recombinant technologies.

The term "fibroblast" as used herein refers to a cell that synthesizes procollagen and other structural proteins. Fibroblasts are widely distributed in the body and found in skin, connective tissue and other tissues.

The term "fluorescent protein" is a protein that is commonly used in genetic engineering technologies used as a reporter of expression of an exogenous polynucleotide. The protein when exposed to ultraviolet or blue light fluoresces and emits a bright visible light. Proteins that emit green light is green fluorescent protein (GFP) and proteins that emit red light is red fluorescent protein (RFP).

The term "gelatin" as used herein refers to collagen that has been further processed by exposure to acid, base or heat. While not wishing to be bound by theory or mechanism, treatment of collagen with acid, base or heat is thought to denature the collagen polypeptides. Aqueous denatured collagen solutions form reversible gels used in foods, cosmetics, pharmaceuticals, industrial products, medical products, laboratory culture growth media, and many other applications.

The term "gene" as used herein refers to a polynucleotide that encodes a specific protein, and which may refer to the coding region alone or may include regulatory sequences preceding (5' non-coding sequences) and following (3' non-coding sequences) the coding sequence.

The term "histidine tag" is a 2-30 contiguous series of histidine residues on a recombinant polypeptide.

The term "host cell" is a cell that is engineered to express an introduced exogenous polynucleotide.

The term" keratinocyte" is a cell that produces keratins found in the epidermal layer of the skin.

The term "lactamase" as used herein refer to enzymes that hydrolyze antibiotics that contain a lactam (cyclic amide) moiety. "Beta-lactamase" or "β-lactamase" are enzymes that hydrolyze antibiotics that contain a β-lactam moiety.

The term "non-naturally occurring" as used herein refers to collagen or elastin that is not normally found in nature. The non-naturally occurring collagen or elastin are recombinantly prepared. The non-naturally occurring collagen or elastin is a recombinant collagen or recombinant elastin. The non-naturally occurring collagen is in one embodiment a truncated collagen. Other non-naturally occurring collagen polypeptides include chimeric collagens. A chimeric collagen is a polypeptide wherein one portion of a collagen polypeptide is contiguous with a portion of a second collagen polypeptide. For example, a collagen molecule comprising a portion of a jellyfish collagen contiguous with a portion of a human collagen is a chimeric collagen. In another embodiment, the non-naturally occurring collagen comprises a fusion polypeptide that includes additional amino acids such as a secretion tag, histidine tag, green fluorescent protein, protease cleavage site, GEK repeats, GDK repeats, and/or beta-lactamase. The non-naturally occurring elastin in one embodiment a truncated elastin. Other non-naturally occurring elastin polypeptides include chimeric elastins. A chimeric elastin is a polypeptide wherein one portion of an elastin polypeptide is contiguous with a portion of a second elastin polypeptide. For example, a collagen molecule comprising a portion of a jellyfish elastin contiguous with a portion of a human elastin is a chimeric elastin. In another embodiment, the non-naturally occurring elastin comprises a fusion polypeptide that includes additional amino acids such as a secretion tag, histidine tag, green fluorescent protein, protease cleavage site and/or beta-lactamase. The chimeric gelatin or the chimeric elastin can comprise additional amino acids such as a secretion tag, histidine tag, green fluorescent protein, protease cleavage site, GEK repeats, GDK repeats, and/or beta-lactamase.

The term "protease cleavage site" is an amino acid sequence that is cleaved by a specific protease.

The term "secretion tag" or "signal peptide" refers to an amino acid sequence that recruits the host cell's cellular machinery to transport an expressed protein to a particular lo cation or cellular organelle of the host cell.

The term "truncated collagen" refers to a monomeric polypeptide that is smaller t han a full-length collagen wherein one or more portions of the full-length collagen is not p resent. Collagen polypeptides are truncated at the C-terminal end, the N-terminal end, or t runcated by removal of internal portion(s) of the full-length collagen polypeptide.

The term "truncated elastin" refers to a monomeric polypeptide that is smaller than a full-length elastin wherein one or more portions of the full-length elastin is not present. Elastin polypeptides are truncated at the C-terminal end, the N-terminal end, or truncated by removal of internal portion(s) of the full-length elastin polypeptide.

In co-owned application PCT/US17/24857, an expression system that uses modified bacterial cells (switched cells) in which cell division is inhibited and growth of the periplasmic space is greatly enhanced was disclosed. In this expression system, the expressed proteins are targeted to the periplasmic space. Recombinant protein production in these switched cells is dramatically increased compared with that in non-switched cells. Structurally, the cells comprise both inner and outer membranes but lack a functional peptidoglycan cell wall, while the cell shape is spherical and increases in volume over time. Notably, while the periplasmic space normally comprises only 10-20% of the total cell volume, the periplasmic compartment of the switched state described herein can comprise more than 20%, 30%, 40% or 50% and up to 60%, 70%, 80% or 90% of the total cell volume.

The modified bacterial cells of PCT/US17/24857 are derived from Gram-negative bacteria, e.g. selected from: gammaproteobacteria and alphaproteobacteria.
In some embodiments, the bacterium is selected from: *Escherichia coli, Vibrio natriegens, Pseudomonas fluorescens, Caulobacter crescentus, Agrobacterium tumefaciens,* and *Brevundimonas diminuta.* In specific embodiments, the bacterium is *Escherichia coli,* e.g. strain BL21(DE3).

In another aspect, the host bacterial cells have an enlarged periplasmic space in a culture medium comprising a magnesium salt, wherein the concentration of magnesium ions in the medium is at least about 3,4, 5 or 6 mM. In further embodiments, the concentration of magnesium ions in the medium is at least about 7, 8, 9 or 10 mM. In some embodiments, the concentration of magnesium ions in the medium is between about 5 mM and 25 mM, between about 6 mM and/or about 20, 15 or 10 mM. In some embodiments, the magnesium salt is selected from: magnesium sulfate and magnesium chloride.

In other embodiments, the culture medium further comprises an osmotic stabilizer, including, e.g. sugars (e.g., arabinose, glucose, sucrose, glycerol, sorbitol, mannitol, fructose, galactose, saccharose, maltotrioseerythritol, ribitol, pentaerythritol, arabitol, galactitol, xylitol, iditol, maltotriose, and the like), betaines (e.g., trimethylglycine), proline, sodium chloride, wherein the concentration of the osmotic stabilizer in the medium is at least about 4%, 5%, 6%, or 7% (w/v). In further embodiments, the concentration of osmotic stabilizer is at least about 8%, 9%, or 10% (w/v). In some embodiments, the concentration of the osmotic stabilizer in the medium is between about 5% to about 20% (w/v).

In some embodiments, the cell culture may further comprise ammonium chloride, ammonium sulfate, calcium chloride, amino acids, iron(II) sulfate, magnesium sulfate, peptone, potassium phosphate, sodium chloride, sodium phosphate, and yeast extract.

The host bacterial cell may be cultured continuously or discontinuously; in a batch process, a fed-batch process or a repeated fed-batch process.

In some embodiments, the antibiotic is selected from: β-lactam antibiotics (e.g. penicillins, cephalosporins, carbapenems, and monobactams), phosphonic acid antibiotics, polypeptide antibiotics, and glycopeptide antibiotics. In particular embodiments, the antibiotic is selected from alafosfalin, amoxicillin, ampicillin, aztreonam, bacitracin, carbenicillin, cefamandole, cefotaxime, cefsulodin, cephalothin, fosmidomycin, methicillin, nafcillin, oxacillin, penicillin g, penicillin v, fosfomycin, primaxin, and vancomycin.

Without being bound by theory, the cell morphology that promotes recombinant protein production and inhibits cell division appears to be driven by the removal of the cell wall under the media conditions stated above. In some embodiments, the methods for removal/inhibition of cell wall synthesis can be through the use of antibiotics that inhibit peptidoglycan synthesis (such as ampicillin, carbenicillin, penicillins or fosfomycin), or other methods known in the art.

When having an appropriate periplasmic targeting signal sequence, recombinantly produced polypeptides can be secreted into the periplasmic space of bacterial cells. Joly, J.C. and Laird, M.W., in The Periplasm ed. Ehrmann, M., ASM Press, Washington D.C., (2007) 345-360. In the chemically oxidizing environment of the periplasm the formation of disulfide bonds and thereby the functionally correct folding of polypeptides is favored.

In general, the signal sequence may be a component of the expression vector, or it may be a part of the exogenous gene that is inserted into the vector. The signal sequence selected should be one that is recognized and processed (i.e., cleaved by a signal peptidase) by the host cell. For bacterial host cells that do not recognize and process the native signal sequence of the exogenous gene, the signal sequence is substituted by any commonly known bacterial signal sequence. In some embodiments, recombinantly produced polypeptides can be targeted to the periplasmic space using the DsbA signal sequence. Dinh and Bernhardt, JBacteriol, Sept. 2011, 4984-4987.

In one aspect, t a non-naturally occurring collagen or elastin is produced by a host cell is provided. The non-naturally occurring collagen or elastin is jellyfish collagen or elastin, t, human collagen or elastin, or *Chondrosia reniform is* (kidney sponge) collagen or elastin, or *Rhincodon typus* collagen or elastin. The non-naturally occurring collagen or elastin is a truncated collagen. The truncation is an internal truncation, a truncation at the N-terminal portion of the collagen or elastin, or a truncation at the C-terminal portion of the collagen or elastin. The collagen or elastin is truncated by a truncation of between 50 amino acids and 1000 amino acids, between, 50 amino acids and 950 amino acids, between 50 amino acids and 900 amino acids, between 50 amino acids and 850 amino acids, between 50 amino acids and 800 amino acids, between 50 amino acids and 850 amino acids, between 50 amino acids and 800 amino acids, between 50 amino acids and 750 amino acids, between 50 amino acids and 700 amino acids, between 50 amino acids and 650 amino acids, between 50 amino acids and 600 amino acids, between 50 amino acids and 650 amino acids, between 50 amino acids and 500 amino acids, between 50 amino acids and 450 amino acids, between 50 amino acids and 400 amino acids, between 50 amino acids and 350 amino acids, between 50 amino acids and 300 amino acids, between 50 amino acids and 250 amino acids, between 50 amino acids and 200 amino acids, between 50 amino acids and 150 amino acids, or between 50 amino acids and 100 amino acids. In another embodiment, the collagen or elastin is truncated by 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, ,510, 520, 530, 540, 550, 560, 570, 580, 590, 600, 650, 700, 750, 800, 850, 900, 950, or 1000 amino acids. The non-naturally occurring collagen or elastin are encoded by a portion of a polynucleotide sequence or the entire polynucleotide sequence disclosed herein.

The non-naturally occurring collagen or elastin further comprises amino acid sequences including a secretion tag. The secretion tag directs the collagen or elastin to the periplasmic space of the host cell. In particular embodiments, the signal peptide is derived from DsbA, PelB, OmpA, TolB, MalE, lpp, TorA, or Hy1A, or a hybrid secretion tag that comprises a portion of one secretion tag fused to a portion of a second secretion tag. In one aspect the secretion tag is attached to the non-naturally occurring collagen or elastin. In another aspect the secretion tag is cleaved from the non-naturally occurring collagen or elastin.

The non-naturally occurring collagen or the non-naturally occurring elastin o further comprises a histidine tag. The histidine tag or polyhistidine tag is a sequence of 2 to 20 histidine residues that are attached to the collagen or elastin. The histidine tag comprises 2 to 20 histidine residues, 5 to 15 histidine residues, 5 to 18 histidine residues, 5 to 16 histidine residues, 5 to 15 histidine residues, 5 to 14 histidine residues, 5 to 13 histidine residues, 5 to 12 histidine residues, 5 to 11, 5 to 10 histidine residues,, 6 to 12 histidine residues, 6 to 11 histidine residues, or 7 to 10 histidine residues. The histidine tags are useful in purification of proteins by chromatographic methods utilizing nickel based chromatographic media. Exemplary fluorescent proteins include green fluorescent protein (GFP) or red fluorescent protein (RFP). Fluorescent proteins are well known in the art. In one embodiment the non-naturally occurring collagen or the on-naturally occurring elastin comprises a GFP and/or RFP. In one embodiment a superfolder GFP is fused to the on-naturally occurring collagen or elastin. The superfolder GFP is a GFP that folds properly even when fused to a poorly folded polypeptide. In one aspect the histidine tag is attached to the non-naturally occurring collagen or elastin. In another aspect the histidine tag is cleaved from the non-naturally occurring collagen or elastin.

The non-naturally occurring collagen or non-naturally occurring elastin further comprises a protease cleavage site. The protease cleavage site is useful to cleave the recombinantly produced collagen or elastin to remove portions of the polypeptide. The portions of the polypeptide that may be removed include the secretion tag, the histidine tag, the fluorescent protein tag and/or the Beta-lactamase. The proteases comprise endoproteases, exoproteases, serine proteases, cysteine proteases, threonine proteases, aspartic proteases, glutamic proteases, and metalloproteases. Exemplary protease cleavage sites include amino acids that are cleaved by Thrombin, TEV protease, Factor Xa, Enteropeptidase, and Rhinovirus 3C Protease. In one aspect the cleavage tag is attached to the non-naturally occurring collagen or elastin. In another aspect the cleavage tag is removed by an appropriate protease from the non-naturally occurring collagen or elastin.

The non-naturally occurring collagen or non-naturally occurring elastin further comprises an enzyme that is a Beta-lactamase. The beta-lactamase is useful as a selection marker. In one aspect the beta-lactamase is attached to the non-naturally occurring collagen or elastin. In another aspect the beta-lactamase is cleaved from the non-naturally occurring collagen or elastin.

The non-naturally occurring collagen or non-naturally occurring elastin further comprises GEK amino acid trimer repeats and/or GDK amino acid trimer repeats. The GEK and the GDK trimer repeats facilitate the gelling of the collagen and/or the gelatin. In one embodiment, the non-naturally occurring collagen or the non-naturally occurring elastin comprises 2-50 GEK and/or 2-50 GDK trimer repeats, 2-40 GEK and/or 2-40 GDK trimer repeats, 2-30 GEK and/or 2-30 GDK trimer repeats, 2-20 GEK and/or 2-20 GDK trimer repeats, 2-15 GEK and/or 2-15 GDK trimer repeats. 2-10 GEK and/or 2-10 GDK trimer repeats, 2-9 GEK and/or 2-9 GDK trimer repeats, 2-8 GEK and/or 2-8 GDK trimer repeats, 2-7 GEK and/or 2-7 GDK trimer repeats, 2-6 GEK and/or 2-6 GDK trimer repeats, 2-5 GEK and/or 2-5 GDK trimer repeats, or 2-4 GEK and/or 2-4 GDK trimer repeats. In one aspect the GEK trimer repeat or the GDK trimer repeat is attached to the non-naturally occurring collagen or elastin. In another aspect the GEK trimer repeat or the GDK trimer repeat is cleaved from the non-naturally occurring collagen or elastin.

Provided herein are compositions that comprise between 0.005% and 30% w/w non-naturally occurring collagen and/ or non-naturally occurring elastin. The composition comprises between 0.005% and 20% w/w non-naturally occurring collagen and/ or non-naturally occurring elastin, between 0.005% and 10% w/w non-naturally occurring collagen and/ or non-naturally occurring elastin, between 0.005% and 5% w/w non-naturally occurring collagen and/ or non-naturally occurring elastin, between 0.005% and 2% w/w non-naturally occurring collagen and/ or non-naturally occurring elastin, between 0.005% and 1% w/w non-naturally occurring collagen and/ or non-naturally occurring elastin, between 0.005% and 0.5% w/w non-naturally occurring collagen and/ or non-naturally occurring elastin, and between 0.005% and 0.2% w/w non-naturally occurring collagen and/ or non-naturally occurring elastin.

The compositions that comprise the between non-naturally occurring collagen and/or non-naturally occurring elastin are personal care products. In some embodiments the compositions are formulated for topical administration. The compositions can contain other cosmetic ingredients suitable for human use. The personal care products are useful for preventing or treating ultraviolet radiation damage to human skin or hair. The personal care products are applied to skin or hair. The compositions include, for example, masks, skin cleaners such as soap, cleansing creams, cleansing lotions, cleansing milks, cleansing pads, facial washes, hair shampoo, hair conditioner and body shampoos.

The compositions that comprise the non-naturally occurring collagen and/ or non-naturally occurring elastin can further comprise at least one additional ingredient comprising a topical carrier or a preservative. The topical carrier comprises a topical carrier selected from the group consisting of liposome, biodegradable microcapsule, lotion, spray, aerosol, dusting powder, biodegradable polymer, mineral oil, triglyceride oil, silicone oil, glycerin, glycerin monostearate, alcohols, emulsifying agents, liquid petroleum, white petrolatum, propylene glycol, polyoxyethylene, polyoxypropylene, wax, sorbitan monostearate, polysorbate, cetyl ester wax, cetearyl alcohol, 2-octyldodecanol, benzyl alcohol, cyclomethicone, cyclopentasiloxane, and water. The preservative comprises a preservative selected from the group consisting of tocopherol, diiodomethyl-p-tolylsulfone, 2-Bromo-2-nitropropane-1,3-diol, cis isomer 1-(3-chloroallyl)-3,5,7-triaza-1-azoniaadamantane chloride, glutaraldehyde, 4,4-dimethyl oxazolidine, 7-Ethylbicyclooxazolidine, methyl paraben, sorbic acid, Germaben II, rosemary extract, and EDTA

Provided are methods of decreasing skin damage, promoting the repair of damaged skin, protecting skin against UV damage, protecting skin cells against the effects of exposure to urban dust.. The method comprises the step of applying the composition comprising the non-naturally occurring collagen and/ or non-naturally occurring elastin to the skin of a subject. Without being bound to a particular theory or mechanism, the collagen and/or the elastin in the composition decrease skin damage by protecting against UV damage, and/or promotes the repair of damaged skin by increasing the viability of cells and/or increasing procollagen synthesis when applied to skin, and/or promotes the viability of skin cells. The collagens and elastins in one aspect decrease the formation of thymine-thymine (TT) dimer formation.

One aspect provides polynucleotides that encode a non-naturally occurring collagen or a non-naturally occurring elastin. The polynucleotides encode collagen or elastin from jellyfish, human, *Chondrosia reniformis* (kidney sponge), or *Rhincodon typus.* The polynucleotides encode for collagen or elastin that is full length or truncated.

Another aspect provides polynucleotides that encode collagen or elastin fusion proteins. The elastin or collagen fusion proteins comprise a secretion tag, a histidine tag, a fluorescent protein tag, a protease cleavage site, a Beta-lactamase along and/or GEK amino acid trimer repeats and/or GDK amino acid trimer repeats together with collagen or elastin.

The polynucleotides are in one aspect vectors used to transform host cells and express the polynucleotides. The polynucleotides further comprise nucleic acids that encode enzymes that permit the host organism to grow in the presence of a selection agent. The selection agents include certain sugars including galactose containing sugars or antibiotics including ampicillin, hygromycin, G418 and others. Enzymes that are used to confer resistance to the selection agent include β-galactosidase or a β-lactamase.

In one aspect host cells that express the polynucleotides of the invention are provided. Host cells can be any host cell including gram negative bacterial cells, gram positive bacterial cells, yeast cells, insect cells, mammalian cells, plant cells or any other cells used to express exogenous polynucleotides. An exemplary gram-negative host cell is *E. coli.*

Bacterial host cells in which the cells have been modified to inhibit cell division and the periplasmic space is increased are taught. As discussed herein and taught in example 1, Beta-lactam antibiotics are useful as a switch to convert wild-type bacterial cells to a modified bacterial cell in which cell replication is inhibited and the periplasmic space is increased. Exemplary Beta-lactam antibiotics including penicillins, cephalosporins, carbapenems, and monobactams.

The switched form of bacteria (L-form) are cultivated in culture media that include certain salts and other nutrients. Salts and media compositions that support the physiological switch physiology that have been tested are M63 salt media, M9 salt media, PYE media, and Luria-Bertani (LB) media. Any necessary supplements besides carbon, nitrogen, and inorganic phosphate sources may also be included at appropriate concentrations introduced alone or as a mixture with another supplement or medium such as a complex nitrogen source. In certain embodiments, the medium further comprises one or more ingredients selected from: ammonium chloride, ammonium sulfate, calcium chloride, casamino acids, iron(II) sulfate, magnesium sulfate, peptone, potassium phosphate, sodium chloride, sodium phosphate, and yeast extract.

Beta-lactamases are enzymes that confer resistance to lactam antibiotics in prokaryotic cells. Typically when Beta-lactamases are expressed in bacterial host cells, the expressed Beta-lactamase protein also includes targeting sequences (secretion tag) that direct the Beta-lactamase protein to the periplasmic space. Beta-lactamases are not functional unless they are transported to the periplasmic space. Beta-lactamase targeted to the periplasmic without the use of an independent secretion tag that targets the enzyme to the periplasmic space are provided. By creating a fusion protein in which a periplasmic secretion tag added to the N-terminus of a protein such as GFP, collagen, or GFP/collagen chimeras, the functionality of the Beta-lactamase lacking a native secretion tag can be used to select for full translation and secretion of the N-terminal fusion proteins. Using this approach, we have used a DsbA-GFP-Collagen-Beta-lactamase fusion to select for truncation products in the target collagens that favor translation and secretion.

Another embodiment provides methods of producing a non-naturally occurring collagen or a non-naturally occurring elastin. The method comprises the steps of inoculating a culture medium with a recombinant host cell comprising polynucleotides that encode the collagen or elastin, cultivating the host cell, and isolating the non-naturally occurring collagen or the non-naturally occurring elastin from the host cell.

A process for fermentative preparation of a protein is provided. The process comprises the steps of:
a) culturing a recombinant Gram-negative bacterial cell in a medium comprising a magnesium salt, wherein the concentration of magnesium ions in the medium is at least about 6 mM, and wherein the bacterial cell comprises an exogenous gene encoding the protein;
b) adding an antibiotic to the medium, wherein the antibiotic inhibits peptidoglycan biogenesis in the bacterial cell; and
c) harvesting the protein from the medium.

The bacteria may be cultured continuously-as described, for example, in WO 05/021772-or discontinuously in a batch process (batch cultivation) or in a fed-batch or repeated fed-batch process for the purpose of producing the target protein. In some embodiments, protein production is conducted on a large-scale. Various large-scale fermentation procedures are available for production of recombinant proteins. Large-scale fermentations have at least 1,000 liters of capacity, preferably about 1,000 to 100,000 liters of capacity. These fermentors use agitator impellers to distribute oxygen and nutrients, especially glucose (the preferred carbon/energy source). Small-scale fermentation refers generally to fermentation in a fermentor that is no more than approximately 20 liters in volumetric capacity.

For accumulation of the target protein, the host cell is cultured under conditions sufficient for accumulation of the target protein. Such conditions include, e.g., temperature, nutrient, and cell-density conditions that permit protein expression and accumulation by the cell. Moreover, such conditions are those under which the cell can perform basic cellular functions of transcription, translation, and passage of proteins from one cellular compartment to another for the secreted proteins, as are known to those skilled in the art.

The bacterial cells are cultured at suitable temperatures. For E. *coli* growth, for example, the typical temperature ranges from about 20 °C to about 39 °C. In one embodiment, the temperature is from about 20° C to about 37° C. In another embodiment, the temperature is at about 30 °C. In one embodiment, the host cells, in the non-switched state or switched state are cultivated at one temperature and switched to a different temperature to induce protein production. The host cells are cultivated first at one temperature to propagate the cells, then to induce protein production the cell are cultivated at a lower temperature. The first temperature is 23°, 24°, 25°, 26°, 27°, 28°, 29°, 30°, 31°, 32°, 33°, 34°, 35°, 36°, or 37° C. The second temperature is 20°, 21°, 22°, 23°, 24°, 25°, 26°, 27°, 28°, 29°, 30°, 31°, 32°, 33°, 34°, 35° or 36° C. The cultivation at the second temperature is conducted between 1 hour and 100 hours, between 5 hours and 90 hours, between 5 hours and 80 hours, between 5 hours and 80 hours, between 5 hours and 70 hours, between 10 hours and 70 hours, between 15 hours and 70 hours, between 15 hours and 65 hours, between 15 hours and 60 hours, between 20 hours and 60 hours, between 20 hours and 55 hours, between 20 hours and 50 hours, between 24 hours and 50 hours, between 24 hours and 48 hours, between 30 hours and 50 hours, between 30 hours and 45 hours, or between 30 hours and 40 hours.

The pH of the culture medium may be any pH from about 5-9, depending mainly on the host organism. For *E. coli,* the pH is from about 6.8 to about 7.4, or about 7.0.

For induction of gene expression, typically the cells are cultured until a certain optical density is achieved, e.g., an OD600 of about 1.1, at which point induction is initiated (e.g., by addition of an inducer, by depletion of a repressor, suppressor, or medium component, etc.) to induce expression of the exogenous gene encoding the target protein. In some embodiments, expression of the exogenous gene is inducible by an inducer selected from, e.g. isopropyl-β-d-1-thiogalactopyranoside, lactose, arabinose, maltose, tetracycline, anhydrotetracycline, vavlycin, xylose, copper, zinc, and the like. The induction of gene expression can also be accomplished by decreasing the dissolved oxygen levels during fermentation. The dissolved oxygen levels of the fermentation during cell propagation is between 10% and 30%. To induce gene expression the dissolved oxygen level is reduced to below 10%, 9%, 8%, 7%, 6%, 5%, 4%, 3%, 2%, 1%, or 0%. In host cells, in either the physiological state or the switched state, protein production can be induced by lowering the temperature of the fermentation as disclosed herein.

After product accumulation, the cells are vortexed and centrifuged in order to induce lysis and release of recombinant proteins. The majority of the proteins is found in the supernatant but any remaining membrane bound proteins can be released using detergents (such as triton X-100).

In a subsequent step, the target protein, as a soluble or insoluble product released from the cellular matrix, is recovered in a manner that minimizes co-recovery of cellular debris with the product. The recovery may be done by any means, but in one embodiment, can comprise histidine tag purification through a nickel column. See, e.g., Purification of Proteins Using Polyhistidine Affinity Tags, Methods Enzymology. 2000 ; 326: 245-254.

### Examples

Recombinant collagen polypeptides according to the claimed invention comprise or consist of an amino acid sequence according to SEQ ID NO: 91. Any sequences disclosed that do not comprise this feature are disclosed for reference purposes only.

### Example 1: Expression System

Materials and methods:

### Strains:

**Tested physiological switch and protein production:**
   *E. coli* BL21(DE3)- From NEB, product #c2527
   *E. coli* K12 NCM3722- From The Coli Genetic Stock Center, CGSC# 12355
**Tested physiological switch:**
   Gammaproteobacteria:
   *Vibrio natriegens* - From ATCC, product #14048
   *Pseudomonas fluorescens* - From ATCC, product # 31948
   *Pseudomonas aeruginosa* PAO1- From ATCC, product # BAA-47
**Alphaproteobacteria:**
   *Caulobacter crescentus* - From ATCC, product #19089
   *Agrobacterium tumefaciens*/*Rhizobium radiobacter -* From ATCC, product #33970
   *Brevundimonas diminuta -* From ATCC, product #13184

### Media compositions:

1 liter **5x m63** salts:
   10 g (NH4)2SO4 - From P212121, product #7783-20-2
   68 g KH2PO4 - From P212121, product #7778-77-0
   2.5 mg FeSO4.7H2O - From Sigma Aldrich, product #F7002
   Bring volume up to 1 liter with milliQ water
   Adjust to pH 7 with KOH (From P212121, product #1310-58-3)
   Autoclave mixture
1 liter **of 1M MgSO4:**
   246.5 g MgSO4 7H2O - From P212121, (Sigma Aldrich, product #10034-99-8)
   Bring volume up to 1 liter with milliQ water.
   Autoclave mixture.
1 liter of switch media 1:
   133.4 mL 5X m63 salts
   10 mL 1M MgSO4
   38.6 g Glucose - From P212121, product #50-99-7
   66.6 g Sucrose - From P212121, product #57-50-1
   8.33 g LB mix - From P212121, product #lb-miller
   Bring volume up to 1 liter with milliQ water.
   Filter sterilize mixture through a 0.22 µM pore vacuum filter (Sigma Aldrich, product #CLS430517).
1 liter of switch media 2:
   133.4 mL 5X m63 salts
   10 mL 1M MgSO4
   38.6 g Glucose - From P212121, product #50-99-7
   66.6 g Sucrose - From P212121, product #57-50-1
   10 g Yeast Extract - From FisherSci.com, product #J60287A1
   Bring volume up to 1 liter with milliQ water.
   Filter sterilize mixture through a 0.22 µM pore vacuum filter (Sigma Aldrich, product #CLS430517).
For Bioreactor growth:
   5 liter of bioreactor media MGZ12:
      1) Autoclave 1L of Glucose at concentration of 500g/L in DI water. (VWR, product #97061-170).
      2) Autoclave 1L of Sucrose at concentration of 500g/L in DI water. (Geneseesci.com, product #62-112).
      3) Autoclave in 3946mL of DI water:
   20 g (NH4)2HPO4. (VWR, product # 97061-932).
   66.5 g KH2PO4. (VWR, product # 97062-348).
   22.5 g H3C6H5O7. (VWR, product #BDH9228-2.5KG).
   2.95 g MgSO4.7H2O. (VWR, product # 97062-134).
   10 mL Trace Metals (Teknova), 1000x. (Teknova, product #T1001).
   After autoclaving add 400 mL of (1) to (3), 65 mL of 10M NaOH (VWR, product #97064-480) to (3), and 666 mL of (2) to (3).
   A feed of 500g/L of glucose can be used during fermentation run as needed.
   At induction add:
      50mL of 1M MgSO4.7H2O to a 5 L bioreactor
      1 to 10mM concentration of IPTG. (carbosynth.com, product # EI05931).
      Add Fosfomycin (50 µg/mL or higher) and Carbenicillin (100 µg/mL or higher).

### Physiological switch:

The physiological switch is optimally flipped at an OD 600 of 1 to 1.1 for *E. coli* for growth in shake flasks at volumes up to 1L. For the other species tested, cultures were grown in switch media and subcultured once cultures reached maximal OD 600. In all cases the physiological switch is flipped through the addition of 100-200ug/mL Carbenicillin (From P212121, product #4800-94-6) and 50-100ug/mL Fosfomycin (From P212121, product #26016-99-9). The majority of the population is in the switched state within a few hours. To confirm that cells underwent a physiological switch, cells were imaged on a Nikon Ti-E with perfect focus system, Nikon CFI60 Plan Apo 100X NA 1.45 objective, Prior automated filter wheels and stage, LED-CFP/YFP/mCherry and LED-DA/FI/TX filter sets (Semrock), a Lumencor Sola II SE LED illumination system, and a Hamamatsu Flash 4.0 V2 CMOS camera.

### Image analysis of physiological switch:

Images were analyzed using ImageJ to measure dimensions. In the switched state, the spherical outline of the outer membrane is treated as a sphere to calculate total volume (V=(4/3)πr3). The cytoplasmic volume is calculated as an ellipsoid that exists within the sphere (V=(4/3)π*(longest radius)*(short radius)2). To calculate the periplasmic volume, the cytoplasmic volume is subtracted from the total volume of the cell.

### Protein Expression and quantification:

E. coli BL21(DE3) (NEB product #c2527) containing pET28a (emd Millipore product #69864) and its derivatives carrying GFP or collagen derivatives were grown in a shaking incubator at 37oC overnight in switch media containing 50mg/mL kanamycin (p212121 product # 2251180). Next day, subcultures are started with a 1:10 dilution of the overnight culture into fresh switch media containing 50mg/mL kanamycin. The culture is then physiologically switched and protein production is induced simultaneously at an OD 600 of 1 to 1.1 (Read on a Molecular Devices Spectramax M2 microplate reader). The physiologically switch and protein production are flipped through the addition of 100ug/mL Carbenicillin, 50ug/mL Fosfomycin, and 100ug/mL IPTG (p212121 product #367-93-1). Protein expression is continued in the switched state from between 8 hours to overnight at room temperature (approximately 22oC) on an orbital shaker. In order to quantify total protein levels, Quick Start^{™} Bradford Protein Assay was used on mixed portion of culture and standard curves are quantitated on a Molecular Devices Spectramax M2 microplate reader. In order to quantitate the relative intensity of target protein production relative to the rest of the protein population the mixed portion of the cultures were run on Mini-PROTEAN^{®} TGX^{™} Gels and stained with Bio-Safe^{™} Coomassie Stain.

### Induction of protein production:

Standard procedures have been followed to induce protein production in the physiological state. We have been using the strain BL21(DE3) containing the plasmid pET28a driving the IPTG/lactose inducible production of recombinant proteins and targeting them to the periplasmic space using the DsbA signal sequence. Using the GFP protein, targeted to the periplasmic space as described above, we have demonstrated the ability to gain and increase of 5-fold in protein production when compared to un-switched cell populations induced at the same optical density, for the same amount of time (figures). The induction was optimal at an OD600 of 1.1 and induction was continued for 10 hours at which point the protein produced was measured at about 200 mg/mL.

### Example 2: Production of Full-Length Collagen

Full length jellyfish collagen was produced using the expression system discussed in Example 1 herein. The wild-type, full length amino acid sequence of *Podocoryna carnea* (jellyfish or Hydrozoan) collagen is provided in SEQ ID NO: 1. 
GPQGVVGADGKDGTPGEKGEQGRTGAAGKQGSPGADGARGPLGSIGQQGA
RGEPGDPGSPGLRGDTGLAGVKGVAGPSGRPGQPGANGLPGVNGRGGLRGK
PGAKGIAGSDGEAGESGAPGQSGPTGPRGQRGPSGEDGNPGLQGLPGSDGEP
GEEGQPGRSGQPGQQGPRGSPGEVGPRGSKGPSGDRGDRGERGVPGQTGSA
GNVGEDGEQGGKGVDGASGPSGALGARGPPGSRGDTGAVGPPGPTGRSGLP
GNAGQKGPSGEPGSPGKAGSAGEQGPPGKDGSNGEPGSPGKEGERGLAGPPG
PDGRRGETGSPGIAGALGKPGLEGPKGYPGLRGRDGTNGKRGEQGETGPDGV
RGIPGNDGQSGKPGIDGIDGTNGQPGEAGYQGGRGTRGQLGETGDVGQNGD
RGAPGPDGSKGSAGRPGLR (SEQ ID NO: 1)
 //www.ncbi.nlm.nih.gov/protein/4379341?report=genbank&log$=protalign&blast_ra nk=1&RID=T1N9ZEUW014

The non-codon optimized polynucleotide sequence encoding the full length jellyfish collagen is disclosed in SEQ ID NO: 2. ggaccacaaggtgttgtaggagetgatggcaaagatggaacaccgggagagaaaggtgageaaggacgaaccggagc tgcaggaaaacagggaagccctggagcagatggagcaagaggccctcttggatcaattggacaacaaggtgctcgtgga gaacctggtgatccaggatctcccggcttaagaggagatactggattggctggagtcaaaggagtagcaggaccatctgg tcgacctggacaacccggtgcaaatggattacctggtgtgaatggcagaggcggtttgagaggcaaacctggtgctaaag gaattgctggcagtgatggagaagcgggagaatctggcgcacctggacagtccggacctaccggtccacgtggtcaacg aggaccaagtggtgaggatggtaatcctggattacagggattgcctggttctgatggagagcccggagaggaaggacaa cctggaagatctggtcaaccaggacagcaaggaccacgtggttcccctggagaggtaggaccaagaggatctaaaggtc catcaggagatcgtggtgacaggggagagagaggtgttcctggacaaacaggttcggctggaaatgtaggagaagatgg agagcaaggaggcaaaggtgtcgatggagcgagtggaccaagtggagctcttggtgctcgtggtcccccaggaagtag aggtgacaccggggcagtgggacctcccggacctactgggcgatctggtttacctggaaacgcaggacaaaagggacc aagtggtgaaccaggtagtccaggaaaagcaggatcagctggtgaacagggtcctcctggtaaagacggatcaaatggt gaacctggatctcctggcaaagagggtgaacgtggtcttgctggtccaccaggtccagatggcagacgtggtgaaacgg gatctccaggtatogctggtgctcttggtaaaccaggtttggaaggacctaaaggttatccaggattaagaggaagagatgg aaccaatggcaaacgaggagaacaaggagaaactggtcctgatggagtcagaggtattcctggaaatgatggacaatctg gcaaaccaggtattgatggtattgacggaacaaatggtcaaccaggtgaggctggataccaaggtggtagaggtacacgt ggtcagttaggtgaaactggtgatgtcggacagaatggagatcgaggagctcctggtcctgatggatctaaaggttctgctg gtagaccaggacttcgtgg (SEQ ID NO: 2) https://www.ncbi.nlm.nih. gov/nucleotide/3 3 5565 6?report=genbank&log$=nuclalig n&blast rank=1&RID=TSYP7CMV014

Two different codon optimized polynucleotide sequences encoding the wild-type, full-length jellyfish collagen were synthesized. The two polynucleotide sequences were slightly different due to slightly different codon optimization methods. In addition to the non-truncated, full-length jellyfish collagen, the polynucleotides also encoded a secretion tag, a 9 amino acid his tag, a short linker, and a thrombin cleavage site. The DsbA secretion tag is encoded by nucleotides 1-71. The histidine tag comprising 9 histidine residues is encoded by nucleotides 73-99 and encodes amino acids 25-33. The linker is encoded by nucleotides 100-111. The thrombin cleavage tag is encoded by nucleotides 112-135 and encodes amino acids 38-45. The truncated collagen is encoded by nucleotides 136-1422. The two polynucleotides are disclosed below in SEQ ID NO: 3 and 4.

ATGAAAAAGATTTGGCTGGCGCTGGCTGGTTTAGTTTTAGCGTTTAGCGCA
TCGGCGGCGCAGTATGAAGATCACCATCACCACCACCATCACCACTC
TGGCTCGAGCCTGGTGCCGCGCGGCAGCCATATGGGTCCGCAGGGTGTTG
TTGGTGCAGATGGTAAAGACGGTACCCCGGGTGAAAAGGAGAACAGGG
ACGTACAGGTGCAGCAGGTAAACAGGGCAGCCCGGGTGCCGATGGTGCC
CGTGGCCCGCTGGGTAGCATTGGTCAGCAGGGTGCAAGAGGCGAACCGG
GCGATCCGGGTAGTCCGGGCCTGCGTGGTGATACGGGTCTGGCCGGTGTT
AAAGGCGTTGCAGGTCCTTCAGGTCGTCCAGGTCAACCGGGTGCAAATGG
TCTGCCGGGTGTTAATGGTCGTGGCGGTCTGCGTGGCAAACCGGGAGCAA
AAGGTATTGCAGGTAGCGATGGAGAAGCCGGTGAAAGCGGTGCCCCGGG
TCAGAGTGGTCCGACCGGTCCGCGCGGTCAGCGTGGTCCGTCTGGTGAAG
ATGGCAATCCGGGTCTGCAGGGTCTGCCTGGTAGTGATGGCGAACCAGGT
GAAGAAGGTCAGCCGGGTCGTTCAGGCCAGCCGGGCCAGCAGGGCCCGC
GTGGTAGCCCGGGCGAAGTTGGCCCGCGGGGTAGTAAAGGTCCTAGTGGC
GATCGCGGTGATCGTGGTGAACGCGGTGTTCCTGGTCAGACCGGTAGCGC
AGGTAATGTTGGCGAAGATGGTGAACAGGGTGGCAAAGGTGTTGATGGTG
CAAGCGGTCCGAGCGGTGCACTGGGTGCACGTGGTCCTCCGGGCAGCCGT
GGTGACACCGGTGCAGTTGGTCCGCCTGGCCCGACCGGCCGTAGTGGCTT
ACCGGGTAATGCAGGTCAGAAAGGTCCGTCAGGTGAACCTGGCAGCCCTG
GTAAAGCAGGTAGTGCCGGTGAGCAGGGTCCGCCGGGCAAAGATGGTAG
TAATGGTGAGCCGGGTAGCCCTGGCAAAGAAGGTGAACGTGGTCTGGCAG
GACCGCCGGGTCCTGATGGTCGCCGCGGTGAAACGGGTTCACCGGGTATT
GCCGGTGCCCTGGGTAAACCAGGTCTGGAAGGTCCGAAAGGTTATCCTGG
TCTGCGCGGTCGTGATGGTACCAATGGCAAACGTGGCGAACAGGGCGAAA
CCGGTCCAGATGGTGTTCGTGGTATTCCGGGTAACGATGGTCAGAGCGGT
AAACCGGGCATTGATGGTATTGATGGCACCAATGGTCAGCCTGGCGAAGC
AGGTTATCAGGGTGGTCGCGGTACCCGTGGTCAGCTGGGTGAAACAGGTG
ATGTTGGTCAGAATGGTGATCGCGGCGCACCGGGTCCGGATGGTAGCAAA
GGTAGCGCCGGTCGTCCGGGTTTACGTtaa (SEQ ID NO: 3)

ATGAAAAAGATTTGGCTGGCGCTGGCTGGTTTAGTTTTAGCGTTTAGCGCA
TCGGCGGCGCAGTATGAAGATCACCATCACCACCACCATCACCACTC
TGGCTCGAGCCTGGTGCCGCGCGGCAGCCATATGGGTCCGCAGGGTGTTG
TTGGTGCAGATGGTAAAGACGGTACCCCGGGTGAAAAAGGtGAACAGGGt
CGTACcGGTGCAGCAGGTAAACAGGGCAGCCCGGGTGCCGATGGTGCCCG

TGGCCCGCTGGGTAGCATTGGTCAGCAGGGTGCAcgtGGCGAACCGGGCGA
TCCGGGTAGcCCGGGCCTGCGTGGTGATACGGGTCTGGCCGGTGTTAAAG
GCGTTGCAGGTCCTTCtGGTCGTCCAGGTCAACCGGGTGCAAATGGTCTGC
CGGGTGTTAATGGTCGTGGCGGTCTGCGTGGCAAACCGGGtGCAAAAGGT
ATTGCAGGTAGCGATGGcGAAGCCGGTGAAAGCGGTGCCCCGGGTCAGAG
cGGTCCGACCGGTCCGCGCGGTCAGCGTGGTCCGTCTGGTGAAGATGGCA
ATCCGGGTCTGCAGGGTCTGCCTGGTagcGATGGCGAACCAGGTGAAGAAG
GTCAGCCGGGTCGTTCtGGCCAGCCGGGCCAGCAGGGCCCGCGTGGTAGC
CCGGGCGAAGTTGGCCCGCGcGGTtcTAAAGGTCCTAGcGGCGATCGCGGT
GATCGTGGTGAACGCGGTGTTCCTGGTCAGACCGGTAGCGCAGGTAATGT
TGGCGAAGATGGTGAACAGGGTGGCAAAGGTGTTGATGGTGCAAGCGGT
CCGAGCGGTGCACTGGGTGCACGTGGTCCTCCGGGCAGCCGTGGTGACAC
CGGTGCAGTTGGTCCGCCTGGCCCGACCGGCCGTAGcGGCctgCCGGGTAAT
GCAGGTCAGAAAGGTCCGTCtGGTGAACCTGGCAGCCCTGGTAAAGCAGG
TAGcGCCGGTGAGCAGGGTCCGCCGGGCAAAGATGGTAGcAATGGTGAGC
CGGGTAGCCCTGGCAAAGAAGGTGAACGTGGTCTGGCAGGtCCGCCGGGT
CCTGATGGTCGCCGCGGTGAAACGGGTTCtCCGGGTATTGCCGGTGCCCTG
GGTAAACCAGGTCTGGAAGGTCCGAAAGGTTATCCTGGTCTGCGCGGTCG
TGATGGTACCAATGGCAAACGTGGCGAACAGGGCGAAACCGGTCCAGAT
GGTGTTCGTGGTATTCCGGGTAACGATGGTCAGAGCGGTAAACCGGGCAT
TGATGGTATTGATGGCACCAATGGTCAGCCTGGCGAAGCAGGTTATCAGG
GTGGTCGCGGTACCCGTGGTCAGCTGGGTGAAACcGGTGATGTTGGTCAG
AATGGTGATCGCGGCGCACCGGGTCCGGATGGTAGCAAAGGTAGCGCCG
GTCGTCCGGGTctgCGTtaa (SEQ ID NO: 4)

The amino acid sequence encoded by the polynucleotides of SEQ ID NO: 3 and SEQ ID NO:4 is disclosed in SEQ ID NO:5 below. In SEQ ID NO: 5 the DsbA secretion tag is encoded by nucleotides 1-71 and encodes amino acids 1-24; the histidine tag comprising 9 histidine residues is encoded by nucleotides 73-99 and encodes amino acids 25-33; the linker is encoded by nucleotides 100-111 and encodes amino acids 34-37; the thrombin cleavage tag is encoded by nucleotides 112-135 and encodes amino acids 38-45; the full-length collagen is encoded by nucleotides 136-1422 and encodes amino acids 46-474.

MKKIWLALAGLVLAFSASAAQYEDHHHHHHHHHHSGSSLVPRGSHMGPQGV
VGADGKDGTPGEKGEQGRTGAAGKQGSPGADGARGPLGSIGQQGARGEPGD

PGSPGLRGDTGLAGVKGVAGPSGRPGQPGANGLPGVNGRGGLRGKPGAKGI
AGSDGEAGESGAPGQSGPTGPRGQRGPSGEDGNPGLQGLPGSDGEPGEEGQP
GRSGQPGQQGPRGSPGEVGPRGSKGPSGDRGDRGERGVPGQTGSAGNVGED
GEQGGKGVDGASGPSGALGARGPPGSRGDTGAVGPPGPTGRSGLPGNAGQK
GPSGEPGSPGKAGSAGEQGPPGKDGSNGEPGSPGKEGERGLAGPPGPDGRRG
ETGSPGIAGALGKPGLEGPKGYPGLRGRDGTNGKRGEQGETGPDGVRGIPGN
DGQSGKPGIDGIDGTNGQPGEAGYQGGRGTRGQLGETGDVGQNGDRGAPGP
DGSKGSAGRPGLR(SEQ ID NO: 5)

The full length jellyfish collagen without the DsbA secretion tag, the histidine tag, linker and thrombin cleavage site is disclosed in SEQ ID NO: 89.

GPQGVVGADGKDGTPGEKGEQGRTGAAGKQGSPGADGARGPLGSIGQQGA
RGEPGDPGSPGLRGDTGLAGVKGVAGPSGRPGQPGANGLPGVNGRGGLRGK
PGAKGIAGSDGEAGESGAPGQSGPTGPRGQRGPSGEDGNPGLQGLPGSDGEP
GEEGQPGRSGQPGQQGPRGSPGEVGPRGSKGPSGDRGDRGERGVPGQTGSA
GNVGEDGEQGGKGVDGASGPSGALGARGPPGSRGDTGAVGPPGPTGRSGLP
GNAGQKGPSGEPGSPGKAGSAGEQGPPGKDGSNGEPGSPGKEGERGLAGPPG
PDGRRGETGSPGIAGALGKPGLEGPKGYPGLRGRDGTNGKRGEQGETGPDGV
RGIPGNDGQSGKPGIDGIDGTNGQPGEAGYQGGRGTRGQLGETGDVGQNGD
RGAPGPDGSKGSAGRPGLR(SEQ ID NO: 89)

The polynucleotides of SEQ ID NO: 3 and SEQ ID NO: 4 were synthesized by Gen9 DNA, now Ginkgo Bioworks internal synthesis. Overlaps between the pET28 vector and SEQ ID NO: 3 and SEQ ID NO: 4 were designed to be between 30 and 40bp long and added using PCR with the enzyme PrimeStar GXL polymerase (http://www.clontech.com/US/Products/PCR/GC_Rich/PrimeSTAR_GXL_DNA_Pol ymerase?sitex=10020:22372:US). The opened pET28a vector and insert DNA (SEQ ID NO: 3 or SEQ ID NO: 4) were then assembled together into the final plasmid using SGI Gibson assembly (https://us.vwr.com/store/product/17613857/gibson-assembly-hifi-1-step-kit-synthetic-genomics-inc). Sequence of plasmid was then verified through sanger sequencing through Eurofins Genomics (www.eurofinsgenomics.com).

The transformed cells were cultivated in minimal media and frozen in 1.5 aliquots with glycerol at a ratio of 50:50 of cells to glycerol. One vial of this frozen culture was revived in 50ml of minimal media overnight at 37 °C, 200rpm. Cells were transferred into 300ml of minimal media and grown for 6-9 hours to reach an OD600 of 5-10.

Minimal media used in this example and throughout this application is prepared as follows. The minimal media (Table 1) was autoclaved in several separate fractions, Salts mix (Ammonium Phosphate dibasic, Potassium phosphate monobasic, Citric acid anhydrous, Magnesium sulfate heptahydrate), the Sucrose at 500g/L, the Glucose at 55%, the Trace Metals TM5 (table 2), and Sodium Hydroxide 10M. The minimal media was then mixed together at the above concentrations post-autoclaving in the hood.

**Table 1 Minimal media recipe for shake flask cultures**

| **chemical** | **Formula** | **MW** | **Conc (g/L)** |
|---|---|---|---|
| Ammonium Phosphate dibasic | (NH₄)₂HPO₄ | 133 | 4 |
| Potassium phosphate monobasic | KH₂PO₄ | 137 | 13.3 |
| Citric acid anhydrous | H₃C₆H₅O₇ | 192.14 | 4.5 |
| Magnesium sulfate heptahydrate | MgSO₄.7H₂O | 246 | 0.59 |
| Trace Metals TM5 | | | 2 |
| Glucose | C₆H₁₂O₆ | 500 | 40 |
| Sodium Hydroxide 10M | NaOH | 400 | 5.2 |
| Sucrose 500g/L | C₁₂H₂₂O₁₁ | 500 | 66.6 |

**Table 2 Trace Metals TM5 composition**

| chemical | Formula | MW | Conc (g/L) |
|---|---|---|---|
| Ferrous Sulfate Heptahydrate | FeSO₄ • 7H₂O | 278.02 | 27.8 |
| Calcium Chloride | CaC₁₂ • 2H₂O | 147 | 2.94 |
| Manganese Chloride | MnC₁₂ | 125.84 | 1.26 |
| Zinc Sulfate | ZnSO₄ • H₂O | 179.5 | 1.8 |
| Nickel Chloride | NiC₁₂ • 6H₂O | 237.69 | 0.48 |
| Sodium Molybate | Na₂MoO₄ • 2H₂O | 241.95 | 0.48 |
| Sodium Selenite | Na₂SeO₃ | 172.94 | 0.35 |
| Boric Acid | H₃BO₃ | 61.83 | 0.12 |

The harvested cells were disrupted in a homogenizer at 14,000 psi pressure in 2 passes. Resulting slurry contained the collagen protein along with other proteins.

The collagen was purified by acid treatment of homogenized cell broth. The pH of the homogenized slurry was decreased to 3 using 6M Hydrochloric acid. Acidified cell slurry was incubated overnight at 4° C with mixing, followed by centrifugation. Supernatant of the acidified slurry was tested on a polyacrylamide gel and found to contain collagen in relatively high abundance compared to starting pellet. The collagen slurry thus obtained was high in salts. To obtain volume and salt reduction, concentration and diafiltration steps were performed using an EMD Millipore Tangential Flow Filtration system with ultrafiltration cassettes of 0.1 m² each. Total area of filtration was 0.2 m² using 2 cassettes in parallel. A volume reduction of 5x and a salt reduction of 19x was achieved in the TFF stage. Final collagen slurry was run on an SDS-PAGE gel to confirm presence of the collagen. This slurry was dried using a multi-tray lyophilizer over 3 days to obtain a white, fluffy collagen powder.

The purified collagen was analyzed on an SDS-PAGE gel and a thick and clear band was observed at the expected size of 42 kilodaltons. The purified collagen was also analyzed by mass spectrometry and it was confirmed that the 42 kilodalton protein was jellyfish collagen.

The fermentations were performed at various temperature ranging from 25° to 28° C. For some fermentations, the temperature of the fermentation was maintained at a constant temperature and immediately upon completion of fermentation (OD600 of 5-10) the collagen was purified. For other fermentations, the temperature of the fermentations was maintained for a desired period of time and when cell densities of OD600 of 5-10 were reached, the temperature was reduced to induce protein production. Typically, the temperature was reduced from 28° C to 25° C. After the fermentation at 25° C was continued for 40-60 hours, the collagen was isolated.

### Additional full length jellyfish collagens

A full length jellyfish collagen without a His tag, linker, and thrombin cleavage site is disclosed below. Two codon-optimized nucleotide sequence encoding this collagen are provided in SEQ ID NO: 6 and SEQ ID NO: 7. The differences in the nucleotide sequences are due to different codon-optimization strategies but encode the same protein. The amino acid sequence is disclosed in SEQ ID NO: 8. The DsbA secretion tag is encoded by nucleotides 1-72 and encodes amino acids 1-24. The collagen sequence is encoded by nucleotides 73-1359 and encodes amino acids 25-453.

ATGAAAAAGATTTGGCTGGCGCTGGCTGGTTTAGTTTTAGCGTTTAGCGCA
TCGGCGGCGCAGTATGAAGATGGTCCGCAGGGTGTTGTTGGTGCAGATGG
TAAAGACGGTACCCCGGGTGAAAAAGGAGAACAGGGACGTACAGGTGCA
GCAGGTAAACAGGGCAGCCCGGGTGCCGATGGTGCCCGTGGCCCGCTGG
GTAGCATTGGTCAGCAGGGTGCAAGAGGCGAACCGGGCGATCCGGGTAG
TCCGGGCCTGCGTGGTGATACGGGTCTGGCCGGTGTTAAAGGCGTTGCAG
GTCCTTCAGGTCGTCCAGGTCAACCGGGTGCAAATGGTCTGCCGGGTGTT
AATGGTCGTGGCGGTCTGCGTGGCAAACCGGGAGCAAAAGGTATTGCAGG
TAGCGATGGAGAAGCCGGTGAAAGCGGTGCCCCGGGTCAGAGTGGTCCG
ACCGGTCCGCGCGGTCAGCGTGGTCCGTCTGGTGAAGATGGCAATCCGGG
TCTGCAGGGTCTGCCTGGTAGTGATGGCGAACCAGGTGAAGAAGGTCAGC
CGGGTCGTTCAGGCCAGCCGGGCCAGCAGGGCCCGCGTGGTAGCCCGGGC
GAAGTTGGCCCGCGGGGTAGTAAAGGTCCTAGTGGCGATCGCGGTGATCG
TGGTGAACGCGGTGTTCCTGGTCAGACCGGTAGCGCAGGTAATGTTGGCG
AAGATGGTGAACAGGGTGGCAAAGGTGTTGATGGTGCAAGCGGTCCGAG
CGGTGCACTGGGTGCACGTGGTCCTCCGGGCAGCCGTGGTGACACCGGTG
CAGTTGGTCCGCCTGGCCCGACCGGCCGTAGTGGCTTACCGGGTAATGCA
GGTCAGAAAGGTCCGTCAGGTGAACCTGGCAGCCCTGGTAAAGCAGGTAG
TGCCGGTGAGCAGGGTCCGCCGGGCAAAGATGGTAGTAATGGTGAGCCG

GGTAGCCCTGGCAAAGAAGGTGAACGTGGTCTGGCAGGACCGCCGGGTCC
TGATGGTCGCCGCGGTGAAACGGGTTCACCGGGTATTGCCGGTGCCCTGG
GTAAACCAGGTCTGGAAGGTCCGAAAGGTTATCCTGGTCTGCGCGGTCGT
GATGGTACCAATGGCAAACGTGGCGAACAGGGCGAAACCGGTCCAGATG
GTGTTCGTGGTATTCCGGGTAACGATGGTCAGAGCGGTAAACCGGGCATT
GATGGTATTGATGGCACCAATGGTCAGCCTGGCGAAGCAGGTTATCAGGG
TGGTCGCGGTACCCGTGGTCAGCTGGGTGAAACAGGTGATGTTGGTCAGA
ATGGTGATCGCGGCGCACCGGGTCCGGATGGTAGCAAAGGTAGCGCCGGT
CGTCCGGGTTTACGTtaa (SEQ ID NO: 6)

ATGAAAAAGATTTGGCTGGCGCTGGCTGGTTTAGTTTTAGCGTTTAGCGCA
TCGGCGGCGCAGTATGAAGATGGTCCGCAGGGTGTTGTTGGTGCAGATGG
TAAAGACGGTACCCCGGGTGAAAAAGGtGAACAGGGtCGTACcGGTGCAGC
AGGTAAACAGGGCAGCCCGGGTGCCGATGGTGCCCGTGGCCCGCTGGGTA
GCATTGGTCAGCAGGGTGCAcgtGGCGAACCGGGCGATCCGGGTAGcCCGG
GCCTGCGTGGTGATACGGGTCTGGCCGGTGTTAAAGGCGTTGCAGGTCCT
TCtGGTCGTCCAGGTCAACCGGGTGCAAATGGTCTGCCGGGTGTTAATGGT
CGTGGCGGTCTGCGTGGCAAACCGGGtGCAAAAGGTATTGCAGGTAGCGA
TGGcGAAGCCGGTGAAAGCGGTGCCCCGGGTCAGAGcGGTCCGACCGGTC
CGCGCGGTCAGCGTGGTCCGTCTGGTGAAGATGGCAATCCGGGTCTGCAG
GGTCTGCCTGGTagcGATGGCGAACCAGGTGAAGAAGGTCAGCCGGGTCGT
TCtGGCCAGCCGGGCCAGCAGGGCCCGCGTGGTAGCCCGGGCGAAGTTGG
CCCGCGcGGTtcTAAAGGTCCTAGcGGCGATCGCGGTGATCGTGGTGAACGC
GGTGTTCCTGGTCAGACCGGTAGCGCAGGTAATGTTGGCGAAGATGGTGA
ACAGGGTGGCAAAGGTGTTGATGGTGCAAGCGGTCCGAGCGGTGCACTGG
GTGCACGTGGTCCTCCGGGCAGCCGTGGTGACACCGGTGCAGTTGGTCCG
CCTGGCCCGACCGGCCGTAGcGGCctgCCGGGTAATGCAGGTCAGAAAGGT
CCGTCtGGTGAACCTGGCAGCCCTGGTAAAGCAGGTAGcGCCGGTGAGCAG
GGTCCGCCGGGCAAAGATGGTAGcAATGGTGAGCCGGGTAGCCCTGGCAA
AGAAGGTGAACGTGGTCTGGCAGGtCCGCCGGGTCCTGATGGTCGCCGCG
GTGAAACGGGTTCtCCGGGTATTGCCGGTGCCCTGGGTAAACCAGGTCTGG
AAGGTCCGAAAGGTTATCCTGGTCTGCGCGGTCGTGATGGTACCAATGGC
AAACGTGGCGAACAGGGCGAAACCGGTCCAGATGGTGTTCGTGGTATTCC
GGGTAACGATGGTCAGAGCGGTAAACCGGGCATTGATGGTATTGATGGCA

CCAATGGTCAGCCTGGCGAAGCAGGTTATCAGGGTGGTCGCGGTACCCGT
GGTCAGCTGGGTGAAACcGGTGATGTTGGTCAGAATGGTGATCGCGGCGC
ACCGGGTCCGGATGGTAGCAAAGGTAGCGCCGGTCGTCCGGGTctgCGTtaa
(SEQ ID NO: 7)

MKKIWLALAGLVLAFSASAAQYEDGPQGVVGADGKDGTPGEKGEQGRTGA
AGKQGSPGADGARGPLGSIGQQGARGEPGDPGSPGLRGDTGLAGVKGVAGP
SGRPGQPGANGLPGVNGRGGLRGKPGAKGIAGSDGEAGESGAPGQSGPTGPR
GQRGPSGEDGNPGLQGLPGSDGEPGEEGQPGRSGQPGQQGPRGSPGEVGPRG
SKGPSGDRGDRGERGVPGQTGSAGNVGEDGEQGGKGVDGASGPSGALGAR
GPPGSRGDTGAVGPPGPTGRSGLPGNAGQKGPSGEPGSPGKAGSAGEQGPPG
KDGSNGEPGSPGKEGERGLAGPPGPDGRRGETGSPGIAGALGKPGLEGPKGY
PGLRGRDGTNGKRGEQGETGPDGVRGIPGNDGQSGKPGIDGIDGTNGQPGEA
GYQGGRGTRGQLGETGDVGQNGDRGAPGPDGSKGSAGRPGLR (SEQ ID NO:
8)

### Example 3: Production of Truncated Collagen

A codon optimized DNA sequence, optimized for expression in *E. coli,* encoding a jellyfish collagen with a truncation of 240 internal amino acids was synthesized and expressed. The DNA sequence is shown below in SEQ ID NO: 9. In SEQ ID NO: 9, The DsbA secretion tag is encoded by nucleotides 1-72 and encodes amino acids 1-24 of SEQ ID NO: 10. The histidine tag comprising 9 histidine residues is encoded by nucleotides 73-99 and encodes amino acids 25-33 of SEQ ID NO: 10. The linker is encoded by nucleotides 100-111 and encodes amino acids 34-37 of SEQ ID NO: 10. The thrombin cleavage site is encoded by nucleotides 112-135 and encodes amino acids 38-45 of SEQ ID NO: 10. The truncated collagen is encoded by nucleotides 136-822 and encodes amino acids 46-274 of SEQ ID NO: 10.

ATGAAAAAGATTTGGCTGGCGCTGGCTGGTTTAGTTTTAGCGTTTAGCGCA
TCGGCGGCGCAGTATGAAGATCACCATCACCACCACCATCACCACTC
TGGCTCGAGCCTGGTGCCGCGCGGCAGCCATATGGGTCCGCAGGGTGTTG
TTGGTGCAGATGGTAAAGACGGTACCCCGGGTGAAAAGGAGAACAGGG
ACGTACAGGTGCAGCAGGTAAACAGGGCAGCCCGGGTGCCGATGGTGCC
CGTGGCCCGCTGGGTAGCATTGGTCAGCAGGGTGCAAGAGGCGAACCGG
GCGATCCGGGTAGTCCGGGCCTGCGTGGTGATACGGGTCTGGCCGGTGTT

AAAGGCGTTGCAGGTCCTTCAGGTCGTCCAGGTCAACCGGGTGCAAATGG
TCTGCCGGGTGTTAATGGTCGTGGCGGTCTGGAACGTGGTCTGGCAGGAC
CGCCGGGTCCTGATGGTCGCCGCGGTGAAACGGGTTCACCGGGTATTGCC
GGTGCCCTGGGTAAACCAGGTCTGGAAGGTCCGAAAGGTTATCCTGGTCT
GCGCGGTCGTGATGGTACCAATGGCAAACGTGGCGAACAGGGCGAAACC
GGTCCAGATGGTGTTCGTGGTATTCCGGGTAACGATGGTCAGAGCGGTAA
ACCGGGCATTGATGGTATTGATGGCACCAATGGTCAGCCTGGCGAAGCAG
GTTATCAGGGTGGTCGCGGTACCCGTGGTCAGCTGGGTGAAACAGGTGAT
GTTGGTCAGAATGGTGATCGCGGCGCACCGGGTCCGGATGGTAGCAAAGG
TAGCGCCGGTCGTCCGGGTTTACGTtaa (SEQ ID NO: 9)

The truncated collagen is approximately 54% of the full length collagen and is disclosed below in SEQ ID NO: 10.

MKKIWLALAGLVLAFSASAAQYEDHHHHHHHHHSGSSLVPRGSHMGPQGV
VGADGKDGTPGEKGEQGRTGAAGKQGSPGADGARGPLGSIGQQGARGEPGD
PGSPGLRGDTGLAGVKGVAGPSGRPGQPGANGLPGVNGRGGLERGLAGPPG
PDGRRGETGSPGIAGALGKPGLEGPKGYPGLRGRDGTNGKRGEQGETGPDGV
RGIPGNDGQSGKPGIDGIDGTNGQPGEAGYQGGRGTRGQLGETGDVGQNGD
RGAPGPDGSKGSAGRPGLR (SEQ ID NO: 10)

The polynucleotide encoding the truncated jellyfish collagen without the DsbA secretion tag, the histidine tag, linker and thrombin cleavage site is disclosed in SEQ ID NO: 85

GTCCGCAGGGTGTTGTTGGTGCAGATGGTAAAGACGGTACCCCGGGTGAA
AAAGGAGAACAGGGACGTACAGGTGCAGCAGGTAAACAGGGCAGCCCGG
GTGCCGATGGTGCCCGTGGCCCGCTGGGTAGCATTGGTCAGCAGGGTGCA
AGAGGCGAACCGGGCGATCCGGGTAGTCCGGGCCTGCGTGGTGATACGG
GTCTGGCCGGTGTTAAAGGCGTTGCAGGTCCTTCAGGTCGTCCAGGTCAA
CCGGGTGCAAATGGTCTGCCGGGTGTTAATGGTCGTGGCGGTCTGGAACG
TGGTCTGGCAGGACCGCCGGGTCCTGATGGTCGCCGCGGTGAAACGGGTT
CACCGGGTATTGCCGGTGCCCTGGGTAAACCAGGTCTGGAAGGTCCGAAA
GGTTATCCTGGTCTGCGCGGTCGTGATGGTACCAATGGCAAACGTGGCGA
ACAGGGCGAAACCGGTCCAGATGGTGTTCGTGGTATTCCGGGTAACGATG
GTCAGAGCGGTAAACCGGGCATTGATGGTATTGATGGCACCAATGGTCAG
CCTGGCGAAGCAGGTTATCAGGGTGGTCGCGGTACCCGTGGTCAGCTGGG

TGAAACAGGTGATGTTGGTCAGAATGGTGATCGCGGCGCACCGGGTCCGG
ATGGTAGCAAAGGTAGCGCCGGTCGTCCGGGTTTACGTtaa (SEQ ID NO: 85)

The truncated jellyfish collagen without the DsbA secretion tag, the histidine tag, linker and thrombin cleavage site is disclosed in SEQ ID NO: 86

GPQGVVGADGKDGTPGEKGEQGRTGAAGKQGSPGADGARGPLGSIGQQGA
RGEPGDPGSPGLRGDTGLAGVKGVAGPSGRPGQPGANGLPGVNGRGGLERG
LAGPPGPDGRRGETGSPGIAGALGKPGLEGPKGYPGLRGRDGTNGKRGEQGE
TGPDGVRGIPGNDGQSGKPGIDGIDGTNGQPGEAGYQGGRGTRGQLGETGDV
GQNGDRGAPGPDGSKGSAGRPGLR (SEQ ID NO: 86)

The polynucleotides of SEQ ID NO: 9 was codon optimized and synthesized by Gen9 DNA, now Ginkgo Bioworks internal synthesis. Overlaps between the pET28 vector and SEQ ID NO: 9 were designed to be between 30 and 40bp long and added using PCRwith the enzyme PrimeStar GXL polymerase (http://www.clontech.com/US/Products/PCR/GC_Rich/PrimeSTAR_GXL_DNA_Pol ymerase?sitex=10020:22372:US). The opened pET28a vector and insert DNA (SEQ ID NO: 9) was then assembled together into the final plasmid using SGI Gibson assembly (https us.vwr.com/store/product/17613857/gibson-assembly-hifi-1-step-kit-synthetic-genomics-inc). Sequence of plasmid was then verified through sanger sequencing through Eurofins Genomics (www.eurofinsgenomics.com).

The transformed cells were cultivated in minimal media and frozen in 1.5 aliquots with glycerol at a ratio of 50:50 of cells to glycerol. One vial of this frozen culture was revived in 50ml of minimal media overnight at 37 °C, 200rpm. Cells were transferred into 300ml of minimal media and grown for 6-9 hours to reach an OD600 of 5-10.

A bioreactor was prepared with 2.7L of minimal media + glucose and 300 ml of OD600 of 5-10 culture was added to bring the starting volume to 3L. Cells were grown at 28°C, pH7 with Dissolved Oxygen maintained at 20% saturation using a cascade containing agitation, air and oxygen. pH was controlled using 28% w/w Ammonium hydroxide solution. Fermentation was run in a fed-batch mode using a DO-stat based feeding algorithm once the initial bolus of 40g/L was depleted around 13hours. After 24-26hours of initial growth, the OD600 reached above 100. At this point, 300mL of 500g/L sucrose was added and temperature was reduced to 25C. High density culture was induced for protein production using 1mM IPTG. Fermentation was continued for another 20-24 hours and cells were harvested using a bench top centrifuge at 9000 rcf, 15C for 60 minutes. Cell pellet recovered from centrifugation was resuspended in a buffer containing 0.5M NaCl and 0.1M KH2PO4 at pH8 in a weight by weight ratio of 2x buffer to 1x cells.

The harvested cells were disrupted in a homogenizer at 14,000 psi pressure in 2 passes. Resulting slurry contained the collagen protein along with other proteins.

The fermentations were performed at various temperature ranging from 25° to 28° C. For some fermentations, the temperature of the fermentation was maintained at a constant temperature and immediately upon completion of fermentation (OD600 of 5-10) the collagen was purified. For other fermentations, the temperature of the fermentations was maintained for a desired period of time and when cell densities of OD600 of 5-10 were reached, the temperature was reduced to induce protein production. Typically, the temperature was reduced from 28° C to 25° C. After the fermentation at 25° C was continued for 40-60 hours, the collagen was isolated.

The collagen was purified by acid treatment of homogenized cell broth. Additionally, acid treatment was also performed on non-homogenized whole cells recovered from the bioreactor after centrifugation and resuspension in the buffer described above. The pH of either the homogenized slurry of the resuspended whole cells was decreased to 3 using 6M Hydrochloric acid. Acidified cell slurry was incubated overnight at 4° C with mixing, followed by centrifugation. Supernatant of the acidified slurry was tested on a polyacrylamide gel and found to contain collagen in relatively high abundance compared to starting pellet. The collagen slurry thus obtained was high in salts. To obtain volume and salt reduction, concentration and diafiltration steps were performed using an EMD Millipore Tangential Flow Filtration system with ultrafiltration cassettes of 0.1 m² each. Total area of filtration was 0.2 m² using 2 cassettes in parallel. A volume reduction of 5x and a salt reduction of 19x was achieved in the TFF stage. Final collagen slurry was run on an SDS-PAGE gel to confirm presence of the collagen. This slurry was dried using a multi-tray lyophilizer over 3 days to obtain a white, fluffy collagen powder.

The purified truncated collagen obtained from homogenized cell broth or non-homogenized cells were analyzed on an SDS-PAGE gel and a thick and clear band was observed at the expected size of 27 kilodaltons. The purified collagen was also analyzed by mass spectrometry and it was confirmed that the 27 kilodalton protein was jellyfish collagen.

An alternative purification method of the full length and truncated collagens is provided below.

The fermentation broth was mixed with 0.3-0.5% w/v of Poly Ethyl Imine (PEI). After 15 minutes of incubation with PEI, the fermentation broth was centrifuged at 9000 rcf for 15 minutes to recover the supernatant, which contained the collagen protein. The pellet containing the cells was discarded and the PEI-treated collagen containing supernatant was mixed with Sodium Bentonite (0.2% final w/v) (Wyopure^{®}, Wyoming Bentonite) and centrifuged. The bentonite containing pellet was discarded and the supernatant was recovered.

The Bentonite treated supernatant was concentrated between 3-6 fold on a tangential flow filtration system (TFF) (EMD Millipore) using a 5kDa cassette. The collagen was retained with almost no losses in the permeate stream. To remove salts, the retentate from the concentration step was diafiltered using the same TFF set-up. Final conductivity of the protein solution was < 10 milliSiemens. The typical conductivity was between 400 microsiemens and 1.5 millisiemens. Highly concentrated collagen solutions had higher conductivities approaching 4 milliSiemens. A skilled artisan will understand that conductivities higher than 10 milliSiemens may be observed depending on the concentration of the collagen. Next, the desalted and concentrated protein was subjected to treatment with activated carbon using the W-L 9000 10 x 40 granulated resin (Carbon Activated Corporation). 5% w/v of the carbon resin was mixed with collagen containing protein feed and mixed at 45-50°C with mild agitation. The carbon-treated slurry was filtered using a Buchner funnel lined with an Ertel Filter Press Pad M-953 (Ertel Alsop) in presence or absence of a filtration aid such as Diatomaceous Earth (Sigma Aldrich). Post-filtration, the collagen solution was filtered through a 0.2 micron filter followed by one to several hours of treatment with Sodium Bentonite (0.2% w/v final) (Wyopure^{®}, Wyoming Bentonite) and centrifuged at 9000 ref, 15-30 minutes to obtain a highly pure, clear and particulate free collagen solution. When removal of endotoxin proteins was desired, the protein was passed through a chromatographic filter like Sartobind-Q (Sartorius-Stedim) to specifically remove endotoxin proteins.

The purified collagen was analyzed on an SDS-PAGE gel and a thick and clear band was observed at 30 kilodaltons. The upshift in size is due to the structure of the collagen molecule and the high glycine/proline amino acid content. The purified collagen was also analyzed by mass spectrometry and it was confirmed that the 30 kilodalton protein was the truncated collagen.

The truncated collagens were further analyzed by HPLC using an Agilent 1100 series HPLC. The column was the 50mm Agilent PLRP-S reverse phase column with an inner diameter of 4.6mm, µM particle size and 1000 Angstrom pore size.

The sample was prepared by diluting 1:1 in a 0.04% sodium azide solution in HPLC-grade water. After dilution, the resulting mixture was filtered through a 0.45 um filter to remove any large particles that can clog the HPLC column. For analysis, the samples are diluted appropriately with a 20 mM ammonium acetate buffer in HPLC-grade water at a pH of about 4.5. After mixing the sample, it was transferred to a 300 µL microvial that was then placed in the autosampler. Using ChemStation, the software that operates the HPLC, the analysis parameters such as sample flowrate, column temperature, mobile phase flowrate, mobile phase composition, etc. can be altered. In one exemplary, but non-limiting analysis the parameters were: sample flow rate of 1 mL/min, column temperature of 80°C, column pressure of 60-70 bar, mobile phase composition of 97.9% water/1.9% acetonitrile with 0.2% trifluoroacetic acid; UV wavelength for analysis of 214.4 nm, injection volume of 10 µL, and sample run time of 10 minutes.

Under these conditions, the truncated jellyfish collation of SEQ ID NO: 91 has an elution time of about 5.4 minutes. ChemStation quantifies the peak area of the elution peak and calculates the protein concentration using a calibration curve that directly relates peak area to protein concentration. The calibration curve is generated using a known collagen solution that is serially diluted to contain collagen concentration ranges of 0.06 mg/mL to 1.00 mg/mL.

### Truncated Collagen Without His Tag-Linker-Thrombin Cleavage Site

A truncated jellyfish collagen without a His tag, linker, and thrombin cleavage site is disclosed below. The codon-optimized nucleotide sequence encoding this collagen is provided in SEQ ID NO: 11. The amino acid sequence is disclosed in SEQ ID NO: 12. The DsbA secretion tag is encoded by nucleotides 1-72 and encodes amino acids 1-24. The truncated collagen sequence is encoded by nucleotides 73-639 and encodes amino acids 25-213.

ATGAAAAAGATTTGGCTGGCGCTGGCTGGTTTAGTTTTAGCGTTTAGCGCA
TCGGCGGCGCAGTATGAAGATGGTCCGCAGGGTGTTGTTGGTGCAGATGG

TAAAGACGGTACCCCGGGTAATGCAGGTCAGAAAGGTCCGTCAGGTGAAC
CTGGCAGCCCTGGTAAAGCAGGTAGTGCCGGTGAGCAGGGTCCGCCGGGC
AAAGATGGTAGTAATGGTGAGCCGGGTAGCCCTGGCAAAGAAGGTGAAC
GTGGTCTGGCAGGACCGCCGGGTCCTGATGGTCGCCGCGGTGAAACGGGT
TCACCGGGTATTGCCGGTGCCCTGGGTAAACCAGGTCTGGAAGGTCCGAA
AGGTTATCCTGGTCTGCGCGGTCGTGATGGTACCAATGGCAAACGTGGCG
AACAGGGCGAAACCGGTCCAGATGGTGTTCGTGGTATTCCGGGTAACGAT
GGTCAGAGCGGTAAACCGGGCATTGATGGTATTGATGGCACCAATGGTCA
GCCTGGCGAAGCAGGTTATCAGGGTGGTCGCGGTACCCGTGGTCAGCTGG
GTGAAACAGGTGATGTTGGTCAGAATGGTGATCGCGGCGCACCGGGTCCG
GATGGTAGCAAAGGTAGCGCCGGTCGTCCGGGTTTACGTtaa (SEQ ID NO:
11)

MKKIWLALAGLVLAFSASAAQYEDGPQGVVGADGKDGTPGNAGQKGPSGE
PGSPGKAGSAGEQGPPGKDGSNGEPGSPGKEGERGLAGPPGPDGRRGETGSP
GIAGALGKPGLEGPKGYPGLRGRDGTNGKRGEQGETGPDGVRGIPGNDGQS
GKPGIDGIDGTNGQPGEAGYQGGRGTRGQLGETGDVGQNGDRGAPGPDGSK
GSAGRPGLR (SEQ ID NO: 12)

A polynucleotide encoding a truncated jellyfish collagen without a His tag, linker and thrombin cleavage site disclosed in SEQ ID NO: 90

GGTCCGCAGGGTGTTGTTGGTGCAGATGGTAAAGACGGTACCCCGGGTAA
TGCAGGTCAGAAAGGTCCGTCAGGTGAACCTGGCAGCCCTGGTAAAGCAG
GTAGTGCCGGTGAGCAGGGTCCGCCGGGCAAAGATGGTAGTAATGGTGA
GCCGGGTAGCCCTGGCAAAGAAGGTGAACGTGGTCTGGCAGGACCGCCG
GGTCCTGATGGTCGCCGCGGTGAAACGGGTTCACCGGGTATTGCCGGTGC
CCTGGGTAAACCAGGTCTGGAAGGTCCGAAAGGTTATCCTGGTCTGCGCG
GTCGTGATGGTACCAATGGCAAACGTGGCGAACAGGGCGAAACCGGTCC
AGATGGTGTTCGTGGTATTCCGGGTAACGATGGTCAGAGCGGTAAACCGG
GCATTGATGGTATTGATGGCACCAATGGTCAGCCTGGCGAAGCAGGTTAT
CAGGGTGGTCGCGGTACCCGTGGTCAGCTGGGTGAAACAGGTGATGTTGG
TCAGAATGGTGATCGCGGCGCACCGGGTCCGGATGGTAGCAAAGGTAGCG
CCGGTCGTCCGGGTTTACGTtaa (SEQ ID NO: 90)

A truncated jellyfish collagen without a His tag, linker and thrombin cleavage site disclosed in SEQ ID NO: 91

GPQGVVGADGKDGTPGNAGQKGPSGEPGSPGKAGSAGEQGPPGKDGSNGEP
GSPGKEGERGLAGPPGPDGRRGETGSPGIAGALGKPGLEGPKGYPGLRGRDG
TNGKRGEQGETGPDGVRGIPGNDGQSGKPGIDGIDGTNGQPGEAGYQGGRGT
RGQLGETGDVGQNGDRGAPGPDGSKGSAGRPGLR (SEQ ID NO: 91)

### Truncated Collagen With GEK Repeats

A jellyfish collagen with GEK repeats is disclosed below. The codon-optimized nucleotide sequence encoding this collagen is provided in SEQ ID NO: 13. The amino acid sequence is disclosed in SEQ ID NO: 14. The DsbA secretion tag is encoded by nucleotides 1-72 and encodes amino acids 1-24. The GEK repeat is encoded by nucleotides 73-126 and encodes the GEK repeats of amino acids 25-42. The truncated collagen sequence is encoded by nucleotides 127-693 and encodes amino acids 43-231.

ATGAAAAAGATTTGGCTGGCGCTGGCTGGTTTAGTTTTAGCGTTTAGCGCA
TCGGCGGCGCAGTATGAAGATGGTGAAAAAGGTGAaAAGGGCGAGAAAG
GTGAGAAAGGCGAAAAGGGTGAAAAAGGTCCGCAGGGTGTTGTTGGTGC
AGATGGTAAAGACGGTACCCCGGGTAATGCAGGTCAGAAAGGTCCGTCA
GGTGAACCTGGCAGCCCTGGTAAAGCAGGTAGTGCCGGTGAGCAGGGTCC
GCCGGGCAAAGATGGTAGTAATGGTGAGCCGGGTAGCCCTGGCAAAGAA
GGTGAACGTGGTCTGGCAGGACCGCCGGGTCCTGATGGTCGCCGCGGTGA
AACGGGTTCACCGGGTATTGCCGGTGCCCTGGGTAAACCAGGTCTGGAAG
GTCCGAAAGGTTATCCTGGTCTGCGCGGTCGTGATGGTACCAATGGCAAA
CGTGGCGAACAGGGCGAAACCGGTCCAGATGGTGTTCGTGGTATTCCGGG
TAACGATGGTCAGAGCGGTAAACCGGGCATTGATGGTATTGATGGCACCA
ATGGTCAGCCTGGCGAAGCAGGTTATCAGGGTGGTCGCGGTACCCGTGGT
CAGCTGGGTGAAACAGGTGATGTTGGTCAGAATGGTGATCGCGGCGCACC
GGGTCCGGATGGTAGCAAAGGTAGCGCCGGTCGTCCGGGTTTACGTtaa
(SEQ ID NO: 13)

MKKIWLALAGLVLAFSASAAQYEDGEKGEKGEKGEKGEKGPQGVVGA
DGKDGTPGNAGQKGPSGEPGSPGKAGSAGEQGPPGKDGSNGEPGSPGKEGE
RGLAGPPGPDGRRGETGSPGIAGALGKPGLEGPKGYPGLRGRDGTNGKRGEQ
GETGPDGVRGIPGNDGQSGKPGIDGIDGTNGQPGEAGYQGGRGTRGQLGETG
DVGQNGDRGAPGPDGSKGSAGRPGLR(SEQ ID NO: 14)

The polynucleotides of SEQ ID NO: 13 was codon optimized and synthesized by Gen9 DNA, now Ginkgo Bioworks internal synthesis. Overlaps between the pET28 vector and SEQ ID NO: 13 was designed to be between 30 and 40bp long and added using PCR with the enzyme PrimeStar GXL polymerase (http://www.clontech.com/US/Products/PCR/GC_Rich/PrimeSTAR_GXL_DNA_Pol ymerase?sitex=10020:22372:US). The opened pET28a vector and insert DNA (SEQ ID NO: 13) was then assembled together into the final plasmid using SGI Gibson assembly (https://us.vwr.com/store/product/17613857/gibson-assembly-hifi-1-step-kit-synthetic-genomics-inc). Sequence of plasmid was then verified through Sanger sequencing through Eurofins Genomics (www.eurofinsgenomics.com).

The transformed cells were cultivated in minimal media and frozen in 1.5 aliquots with glycerol at a ratio of 50:50 of cells to glycerol. One vial of this frozen culture was revived in 50ml of minimal media overnight at 37 °C, 200rpm. Cells were transferred into 300ml of minimal media and grown for 6-9 hours to reach an OD600 of 5-10.

A bioreactor was prepared with 2.7L of minimal media + glucose and 300 ml of OD600 of 5-10 culture was added to bring the starting volume to 3L. Cells were grown at 28°C, pH7 with Dissolved Oxygen maintained at 20% saturation using a cascade containing agitation, air and oxygen. pH was controlled using 28% w/w Ammonium hydroxide solution. Fermentation was run in a fed-batch mode using a DO-stat based feeding algorithm once the initial bolus of 40g/L was depleted around 13hours. After 24-26 hours of initial growth, the OD600 reached above 100. At this point, 300mL of 500g/L sucrose was added and temperature was reduced to 25C. High density culture was induced for protein production using 1mM IPTG. Fermentation was continued for another 20-24 hours and cells were harvested using a bench top centrifuge at 9000 rcf, 15C for 60 minutes. Cell pellet recovered from centrifugation was resuspended in a buffer containing 0.5M NaCl and 0.1M KH2PO4 at pH8 in a weight by weight ratio of 2x buffer to 1x cells.

The harvested cells were disrupted in a homogenizer at 14,000 psi pressure in 2 passes. Resulting slurry contained the collagen protein along with other proteins.

The collagen was purified by acid treatment whole cells recovered from bioreactor after centrifugation and resuspension in a buffer as described above- The pH of either the homogenized slurry or the resuspended suspension was decreased to 3 using 6M Hydrochloric acid. Acidified cell slurry was incubated overnight at 4° C with mixing, followed by centrifugation. Supernatant of the acidified slurry was tested on a polyacrylamide gel and found to contain collagen in relatively high abundance compared to starting pellet. The collagen slurry thus obtained was high in salts. To obtain volume and salt reduction, concentration and diafiltration steps were performed using an EMD Millipore Tangential Flow Filtration system with ultrafiltration cassettes of 0.1 m² each. Total area of filtration was 0.2 m² using 2 cassettes in parallel. A volume reduction of 5x and a salt reduction of 19x was achieved in the TFF stage. Final collagen slurry was run on an SDS-PAGE gel to confirm presence of the collagen. This slurry was dried using a multi-tray lyophilizer over 3 days to obtain a white, fluffy collagen powder.

The purified collagen was analyzed on an SDS-PAGE gel and was observed to run at an apparent molecular weight of 35 kilodaltons. The 35 kilodalton band does not correspond to the expected size of 22 kilodaltons. The upshift between the expected size and the apparent size is thought to be due to the GEK repeats interacting with the gel matrix. The 35 kDa band was confirmed by mass spectrometry to be the correct collagen with the GEK repeats.

### Truncated Collagen With GDK Repeats

A jellyfish collagen with GDK repeats is disclosed below. The codon-optimized nucleotide sequence encoding this collagen is provided in SEQ ID NO: 15. The amino acid sequence is disclosed in SEQ ID NO: 16. The DsbA secretion tag is encoded by nucleotides 1-72 and encodes amino acids 1-24. The GDK repeat is encoded by nucleotides 73-126 and encodes the GDK repeats of amino acids 25-42. The truncated collagen sequence is encoded by nucleotides 127-693 and encodes amino acids 43-231.

ATGAAAAAGATTTGGCTGGCGCTGGCTGGTTTAGTTTTAGCGTTTAGCGCA
TCGGCGGCGCAGTATGAAGATGGTGATAAAGGTGATAAGGGCGACAAAG
GTGACAAAGGCGATAAGGGTGATAAAGGTCCGCAGGGTGTTGTTGGTGCA
GATGGTAAAGACGGTACCCCGGGTAATGCAGGTCAGAAAGGTCCGTCAG
GTGAACCTGGCAGCCCTGGTAAAGCAGGTAGTGCCGGTGAGCAGGGTCCG
CCGGGCAAAGATGGTAGTAATGGTGAGCCGGGTAGCCCTGGCAAAGAAG
GTGAACGTGGTCTGGCAGGACCGCCGGGTCCTGATGGTCGCCGCGGTGAA
ACGGGTTCACCGGGTATTGCCGGTGCCCTGGGTAAACCAGGTCTGGAAGG
TCCGAAAGGTTATCCTGGTCTGCGCGGTCGTGATGGTACCAATGGCAAAC
GTGGCGAACAGGGCGAAACCGGTCCAGATGGTGTTCGTGGTATTCCGGGT
AACGATGGTCAGAGCGGTAAACCGGGCATTGATGGTATTGATGGCACCAA
TGGTCAGCCTGGCGAAGCAGGTTATCAGGGTGGTCGCGGTACCCGTGGTC

AGCTGGGTGAAACAGGTGATGTTGGTCAGAATGGTGATCGCGGCGCACCG
GGTCCGGATGGTAGCAAAGGTAGCGCCGGTCGTCCGGGTTTACGTtaa (SEQ
ID NO: 15)

MKKIWLALAGLVLAFSASAAQYEDGDKGDKGDKGDKGDKGPQGVVG
ADGKDGTPGNAGQKGPSGEPGSPGKAGSAGEQGPPGKDGSNGEPGSPGKEG
ERGLAGPPGPDGRRGETGSPGIAGALGKPGLEGPKGYPGLRGRDGTNGKRGE
QGETGPDGVRGIPGNDGQSGKPGIDGIDGTNGQPGEAGYQGGRGTRGQLGET
GDVGQNGDRGAPGPDGSKGSAGRPGLR (SEQ ID NO: 16)

The polynucleotides of SEQ ID NO: 15 was codon optimized and synthesized by Gen9 DNA, now Ginkgo Bioworks internal synthesis. Overlaps between the pET28 vector and SEQ ID NO: 15 was designed to be between 30 and 40bp long and added using PCR with the enzyme PrimeStar GXL polymerase (http://www.clontech.com/US/Products/PCR/GC_Rich/PrimeSTAR_GXL_DNA_Pol ymerase?sitex=10020:22372:US). The opened pET28a vector and insert DNA (SEQ ID NO: 15) was then assembled together into the final plasmid using SGI Gibson assembly (https://us.vwr.com/store/product/17613857/gibson-assembly-hifi-1-step-kit-synthetic-genomics-inc). Sequence of plasmid was then verified through sanger sequencing through Eurofins Genomics (www.eurofinsgenomics.com).

The transformed cells were cultivated in minimal media and frozen in 1.5 aliquots with glycerol at a ratio of 50:50 of cells to glycerol. One vial of this frozen culture was revived in 50ml of minimal media overnight at 37 °C, 200rpm. Cells were transferred into 300ml of minimal media and grown for 6-9 hours to reach an OD600 of 5-10.

A bioreactor was prepared with 2.7L of minimal media + glucose and 300 ml of OD600 of 5-10 culture was added to bring the starting volume to 3L. Cells were grown at 28°C, pH7 with Dissolved Oxygen maintained at 20% saturation using a cascade containing agitation, air and oxygen. pH was controlled using 28% w/w Ammonium hydroxide solution. Fermentation was run in a fed-batch mode using a DO-stat based feeding algorithm once the initial bolus of 40g/L was depleted around 13hours. After 24-26hours of initial growth, the OD600 reached above 100. At this point, 300mL of 500g/L sucrose was added and temperature was reduced to 25C. High density culture was induced for protein production using 1mM IPTG. Fermentation was continued for another 20-24 hours and cells were harvested using a bench top centrifuge at 9000 ref, 15C for 60 minutes. Cell pellet recovered from centrifugation was resuspended in a buffer containing 0.5M NaCl and 0.1M KH2PO4 at pH8 in a weight by weight ratio of 2x buffer to 1x cells.

The harvested cells were disrupted in a homogenizer at 14,000 psi pressure in 2 passes. Resulting slurry contained the collagen protein along with other proteins.

The collagen was purified by acid treatment of whole cells recovered from bioreactor after centrifugation and resuspension in a buffer as described above. The pH of either the homogenized slurry was decreased to 3 using 6M Hydrochloric acid. Acidified cell slurry was incubated overnight at 4° C with mixing, followed by centrifugation. Supernatant of the acidified slurry was tested on a polyacrylamide gel and found to contain collagen in relatively high abundance compared to starting pellet. The collagen slurry thus obtained was high in salts. To obtain volume and salt reduction, concentration and diafiltration steps were performed using an EMD Millipore Tangential Flow Filtration system with ultrafiltration cassettes of 0.1 m² each. Total area of filtration was 0.2 m² using 2 cassettes in parallel. A volume reduction of 5x and a salt reduction of 19x was achieved in the TFF stage. Final collagen slurry was run on an SDS-PAGE gel to confirm presence of the collagen. This slurry was dried using a multi-tray lyophilizer over 3 days to obtain a white, fluffy collagen powder.

The purified collagen was analyzed on an SDS-PAGE gel and was observed to run at an apparent molecular weight of 35 kilodaltons. The 35 kilodalton band does not correspond to the expected size of 22 kilodaltons. The upshift between the expected size and the apparent size is thought to be due to the GDK repeats interacting with the gel matrix. The 35 kDa band was confirmed by mass spectrometry to be the correct collagen with the GDK repeats.

### Truncated Collagen with DsbA secretion tag-His tag-Linker-Thrombin cleavage site and GFP Beta-lactamase fusion (version 1):

A jellyfish collagen with DsbA secretion tag-His tag-Linker-Thrombin cleavage site and GFP Beta-lactamase fusion is disclosed below. The codon-optimized nucleotide sequence encoding this collagen is provided in SEQ ID NO: 17. The amino acid sequence is disclosed in SEQ ID NO: 18. The DsbA secretion tag is encoded by nucleotides 1-72 and encodes amino acids 1-24. The His tag is encoded by nucleotides 73-99 and encodes a 9 histidine tag of amino acids 25-33. The linker is encoded by nucleotides 100-111 and encodes amino acids 34-37. The thrombin cleavage side is encoded by nucleotides 112-135 and encodes amino acids 38-45. The green fluorescent protein (GFP) with linker is encoded by nucleotides 136-873 and encodes amino acids 46-291. The truncated collagen sequence is encoded by nucleotides 874-1440 and encodes amino acids 292-480. The Beta-lactamase with linker is encoded by nucleotides 1441-2232 and encodes amino acids 481-744. The Beta-lactamase was properly targeted to the periplasmic space even though the polypeptide did not have an independent secretion tag. The DsbA secretion tag directed the entire transcript (Truncated Collagen with DsbA secretion tag-His tag-Linker-Thrombin cleavage site and GFP Beta-lactamase fusion protein) to the periplasmic space and the Beta-lactamase functioned properly.

ATGAAAAAGATTTGGCTGGCGCTGGCTGGTTTAGTTTTAGCGTTTAGCGCA
TCGGCGGCGCAGTATGAAGATCACCATCACCACCACCACCATCACCACTC
TGGCTCGAGCCTGGTGCCGCGCGGCAGCCATATGTCTGGCTCGAGCAGTA
AAGGTGAAGAACTGTTCACCGGTGTTGTTCCGATCCTGGTTGAACTGGAT
GGTGATGTTAACGGCCACAAATTCTCTGTTCGTGGTGAAGGTGAAGGTGA
TGCAACCAACGGTAAACTGACCCTGAAATTCATCTGCACTACCGGTAAAC
TGCCGGTTCCATGGCCGACTCTGGTGACTACCCTGACCTATGGTGTTCAGT
GTTTTTCTCGTTACCCGGATCACATGAAGCAGCATGATTTCTTCAAATCTG
CAATGCCGGAAGGTTATGTACAGGAGCGCACCATTTCTTTCAAAGACGAT
GGCACCTACAAAACCCGTGCAGAGGTTAAATTTGAAGGTGATACTCTGGT
GAACCGTATTGAACTGAAAGGCATTGATTTCAAAGAGGACGGCAACATCC
TGGGCCACAAACTGGAATATAACTTCAACTCCCATAACGTTTACATCACC
GCAGACAAACAGAAGAACGGTATCAAAGCTAACTTCAAAATTCGCCATAA
CGTTGAAGACGGTAGCGTACAGCTGGCGGACCACTACCAGCAGAACACTC
CGATCGGTGATGGTCCGGTTCTGCTGCCGGATAACCACTACCTGTCCACCC
AGTCTaaaCTGTCCAAAGACCCGAACGAAAAGCGCGACCACATGGTGCTGC
TGGAGTTCGTTACTGCAGCAGGTATCACGCACGGCATGGATGAACTCTAC
AAATCTGGCGCGCCGGGCGGTCCGCAGGGTGTTGTTGGTGCAGATGGTAA
AGACGGTACCCCGGGTAATGCAGGTCAGAAAGGTCCGTCAGGTGAACCTG
GCAGCCCTGGTAAAGCAGGTAGTGCCGGTGAGCAGGGTCCGCCGGGCAA
AGATGGTAGTAATGGTGAGCCGGGTAGCCCTGGCAAAGAAGGTGAACGT
GGTCTGGCAGGACCGCCGGGTCCTGATGGTCGCCGCGGTGAAACGGGTTC
ACCGGGTATTGCCGGTGCCCTGGGTAAACCAGGTCTGGAAGGTCCGAAAG
GTTATCCTGGTCTGCGCGGTCGTGATGGTACCAATGGCAAACGTGGCGAA
CAGGGCGAAACCGGTCCAGATGGTGTTCGTGGTATTCCGGGTAACGATGG

TCAGAGCGGTAAACCGGGCATTGATGGTATTGATGGCACCAATGGTCAGC
CTGGCGAAGCAGGTTATCAGGGTGGTCGCGGTACCCGTGGTCAGCTGGGT
GAAACAGGTGATGTTGGTCAGAATGGTGATCGCGGCGCACCGGGTCCGGA
TGGTAGCAAAGGTAGCGCCGGTCGTCCGGGTTTACGTcacccagaaacgctggtgaaa
gtaaaagatgctgaagatcagttgggtgcacgagtgggttacatcgaactggatctcaacagcggtaagatccttgagagtt
ttcgccccgaagaacgttttccaatgatgagcacttttaaagttctgctatgtggcgcggtattatcccgtattgacgccgggc
aagagcaactcggtcgccgcatacactattctcagaatgacttggttgagtactcaccagtcacagaaaagcatcttacgga
tggcatgacagtaagagaattatgcagtgctgccataaccatgagtgataacactgcggccaacttacttctgacaacgatc
ggaggaccgaaggagctaaccgcttttttgcacaacatgggggatcatgtaactcgccttgatcgttgggaaccggagctg
aatgaagccataccaaacgacgagcgtgacaccacgatgcctgtagcaatggcaacaacgttgcgcaaactattaactgg
cgaactacttactctagcttcccggcaacaattaatagactggatggaggcggataaagttgcaggaccacttctgcgctcg
gcccttccggctggctggtttattgctgataaatctggagccggtgagcgtgggtctcgcggtatcattgcagcactggggc
cagatggtaagccctcccgtatcgtagttatctacacgacggggagtcaggcaactatggatgaacgaaatagacagatcg
ctgagataggtgcctcactgattaagcattggtaa (SEQ ID NO: 17)

MKKIWLALAGLVLAFSASAAQYEDHHHHHHHHHHSGSSLVPRGSHMSGSSSK
GEELFTGVVPILVELDGDVNGHKFSVRGEGEGDATNGKLTLKFICTTGKLPVP
WPTLVTTLTYGVQCFSRYPDHMKQHDFFKSAMPEGYVQERTISFKDDGTYK
TRAEVKFEGDTLVNRIELKGIDFKEDGNILGHKLEYNFNSHNVYITADKQKNG
IKANFKIRHNVEDGSVQLADHYQQNTPIGDGPVLLPDNHYLSTQSKLSKDPNE
KRDHMVLLEFVTAAGITHGMDELYKSGAPGGPQGVVGADGKDGTPGNAGQ
KGPSGEPGSPGKAGSAGEQGPPGKDGSNGEPGSPGKEGERGLAGPPGPDGRR
GETGSPGIAGALGKPGLEGPKGYPGLRGRDGTNGKRGEQGETGPDGVRGIPG
NDGQSGKPGIDGIDGTNGQPGEAGYQGGRGTRGQLGETGDVGQNGDRGAPG
PDGSKGSAGRPGLRHPETLVKVKDAEDQLGARVGYIELDLNSGKILESFRPEE
RFPMMSTFKVLLCGAVLSRIDAGQEQLGRRIHYSQNDLVEYSPVTEKHLTDG
MTVRELCSAAITMSDNTAANLLLTTIGGPKELTAFLHNMGDHVTRLDRWEPE
LNEAIPNDERDTTMPVAMATTLRKLLTGELLTLASRQQLIDWMEADKVAGPL
LRSALPAGWFIADKSGAGERGSRGIIAALGPDGKPSRIVVIYTTGSQATMDER
NRQIAEIGASLIKHW (SEQ ID NO: 18)

The polynucleotides of SEQ ID NO: 17 was constructed by assembling several DNA fragments. The collagen containing sequence was codon optimized and synthesized by Gen9 DNA, now Ginkgo Bioworks internal synthesis. The GFP was also synthesized by Gen9. The Beta-lactamase was cloned out of the plasmid pKD46 (http://cgsc2.biology.yale.edu/Strain.php?ID=68099) using PCR with the enzyme PrimeStar GXL polymerase (http://www.clontech.com/US/Products/PCR/GC_Rich/PrimeSTAR_GXL_DNA_Pol ymerase?sitex=10020:22372:US). Overlaps between the pET28 vector, GFP, Collagen, and Beta-lactamase was designed to be between 30 and 40bp long and added using PCR with the enzyme PrimeStar GXL polymerase. The opened pET28a vector and inserts were then assembled together into the final plasmid using SGI Gibson assembly (https://us.vwr.com/store/product/17613857/gibson-assembly-hifi-1-step-kit-synthetic-genomics-inc). Sequence of plasmid was then verified through sanger sequencing through Eurofins Genomics (www.eurofinsgenomics.com).

The transformed cells were cultivated in minimal media and frozen in 1.5 aliquots with glycerol at a ratio of 50:50 of cells to glycerol. One vial of this frozen culture was revived in 50ml of minimal media overnight at 37 °C, 200rpm. Cells were transferred into 300ml of minimal media and grown for 6-9 hours to reach an OD600 of 5-10.

A bioreactor was prepared with 2.7L of minimal media + glucose and 300 ml of OD600 of 5-10 culture was added to bring the starting volume to 3L. Cells were grown at 28°C, pH7 with Dissolved Oxygen maintained at 20% saturation using a cascade containing agitation, air and oxygen. pH was controlled using 28% w/w Ammonium hydroxide solution. Fermentation was run in a fed-batch mode using a DO-stat based feeding algorithm once the initial bolus of 40g/L was depleted around 13hours. After 24-26hours of initial growth, the OD600 reached above 100. At this point, 300mL of 500g/L sucrose was added and temperature was reduced to 25C. High density culture was induced for protein production using 1mM IPTG. Fermentation was continued for another 20-24 hours and cells were harvested using a bench top centrifuge at 9000 ref, 15C for 60 minutes. Cell pellet recovered from centrifugation was resuspended in a buffer containing 0.5M NaCl and 0.1M KH2PO4 at pH8 in a weight by weight ratio of 2x buffer to 1x cells.

The harvested cells were disrupted in a homogenizer at 14,000 psi pressure in 2 passes. Resulting slurry contained the collagen protein along with other proteins.

The collagen was purified by acid treatment of non-homogenized whole cells recovered from the bioreactor after centrifugation and resuspension in the buffer described above. The pH of the resuspended suspension-was decreased to 3 using 6M Hydrochloric acid. Acidified cell slurry was incubated overnight at 4° C with mixing, followed by centrifugation. The pH was then raised to 9 using 10N NaOH and the supernatant of the slurry was tested on a polyacrylamide gel and found to contain collagen in relatively high abundance compared to starting pellet. The collagen slurry thus obtained was high in salts. To obtain volume and salt reduction, concentration and diafiltration steps were performed using an EMD Millipore Tangential Flow Filtration system with ultrafiltration cassettes of 0.1 m2 each. Total area of filtration was 0.2 m2 using 2 cassettes in parallel. A volume reduction of 5x and a salt reduction of 19x was achieved in the TFF stage. Final collagen slurry was run on an SDS-PAGE gel to confirm presence of the collagen. This slurry was dried using a multi-tray lyophilizer over 3 days to obtain a white, fluffy collagen powder.

The purified collagen-GFP-Beta-lactamase fusion protein was analyzed on an SDS-PAGE gel and was observed to run at an apparent molecular weight of 90 kilodaltons. The expected size of the fusion protein is 85 kd. The 90 kDa band was confirmed by mass spectrometry to be the correct collagen fusion protein. Truncated Collagen with DsbA secretion tag-His tag-Linker-Thrombin cleavage site and GFP Beta-lactamase fusion (version 2):

A jellyfish collagen with DsbA secretion tag-His tag-Linker-Thrombin cleavage site and GFP Beta-lactamase fusion is disclosed below. The codon-optimized nucleotide sequence encoding this collagen is provided in SEQ ID NO: 19. The amino acid sequence is disclosed in SEQ ID NO: 20. The DsbA secretion tag is encoded by nucleotides 1-72 and encodes amino acids 1-24. The His tag is encoded by nucleotides 73-99 and encodes a 9 histidine tag of amino acids 25-33. The linker is encoded by nucleotides 100-111 and encodes amino acids 34-37. The thrombin cleavage side is encoded by nucleotides 112-135 and encodes amino acids 38-45. The green fluorescent protein (GFP) with linker is encoded by nucleotides 136-873 and encodes amino acids 46-291 The truncated collagen sequence is encoded by nucleotides 874-1440 and encodes amino acids 292-480. The Beta-lactamase with linker is encoded by nucleotides 1441-2232 and encodes amino acids 481-744.

ATGAAAAAGATTTGGCTGGCGCTGGCTGGTTTAGTTTTAGCGTTTAGCGCA
TCGGCGGCGCAGTATGAAGATCACCATCACCACCACCATCACCACTC
TGGCTCGAGCCTGGTGCCGCGCGGCAGCCATATGTCTGGCTCGAGCAGTA
AAGGTGAAGAACTGTTCACCGGTGTTGTTCCGATCCTGGTTGAACTGGAT
GGTGATGTTAACGGCCACAAATTCTCTGTTCGTGGTGAAGGTGAAGGTGA
TGCAACCAACGGTAAACTGACCCTGAAATTCATCTGCACTACCGGTAAAC
TGCCGGTTCCATGGCCGACTCTGGTGACTACCCTGACCTATGGTGTTCAGT

GTTTTTCTCGTTACCCGGATCACATGAAGCAGCATGATTTCTTCAAATCTG
CAATGCCGGAAGGTTATGTACAGGAGCGCACCATTTCTTTCAAAGACGAT
GGCACCTACAAAACCCGTGCAGAGGTTAAATTTGAAGGTGATACTCTGGT
GAACCGTATTGAACTGAAAGGCATTGATTTCAAAGAGGACGGCAACATCC
TGGGCCACAAACTGGAATATAACTTCAACTCCCATAACGTTTACATCACC
GCAGACAAACAGAAGAACGGTATCAAAGCTAACTTCAAAATTCGCCATAA
CGTTGAAGACGGTAGCGTACAGCTGGCGGACCACTACCAGCAGAACACTC
CGATCGGTGATGGTCCGGTTCTGCTGCCGGATAACCACTACCTGTCCACCC
AGTCTaaaCTGTCCAAAGACCCGAACGAAAAGCGCGACCACATGGTGCTGC
TGGAGTTCGTTACTGCAGCAGGTATCACGCACGGCATGGATGAACTCTAC
AAATCTGGCGCGCCGGGCGGTCCGCAGGGTGTTGTTGGTGCAGATGGTAA
AGACGGTACCCCGGGTAATGCAGGTCAGAAAGGTCCGTCAGGTGAACCTG
GCAGCCCTGGTAAAGCAGGTAGTGCCGGTGAGCAGGGTCCGCCGGGCAA
AGATGGTAGTAATGGTGAGCCGGGTAGCCCTGGCAAAGAAGGTGAACGT
GGTCTGGCAGGACCGCCGGGTCCTGATGGTCGCCGCGGTGAAACGGGTTC
ACCGGGTATTGCCGGTGCCCTGGGTAAACCAGGTCTGGAAGGTCCGAAAG
GTTATCCTGGTCTGCGCGGTCGTGATGGTACCAATGGCAAACGTGGCGAA
CAGGGCGAAACCGGTCCAGATGGTGTTCGTGGTATTCCGGGTAACGATGG
TCAGAGCGGTAAACCGGGCATTGATGGTATTGATGGCACCAATGGTCAGC
CTGGCGAAGCAGGTTATCAGGGTGGTCGCGGTACCCGTGGTCAGCTGGGT
GAAACAGGTGATGTTGGTCAGAATGGTGATCGCGGCGCACCGGGTCCGGA
TGGTAGCAAAGGTAGCGCCGGTCGTCCGGGTTTACGTcacccagaaacgctggtgaaa
gtaaaagatgctgaagatcagttgggtgcacgagtgggttacatcgaactggatctcaacagcggtaagatccttgagagtt
ttcgccccgaagaacgttttccaatgatgagcacttttaaagttctgctatgtggcgcggtattatcccgtattgacgccgggc
aagagcaactcggtcgccgcatacactattctcagaatgacttggttgagtactcaccagtcacagaaaagcatcttacgga
tggcatgacagtaagagaattatgcagtgctgccataaccatgagtgataacactgcggccaacttacttctgacaacgatc
ggaggaccgaaggagctaaccgcttttttgcacaacatgggggatcatgtaactcgccttgatcgttgggaaccggagctg
aatgaagccataccaaacgacgagcgtgacaccacgatgcctgtagcaatggcaacaacgttgcgcaaactattaactgg
cgaactacttactctagcttcccggcaacaattaatagactggatggaggcggataaagttgcaggaccacttctgcgctcg
gcccttccggctggctggtttattgctgataaatctggagccggtgagcgtgggtctcgcggtatcattgcagcactggggc
cagatggtaagccctcccgtatcgtagttatctacacgacggggagtcaggcaactatggatgaacgaaatagacagatcg
ctgagataggtgcctcactgattaagcattggtaa (SEQ ID NO: 19)

MKKIWLALAGLVLAFSASAAQYEDHHHHHHHHHSGSSLVPRGSHMSGSSSK
GEELFTGVVPILVELDGDVNGHKFSVRGEGEGDATNGKLTLKFICTTGKLPVP

WPTLVTTLTYGVQCFSRYPDHMKQHDFFKSAMPEGYVQERTISFKDDGTYK
TRAEVKFEGDTLVNRIELKGIDFKEDGNILGHKLEYNFNSHNVYITADKQKNG
IKANFKIRHNVEDGSVQLADHYQQNTPIGDGPVLLPDNHYLSTQSKLSKDPNE
KRDHMVLLEFVTAAGITHGMDELYKSGAPGGPQGVVGADGKDGTPGNAGQ
KGPSGEPGSPGKAGSAGEQGPPGKDGSNGEPGSPGKEGERGLAGPPGPDGRR
GETGSPGIAGALGKPGLEGPKGYPGLRGRDGTNGKRGEQGETGPDGVRGIPG
NDGQSGKPGIDGIDGTNGQPGEAGYQGGRGTRGQLGETGDVGQNGDRGAPG
PDGSKGSAGRPGLRHPETLVKVKDAEDQLGARVGYIELDLNSGKILESFRPEE
RFPMMSTFKVLLCGAVLSRIDAGQEQLGRRIHYSQNDLVEYSPVTEKHLTDG
MTVRELCSAAITMSDNTAANLLLTTIGGPKELTAFLHNMGDHVTRLDRWEPE
LNEAIPNDERDTTMPVAMATTLRKLLTGELLTLASRQQLIDWMEADKVAGPL
LRSALPAGWFIADKSGAGERGSRGIIAALGPDGKPSRIVVIYTTGSQATMDER
NRQIAEIGASLIKHW (SEQ ID NO: 20)

### Example 4: Production of Full-Length Elastin

Full length human elastin were expressed as described below. The wild-type, full length amino acid sequence of human elastin is provided below. 
MAGLTAAAPRPGVLLLLLSILHPSRPGGVPGAIPGGVPGGVFYPGAGLGALG
GGALGPGGKPLKPVPGGLAGAGLGAGLGAFPAVTFPGALVPGGVADAAAAY
KAAKAGAGLGGVPGVGGLGVSAGAVVPQPGAGVKPGKVPGVGLPGVYPGG
VLPGARFPGVGVLPGVPTGAGVKPKAPGVGGAFAGIPGVGPFGGPQPGVPLG
YPIKAPKLPGGYGLPYTTGKLPYGYGPGGVAGAAGKAGYPTGTGVGPQAAA
AAAAKAAAKFGAGAAGVLPGVGGAGVPGVPGAIPGIGGIAGVGTPAAAAA
AAAAKAAKYGAAAGLVPGGPGFGPGVVGVPGAGVPGVGVPGAGIPVVPGA
GIPGAAVPGVVSPEAAAKAAAKAAKYGARPGVGVGGIPTYGVGAGGFPGFG
VGVGGIPGVAGVPGVGGVPGVGGVPGVGISPEAQAAAAKAAKYGAAGAG
VLGGLVPGPQAAVPGVPGTGGVPGVGTPAAAAAKAAAKAAQFGLVPGVGV
APGVGVAPGVGVAPGVGLAPGVGVAPGVGVAPGVGVAPGIGPGGVAAAAK
SAAKVAAKAQLRAAAGLGAGIPGLGVGVGVPGLGVGAGVPGLGVGAGVPG
FGAGADEGVRRSLSPELREGDPSSSQHLPSTPSSPRVPGALAAAKAAKYGAA
VPGVLGGLGALGGVGIPGGVVGAGPAAAAAAAKAAAKAAQFGLVGAAGLG
GLGVGGLGVPGVGGLGGIPPAAAAKAAKYGAAGLGGVLGGAGQFPLGGVA
ARPGFGLSPIFPGGACLGKACGRKRK (SEQ ID NO: 21)
 http://www.uniprot.org/uniprot/P15502

The non-codon optimized polynucleotide sequence encoding the full length elastin is disclosed below. In SEQ ID NO: 22, nucleotides 1-78 encode the DsbA secretion tag and nucleotides 79-2358 encode the full length human elastin.

ATGGCGGGTCTGACGGCGGCGGCCCCGCGGCCCGGAGTCCTCCTGCTCCT
GCTGTCCATCCTCCACCCCTCTCGGCCTGGAGGGGTCCCTGGGGCCATTCC
TGGTGGAGTTCCTGGAGGAGTCTTTATCCAGGGGCTGGTCTCGGAGCCCT
TGGAGGAGGAGCGCTGGGGCCTGGAGGCAAACCTCTTAAGCCAGTTCCCG
GAGGGCTTGCGGGTGCTGGCCTTGGGGCAGGGCTCGGCGCCTTCCCCGCA
GTTACCTTTCCGGGGGCTCTGGTGCCTGGTGGAGTGGCTGACGCTGCTGCA
GCCTATAAAGCTGCTAAGGCTGGCGCTGGGCTTGGTGGTGTCCCAGGAGT
TGGTGGCTTAGGAGTGTCTGCAGGTGCGGTGGTTCCTCAGCCTGGAGCCG
GAGTGAAGCCTGGGAAAGTGCCGGGTGTGGGGCTGCCAGGTGTATACCCA
GGTGGCGTGCTCCCAGGAGCTCGGTTCCCCGGTGTGGGGGTGCTCCCTGG
AGTTCCCACTGGAGCAGGAGTTAAGCCCAAGGCTCCAGGTGTAGGTGGAG
CTTTTGCTGGAATCCCAGGAGTTGGACCCTTTGGGGGACCGCAACCTGGA
GTCCCACTGGGGTATCCCATCAAGGCCCCCAAGCTGCCTGGTGGCTATGG
ACTGCCCTACACCACAGGGAAACTGCCCTATGGCTATGGGCCCGGAGGAG
TGGCTGGTGCAGCGGGCAAGGCTGGTTACCCAACAGGGACAGGGGTTGGC
CCCCAGGCAGCAGCAGCAGCGGCAGCTAAAGCAGCAGCAAAGTTCGGTG
CTGGAGCAGCCGGAGTCCTCCCTGGTGTTGGAGGGGCTGGTGTTCCTGGC
GTGCCTGGGGCAATTCCTGGAATTGGAGGCATCGCAGGCGTTGGGACTCC
AGCTGCAGCTGCAGCTGCAGCAGCAGCCGCTAAGGCAGCCAAGTATGGA
GCTGCTGCAGGCTTAGTGCCTGGTGGGCCAGGCTTTGGCCCGGGAGTAGT
TGGTGTCCCAGGAGCTGGCGTTCCAGGTGTTGGTGTCCCAGGAGCTGGGA
TTCCAGTTGTCCCAGGTGCTGGGATCCCAGGTGCTGCGGTTCCAGGGGTTG
TGTCACCAGAAGCAGCTGCTAAGGCAGCTGCAAAGGCAGCCAAATACGG
GGCCAGGCCCGGAGTCGGAGTTGGAGGCATTCCTACTTACGGGGTTGGAG
CTGGGGGCTTTCCCGGCTTTGGTGTCGGAGTCGGAGGTATCCCTGGAGTCG
CAGGTGTCCCTGGTGTCGGAGGTGTTCCCGGAGTCGGAGGTGTCCCGGGA
GTTGGCATTTCCCCCGAAGCTCAGGCAGCAGCTGCCGCCAAGGCTGCCAA
GTACGGTGCTGCAGGAGCAGGAGTGCTGGGTGGGCTAGTGCCAGGTCCCC
AGGCGGCAGTCCCAGGTGTGCCGGGCACGGGAGGAGTGCCAGGAGTGGG
GACCCCAGCAGCTGCAGCTGCTAAAGCAGCCGCCAAAGCCGCCCAGTTTG
GGTTAGTTCCTGGTGTCGGCGTGGCTCCTGGAGTTGGCGTGGCTCCTGGTG

TCGGTGTGGCTCCTGGAGTTGGCTTGGCTCCTGGAGTTGGCGTGGCTCCTG
GAGTTGGTGTGGCTCCTGGCGTTGGCGTGGCTCCCGGCATTGGCCCTGGTG
GAGTTGCAGCTGCAGCAAAATCCGCTGCCAAGGTGGCTGCCAAAGCCCAG
CTCCGAGCTGCAGCTGGGCTTGGTGCTGGCATCCCTGGACTTGGAGTTGGT
GTCGGCGTCCCTGGACTTGGAGTTGGTGCTGGTGTTCCTGGACTTGGAGTT
GGTGCTGGTGTTCCTGGCTTCGGGGCAGGTGCAGATGAGGGAGTTAGGCG
GAGCCTGTCCCCTGAGCTCAGGGAAGGAGATCCCTCCTCCTCTCAGCACC
TCCCCAGCACCCCCTCATCACCCAGGGTACCTGGAGCCCTGGCTGCCGCT
AAAGCAGCCAAATATGGAGCAGCAGTGCCTGGGGTCCTTGGAGGGCTCGG
GGCTCTCGGTGGAGTAGGCATCCCAGGCGGTGTGGTGGGAGCCGGACCCG
CCGCCGCCGCTGCCGCAGCCAAAGCTGCTGCCAAAGCCGCCCAGTTTGGC
CTAGTGGGAGCCGCTGGGCTCGGAGGACTCGGAGTCGGAGGGCTTGGAGT
TCCAGGTGTTGGGGGCCTTGGAGGTATACCTCCAGCTGCAGCCGCTAAAG
CAGCTAAATACGGTGCTGCTGGCCTTGGAGGTGTCCTAGGGGGTGCCGGG
CAGTTCCCACTTGGAGGAGTGGCAGCAAGACCTGGCTTCGGATTGTCTCC
CATTTTCCCAGGTGGGGCCTGCCTGGGGAAAGCTTGTGGCCGGAAGAGAA
AATGA (SEQ ID NO: 22)

### Codon optimized Elastin with DsbA secretion tag-His tag-Linker-Thrombin cleavage site

The codon optimized polynucleotide sequence encoding the full length human elastin with DsbA secretion tag-His tag-Linker-Thrombin cleavage site is disclosed below. In SEQ ID NO: 23: nucleotides 1-72 encode the DsbA secretion tag encoding amino acids 1-24 of SEQ ID NO: 24; nucleotides 73-99 encode the 9 His tag encoding amino acids 25-33 of SEQ ID NO: 24; nucleotides 100-111 encode the linker encoding amino acids 34-37 of SEQ ID NO: 24; nucleotides 112-135 encode the thrombin cleavage tag encoding amino acids 38-45 of SEQ ID NO: 24; nucleotides 136-2415 encode the amino acids 46-805 of the full length human elastin of SEQ ID NO: 24.

ATGAAAAAGATTTGGCTGGCGCTGGCTGGTTTAGTTTTAGCGTTTAGCGCA
TCGGCGGCGCAGTATGAAGATCACCATCACCACCACCATCACCACTC
TGGCTCGAGCCTGGTGCCGCGCGGCAGCCATATGGGTGGCGTACCAGGCG
CAATTCCTGGGGGTGTCCCAGGCGGTGTTTTTATCCGGGCGCCGGTCTTG
GCGCACTGGGTGGCGGTGCACTGGGCCCGGGCGGCAAACCGCTGAAACC
GGTACCAGGTGGTTTAGCAGGCGCCGGCTTAGGCGCAGGTCTGGGAGCAT

TTCCGGCAGTTACCTTTCCAGGGGCACTGGTTCCTGGAGGTGTGGCCGATG
CAGCCGCGGCATATAAAGCCGCTAAAGCCGGTGCGGGTTTAGGAGGCGTC
CCAGGTGTCGGTGGCCTGGGTGTTAGCGCCGGTGCAGTTGTTCCGCAGCC
GGGAGCAGGGGTTAAACCTGGTAAAGTGCCGGGAGTAGGTCTGCCAGGC
GTTTATCCTGGTGGTGTTTTGCCGGGTGCCCGTTTTCCGGGCGTTGGTGTTC
TTCCAGGCGTGCCGACCGGAGCCGGTGTTAAACCGAAAGCCCCGGTGTT
GGAGGTGCATTTGCAGGCATCCCGGGAGTTGGCCCGTTTGGTGGTCCGCA
ACCTGGGGTTCCGTTAGGTTATCCGATTAAAGCACCGAAACTGCCCGGCG
GTTATGGTCTGCCGTACACAACCGGTAAACTGCCGTATGGTTATGGCCCG
GGTGGAGTTGCGGGTGCAGCAGGTAAAGCGGGTTATCCTACCGGAACCGG
TGTAGGTCCGCAGGCCGCTGCTGCCGCCGCCGCAAAAGCAGCGGCTAAAT
TTGGCGCCGGAGCAGCGGGTGTTCTGCCTGGAGTTGGTGGTGCGGGCGTG
CCAGGGGTACCTGGTGCAATTCCGGGTATTGGTGGTATTGCCGGTGTCGG
CACCCCGGCCGCGGCAGCTGCGGCAGCGGCGGCTGCCAAAGCTGCTAAAT
ACGGTGCCGCGGCGGGTCTGGTGCCAGGAGGTCCGGGTTTTGGTCCGGGA
GTGGTTGGCGTGCCTGGCGCAGGCGTTCCTGGTGTGGGCGTTCCAGGTGC
AGGGATTCCTGTTGTGCCTGGTGCCGGTATTCCCGGCGCGGCCGTTCCGGG
GGTGGTTAGCCCGGAAGCCGCAGCGAAGGCTGCGGCAAAGGCAGCAAAG
TATGGCGCACGCCCAGGAGTCGGCGTGGGTGGTATCCCGACCTATGGGGT
GGGCGCAGGGGGTTTTCCTGGTTTCGGCGTAGGTGTAGGAGGTATACCGG
GCGTGGCCGGTGTACCAGGGGTTGGTGGCGTCCCTGGTGTTGGCGGTGTG
CCAGGTGTTGGTATTTCACCGGAAGCACAGGCAGCAGCCGCAGCTAAGGC
AGCGAAATATGGTGCCGCCGGCGCAGGAGTTTTAGGTGGGCTGGTTCCGG
GCCCGCAGGCAGCTGTGCCGGGGGTTCCAGGCACCGGTGGTGTCCCTGGA
GTCGGTACGCCGGCTGCAGCGGCAGCCAAAGCGGCTGCGAAAGCAGCAC
AGTTTGGCTTAGTACCGGGTGTGGGAGTTGCCCCCGGCGTTGGCGTTGCTC
CAGGGGTGGGTGTTGCTCCTGGCGTCGGTCTGGCTCCTGGAGTGGGCGTA
GCACCCGGTGTGGGGGTGGCCCCGGGTGTTGGGGTTGCACCGGGTATCGG
TCCGGGCGGTGTCGCAGCAGCAGCTAAAAGCGCGGCGAAAGTTGCGGCC
AAAGCCCAACTGCGCGCCGCCGCGGGCCTCGGTGCAGGTATTCCGGGGCT
GGGTGTCGGAGTTGGAGTCCCGGGTTTGGGCGTGGGCGCGGGAGTTCCGG
GACTGGGAGTGGGTGCCGGAGTTCCTGGCTTTGGTGCAGGCGCAGATGAA
GGTGTTCGTCGTAGCCTGAGTCCGGAACTGCGTGAAGGTGATCCGAGTAG
CAGCCAGCATCTGCCGAGCACCCCGAGCAGCCCGCGTGTTCCGGGTGCAT

TAGCTGCAGCAAAAGCCGCCAAGTATGGTGCAGCCGTGCCGGGCGTCTTA
GGTGGTCTGGGCGCCCTGGGTGGTGTAGGCATTCCGGGAGGTGTTGTGGG
TGCAGGACCGGCCGCCGCAGCTGCGGCCGCCAAAGCAGCTGCAAAAGCG
GCCCAGTTTGGTTTAGTGGGCGCCGCAGGTTTAGGCGGTTTAGGTGTGGGT
GGACTGGGTGTACCTGGCGTAGGCGGTCTGGGTGGAATTCCGCCCGCAGC
GGCCGCGAAAGCGGCAAAATATGGCGCGGCAGGCCTGGGCGGCGTGCTG
GGTGGGGCAGGTCAGTTTCCGCTGGGCGGGGTTGCCGCACGTCCGGGATT
TGGTCTGAGCCCGATTTTCCCTGGCGGCGCATGTCTGGGTAAAGCATGTGG
TCGTAAACGTAAAtaa (SEQ ID NO: 23)

MKKIWLALAGLVLAFSASAAQYEDHHHHHHHHHSGSSLVPRGSHMGGVPG
AIPGGVPGGVFYPGAGLGALGGGALGPGGKPLKPVPGGLAGAGLGAGLGAF
PAVTFPGALVPGGVADAAAAYKAAKAGAGLGGVPGVGGLGVSAGAVVPQP
GAGVKPGKVPGVGLPGVYPGGVLPGARFPGVGVLPGVPTGAGVKPKAPGVG
GAFAGIPGVGPFGGPQPGVPLGYPIKAPKLPGGYGLPYTTGKLPYGYGPGGV
AGAAGKAGYPTGTGVGPQAAAAAAKAAAKFGAGAAGVLPGVGGAGVPG
VPGAIPGIGGIAGVGTPAAAAAAAAAKAAKYGAAAGLVPGGPGFGPGVVG
VPGAGVPGVGVPGAGIPVVPGAGIPGAAVPGVVSPEAAAKAAAKAAKYGAR
PGVGVGGIPTYGVGAGGFPGFGVGVGGIPGVAGVPGVGGVPGVGGVPGVGI
SPEAQAAAAAKAAKYGAAGAGVLGGLVPGPQAAVPGVPGTGGVPGVGTPA
AAAAKAAAKAAQFGLVPGVGVAPGVGVAPGVGVAPGVGLAPGVGVAPGV
GVAPGVGVAPGIGPGGVAAAAKSAAKVAAKAQLRAAAGLGAGIPGLGVGV
GVPGLGVGAGVPGLGVGAGVPGFGAGADEGVRRSLSPELREGDPSSSQHLPS
TPSSPRVPGALAAAKAAKYGAAVPGVLGGLGALGGVGIPGGVVGAGPAAAA
AAAKAAAKAAQFGLVGAAGLGGLGVGGLGVPGVGGLGGIPPAAAAKAAKY
GAAGLGGVLGGAGQFPLGGVAARPGFGLSPIFPGGACLGKACGRKRK (SEQ
ID NO: 24)

The polynucleotide encoding the full length human elastin without the native sequence tag is disclosed in SEQ ID NO: 87.

GGTGGCGTACCAGGCGCAATTCCTGGGGGTGTCCCAGGCGGTGTTTTTAT
CCGGGCGCCGGTCTTGGCGCACTGGGTGGCGGTGCACTGGGCCCGGGCGG
CAAACCGCTGAAACCGGTACCAGGTGGTTTAGCAGGCGCCGGCTTAGGCG
CAGGTCTGGGAGCATTTCCGGCAGTTACCTTTCCAGGGGCACTGGTTCCTG
GAGGTGTGGCCGATGCAGCCGCGGCATATAAAGCCGCTAAAGCCGGTGCG
GGTTTAGGAGGCGTCCCAGGTGTCGGTGGCCTGGGTGTTAGCGCCGGTGC

AGTTGTTCCGCAGCCGGGAGCAGGGGTTAAACCTGGTAAAGTGCCGGGAG
TAGGTCTGCCAGGCGTTTATCCTGGTGGTGTTTTGCCGGGTGCCCGTTTTC
CGGGCGTTGGTGTTCTTCCAGGCGTGCCGACCGGAGCCGGTGTTAAACCG
AAAGCCCCCGGTGTTGGAGGTGCATTTGCAGGCATCCCGGGAGTTGGCCC
GTTTGGTGGTCCGCAACCTGGGGTTCCGTTAGGTTATCCGATTAAAGCACC
GAAACTGCCCGGCGGTTATGGTCTGCCGTACACAACCGGTAAACTGCCGT
ATGGTTATGGCCCGGGTGGAGTTGCGGGTGCAGCAGGTAAAGCGGGTTAT
CCTACCGGAACCGGTGTAGGTCCGCAGGCCGCTGCTGCCGCCGCCGCAAA
AGCAGCGGCTAAATTTGGCGCCGGAGCAGCGGGTGTTCTGCCTGGAGTTG
GTGGTGCGGGCGTGCCAGGGGTACCTGGTGCAATTCCGGGTATTGGTGGT
ATTGCCGGTGTCGGCACCCCGGCCGCGGCAGCTGCGGCAGCGGCGGCTGC
CAAAGCTGCTAAATACGGTGCCGCGGCGGGTCTGGTGCCAGGAGGTCCGG
GTTTTGGTCCGGGAGTGGTTGGCGTGCCTGGCGCAGGCGTTCCTGGTGTGG
GCGTTCCAGGTGCAGGGATTCCTGTTGTGCCTGGTGCCGGTATTCCCGGCG
CGGCCGTTCCGGGGGTGGTTAGCCCGGAAGCCGCAGCGAAGGCTGCGGC
AAAGGCAGCAAAGTATGGCGCACGCCCAGGAGTCGGCGTGGGTGGTATC
CCGACCTATGGGGTGGGCGCAGGGGGTTTTCCTGGTTTCGGCGTAGGTGT
AGGAGGTATACCGGGCGTGGCCGGTGTACCAGGGGTTGGTGGCGTCCCTG
GTGTTGGCGGTGTGCCAGGTGTTGGTATTTCACCGGAAGCACAGGCAGCA
GCCGCAGCTAAGGCAGCGAAATATGGTGCCGCCGGCGCAGGAGTTTTAGG
TGGGCTGGTTCCGGGCCCGCAGGCAGCTGTGCCGGGGGTTCCAGGCACCG
GTGGTGTCCCTGGAGTCGGTACGCCGGCTGCAGCGGCAGCCAAAGCGGCT
GCGAAAGCAGCACAGTTTGGCTTAGTACCGGGTGTGGGAGTTGCCCCCGG
CGTTGGCGTTGCTCCAGGGGTGGGTGTTGCTCCTGGCGTCGGTCTGGCTCC
TGGAGTGGGCGTAGCACCCGGTGTGGGGGTGGCCCCGGGTGTTGGGGTTG
CACCGGGTATCGGTCCGGGCGGTGTCGCAGCAGCAGCTAAAAGCGCGGC
GAAAGTTGCGGCCAAAGCCCAACTGCGCGCCGCCGCGGGCCTCGGTGCAG
GTATTCCGGGGCTGGGTGTCGGAGTTGGAGTCCCGGGTTTGGGCGTGGGC
GCGGGAGTTCCGGGACTGGGAGTGGGTGCCGGAGTTCCTGGCTTTGGTGC
AGGCGCAGATGAAGGTGTTCGTCGTAGCCTGAGTCCGGAACTGCGTGAAG
GTGATCCGAGTAGCAGCCAGCATCTGCCGAGCACCCCGAGCAGCCCGCGT
GTTCCGGGTGCATTAGCTGCAGCAAAAGCCGCCAAGTATGGTGCAGCCGT
GCCGGGCGTCTTAGGTGGTCTGGGCGCCCTGGGTGGTGTAGGCATTCCGG
GAGGTGTTGTGGGTGCAGGACCGGCCGCCGCAGCTGCGGCCGCCAAAGC

AGCTGCAAAAGCGGCCCAGTTTGGTTTAGTGGGCGCCGCAGGTTTAGGCG
GTTTAGGTGTGGGTGGACTGGGTGTACCTGGCGTAGGCGGTCTGGGTGGA
ATTCCGCCCGCAGCGGCCGCGAAAGCGGCAAAATATGGCGCGGCAGGCC
TGGGCGGCGTGCTGGGTGGGGCAGGTCAGTTTCCGCTGGGCGGGGTTGCC
GCACGTCCGGGATTTGGTCTGAGCCCGATTTTCCCTGGCGGCGCATGTCTG
GGTAAAGCATGTGGTCGTAAACGTAAAtaa (SEQ ID NO: 87)

The full length human elastin sequence without the native sequence tag is disclosed in SEQ ID NO: 88.

GGVPGAIPGGVPGGVFYPGAGLGALGGGALGPGGKPLKPVPGGLAGAGLGA
GLGAFPAVTFPGALVPGGVADAAAAYKAAKAGAGLGGVPGVGGLGVSAGA
VVPQPGAGVKPGKVPGVGLPGVYPGGVLPGARFPGVGVLPGVPTGAGVKPK
APGVGGAFAGIPGVGPFGGPQPGVPLGYPIKAPKLPGGYGLPYTTGKLPYGY
GPGGVAGAAGKAGYPTGTGVGPQAAAAAAKAAKFGAGAAGVLPGVGG
AGVPGVPGAIPGIGGIAGVGTPAAAAAAAAAKAAKYGAAAGLVPGGPGFG
PGVVGVPGAGVPGVGVPGAGIPVVPGAGIPGAAVPGVVSPEAAAKAAAKAA
KYGARPGVGVGGIPTYGVGAGGFPGFGVGVGGIPGVAGVPGVGGVPGVGGV
PGVGISPEAQAAAAAKAAKYGAAGAGVLGGLVPGPQAAVPGVPGTGGVPG
VGTPAAAAAKAAAKAAQFGLVPGVGVAPGVGVAPGVGVAPGVGLAPGVGV
APGVGVAPGVGVAPGIGPGGVAAAAKSAAKVAAKAQLRAAAGLGAGIPGL
GVGVGVPGLGVGAGVPGLGVGAGVPGFGAGADEGVRRSLSPELREGDPSSS
QHLPSTPSSPRVPGALAAAKAAKYGAAVPGVLGGLGALGGVGIPGGVVGAG
PAAAAAAAKAAAKAAQFGLVGAAGLGGLGVGGLGVPGVGGLGGIPPAAAA
KAAKYGAAGLGGVLGGAGQFPLGGVAARPGFGLSPIFPGGACLGKACGRKR
K (SEQ ID NO: 88)

### Codon optimized Elastin with DsbA secretion tag

The codon optimized polynucleotide sequence encoding the full length human elastin with a DsbA secretion tag is disclosed in SEQ ID NO: 25. In SEQ ID NO: 25: nucleotides 1-72 encode the DsbA secretion tag encoding amino acids 1-24 of SEQ ID NO: 26; nucleotides 73-2355 encode the amino acids 25-785 of the full length human elastin of SEQ ID NO: 26.

ATGAAAAAGATTTGGCTGGCGCTGGCTGGTTTAGTTTTAGCGTTTAGCGCA
TCGGCGGCGCAGTATGAAGATATGGGTGGCGTACCAGGCGCAATTCCTGG
GGGTGTCCCAGGCGGTGTTTTTTATCCGGGCGCCGGTCTTGGCGCACTGGG
TGGCGGTGCACTGGGCCCGGGCGGCAAACCGCTGAAACCGGTACCAGGT

GGTTTAGCAGGCGCCGGCTTAGGCGCAGGTCTGGAGCATTTCCGGCAGT
TACCTTTCCAGGGGCACTGGTTCCTGGAGGTGTGGCCGATGCAGCCGCGG
CATATAAAGCCGCTAAAGCCGGTGCGGGTTTAGGAGGCGTCCCAGGTGTC
GGTGGCCTGGGTGTTAGCGCCGGTGCAGTTGTTCCGCAGCCGGGAGCAGG
GGTTAAACCTGGTAAAGTGCCGGGAGTAGGTCTGCCAGGCGTTTATCCTG
GTGGTGTTTTGCCGGGTGCCCGTTTTCCGGGCGTTGGTGTTCTTCCAGGCG
TGCCGACCGGAGCCGGTGTTAAACCGAAAGCCCCCGGTGTTGGAGGTGCA
TTTGCAGGCATCCCGGGAGTTGGCCCGTTTGGTGGTCCGCAACCTGGGGTT
CCGTTAGGTTATCCGATTAAAGCACCGAAACTGCCCGGCGGTTATGGTCT
GCCGTACACAACCGGTAAACTGCCGTATGGTTATGGCCCGGGTGGAGTTG
CGGGTGCAGCAGGTAAAGCGGGTTATCCTACCGGAACCGGTGTAGGTCCG
CAGGCCGCTGCTGCCGCCGCCGCAAAAGCAGCGGCTAAATTGGCGCCGG
AGCAGCGGGTGTTCTGCCTGGAGTTGGTGGTGCGGGCGTGCCAGGGGTAC
CTGGTGCAATTCCGGGTATTGGTGGTATTGCCGGTGTCGGCACCCCGGCCG
CGGCAGCTGCGGCAGCGGCGGCTGCCAAAGCTGCTAAATACGGTGCCGCG
GCGGGTCTGGTGCCAGGAGGTCCGGGTTTTGGTCCGGGAGTGGTTGGCGT
GCCTGGCGCAGGCGTTCCTGGTGTGGGCGTTCCAGGTGCAGGGATTCCTG
TTGTGCCTGGTGCCGGTATTCCCGGCGCGGCCGTTCCGGGGGTGGTTAGCC
CGGAAGCCGCAGCGAAGGCTGCGGCAAAGGCAGCAAAGTATGGCGCACG
CCCAGGAGTCGGCGTGGGTGGTATCCCGACCTATGGGGTGGCGCAGGGG
GTTTTCCTGGTTTCGGCGTAGGTGTAGGAGGTATACCGGGCGTGGCCGGT
GTACCAGGGGTTGGTGGCGTCCCTGGTGTTGGCGGTGTGCCAGGTGTTGG
TATTTCACCGGAAGCACAGGCAGCAGCCGCAGCTAAGGCAGCGAAATATG
GTGCCGCCGGCGCAGGAGTTTTAGGTGGGCTGGTTCCGGGCCCGCAGGCA
GCTGTGCCGGGGGTTCCAGGCACCGGTGGTGTCCCTGGAGTCGGTACGCC
GGCTGCAGCGGCAGCCAAAGCGGCTGCGAAAGCAGCACAGTTTGGCTTAG
TACCGGGTGTGGGAGTTGCCCCCGGCGTTGGCGTTGCTCCAGGGGTGGGT
GTTGCTCCTGGCGTCGGTCTGGCTCCTGGAGTGGGCGTAGCACCCGGTGTG
GGGGTGGCCCCGGGTGTTGGGGTTGCACCGGGTATCGGTCCGGGCGGTGT
CGCAGCAGCAGCTAAAAGCGCGGCGAAAGTTGCGGCCAAAGCCCAACTG
CGCGCCGCCGCGGGCCTCGGTGCAGGTATTCCGGGGCTGGGTGTCGGAGT
TGGAGTCCCGGGTTTGGGCGTGGGCGCGGGAGTTCCGGGACTGGGAGTGG
GTGCCGGAGTTCCTGGCTTTGGTGCAGGCGCAGATGAAGGTGTTCGTCGT
AGCCTGAGTCCGGAACTGCGTGAAGGTGATCCGAGTAGCAGCCAGCATCT

GCCGAGCACCCCGAGCAGCCCGCGTGTTCCGGGTGCATTAGCTGCAGCAA
AAGCCGCCAAGTATGGTGCAGCCGTGCCGGGCGTCTTAGGTGGTCTGGGC
GCCCTGGGTGGTGTAGGCATTCCGGGAGGTGTTGTGGGTGCAGGACCGGC
CGCCGCAGCTGCGGCCGCCAAAGCAGCTGCAAAAGCGGCCCAGTTTGGTT
TAGTGGGCGCCGCAGGTTTAGGCGGTTTAGGTGTGGGTGGACTGGGTGTA
CCTGGCGTAGGCGGTCTGGGTGGAATTCCGCCCGCAGCGGCCGCGAAAGC
GGCAAAATATGGCGCGGCAGGCCTGGGCGGCGTGCTGGGTGGGGCAGGT
CAGTTTCCGCTGGGCGGGGTTGCCGCACGTCCGGGATTTGGTCTGAGCCC
GATTTTCCCTGGCGGCGCATGTCTGGGTAAAGCATGTGGTCGTAAACGTA
AAtaa (SEQ ID NO: 25)

MKKIWLALAGLVLAFSASAAQYEDMGGVPGAIPGGVPGGVFYPGAGLGALG
GGALGPGGKPLKPVPGGLAGAGLGAGLGAFPAVTFPGALVPGGVADAAAAY
KAAKAGAGLGGVPGVGGLGVSAGAVVPQPGAGVKPGKVPGVGLPGVYPGG
VLPGARFPGVGVLPGVPTGAGVKPKAPGVGGAFAGIPGVGPFGGPQPGVPLG
YPIKAPKLPGGYGLPYTTGKLPYGYGPGGVAGAAGKAGYPTGTGVGPQAAA
AAAAKAAAKFGAGAAGVLPGVGGAGVPGVPGAIPGIGGIAGVGTPAAAAAA
AAAAKAAKYGAAAGLVPGGPGFGPGVVGVPGAGVPGVGVPGAGIPVVPGA
GIPGAAVPGVVSPEAAAKAAAKAAKYGARPGVGVGGIPTYGVGAGGFPGFG
VGVGGIPGVAGVPGVGGVPGVGGVPGVGISPEAQAAAAAKAAKYGAAGAG
VLGGLVPGPQAAVPGVPGTGGVPGVGTPAAAAAKAAAKAAQFGLVPGVGV
APGVGVAPGVGVAPGVGLAPGVGVAPGVGVAPGVGVAPGIGPGGVAAAAK
SAAKVAAKAQLRAAAGLGAGIPGLGVGVGVPGLGVGAGVPGLGVGAGVPG
FGAGADEGVRRSLSPELREGDPSSSQHLPSTPSSPRVPGALAAAKAAKYGAA
VPGVLGGLGALGGVGIPGGVVGAGPAAAAAAAKAAAKAAQFGLVGAAGLG
GLGVGGLGVPGVGGLGGIPPAAAAKAAKYGAAGLGGVLGGAGQFPLGGVA
ARPGFGLSPIFPGGACLGKACGRKRK (SEQ ID NO: 26)

The polynucleotides of SEQ ID NO: 22 was codon optimized and synthesized by Gen9 DNA, now Ginkgo Bioworks internal synthesis. Overlaps between the pET28 vector and SEQ ID NO: 22 was designed to be between 30 and 40bp long and added using PCR with the enzyme PrimeStar GXL polymerase (http://www.clontech.com/US/Products/PCR/GC_Rich/PrimeSTAR_GXL_DNA_Pol ymerase?sitex=10020:22372:US). The opened pET28a vector and insert DNA (SEQ ID NO: 22) was then assembled together into the final plasmid using SGI Gibson assembly (https://us.vwr.com/store/product/17613857/gibson-assembly-hifi-1-step-kit-synthetic-genomics-inc). Sequence of plasmid was then verified through sanger sequencing through Eurofins Genomics (www.eurofinsgenomics.com).

The transformed cells were cultivated in minimal media and frozen in 1.5 aliquots with glycerol at a ratio of 50:50 of cells to glycerol. One vial of this frozen culture was revived in 50ml of minimal media overnight at 37 °C, 200rpm. Cells were transferred into 300ml of minimal media and grown for 6-9 hours to reach an OD600 of 5-10.

A bioreactor was prepared with 2.7L of minimal media + glucose and 300 ml of OD600 of 5-10 culture was added to bring the starting volume to 3L. Cells were grown at 28°C, pH7 with Dissolved Oxygen maintained at 20% saturation using a cascade containing agitation, air and oxygen. pH was controlled using 28% w/w Ammonium hydroxide solution. Fermentation was run in a fed-batch mode using a DO-stat based feeding algorithm once the initial bolus of 40g/L was depleted around 13hours. After 24-26hours of initial growth, the OD600 reached above 100. At this point, 300mL of 500g/L sucrose was added and temperature was reduced to 25C. High density culture was induced for protein production using 1mM IPTG. Fermentation was continued for another 20-24 hours and cells were harvested using a bench top centrifuge at 9000 ref, 15C for 60 minutes. Cell pellet recovered from centrifugation was resuspended in a buffer containing 0.5M NaCl and 0.1M KH2PO4 at pH8 in a weight by weight ratio of 2x buffer to 1x cells.

The fermentations were performed at various temperature ranging from 25° to 28° C. For some fermentations, the temperature of the fermentation was maintained at a constant temperature and immediately upon completion of fermentation (OD600 of 5-10) the elastin was purified. For other fermentations, the temperature of the fermentations is maintained for a desired period of time and when cell densities of OD600 of 5-10 are reached, the temperature is reduced to induce protein production. Typically, the temperature is reduced from 28° C to 25° C. After the fermentation at 25° C is continued for 40-60 hours, the elastin is isolated.

The harvested cells were disrupted in a homogenizer at 14,000 psi pressure in 2 passes. Resulting slurry contained the collagen protein along with other proteins.

The supernatant from the homogenized cells was analyzed on an SDS-PAGE gel and a clear band was observed at around 70 kilodaltons corresponding to the expected size of 68 kilodaltons. The purified elastin is analyzed by mass spectrom etry.

### Full Length Elastin with DsbA secretion tag-His tag-Linker-Thrombin cleavage site and GFP Beta-lactamase fusion

A human elastin with DsbA secretion tag-His tag-Linker-Thrombin cleavage site and GFP Beta-lactamase fusion is disclosed below. The codon-optimized nucleotide sequence encoding this elastin is provided in SEQ ID NO: 27. The amino acid sequence is disclosed in SEQ ID NO: 28. The DsbA secretion tag is encoded by nucleotides 1-72 and encodes amino acids 1-24. The His tag is encoded by nucleotides 73-99 and encodes a 9 histidine tag of amino acids 25-33. The linker is encoded by nucleotides 100-111 and encodes amino acids 34-37. The thrombin cleavage side is encoded by nucleotides 112-135 and encodes amino acids 38-45. The green fluorescent protein (GFP) with linker is encoded by nucleotides 136-873 and encodes amino acids 46-291. The full-length elastin sequence is encoded by nucleotides 874-3153 and encodes amino acids 292-1051. The Beta-lactamase with linker is encoded by nucleotides 3154-3945 and encodes amino acids 1052-1315.

ATGAAAAAGATTTGGCTGGCGCTGGCTGGTTTAGTTTTAGCGTTTAGCGCA
TCGGCGGCGCAGTATGAAGATCACCATCACCACCACCACCATCACCACTC
TGGCTCGAGCCTGGTGCCGCGCGGCAGCCATATGTCTGGCTCGAGCAGTA
AAGGTGAAGAACTGTTCACCGGTGTTGTTCCGATCCTGGTTGAACTGGAT
GGTGATGTTAACGGCCACAAATTCTCTGTTCGTGGTGAAGGTGAAGGTGA
TGCAACCAACGGTAAACTGACCCTGAAATTCATCTGCACTACCGGTAAAC
TGCCGGTTCCATGGCCGACTCTGGTGACTACCCTGACCTATGGTGTTCAGT
GTTTTTCTCGTTACCCGGATCACATGAAGCAGCATGATTTCTTCAAATCTG
CAATGCCGGAAGGTTATGTACAGGAGCGCACCATTTCTTTCAAAGACGAT
GGCACCTACAAAACCCGTGCAGAGGTTAAATTTGAAGGTGATACTCTGGT
GAACCGTATTGAACTGAAAGGCATTGATTTCAAAGAGGACGGCAACATCC
TGGGCCACAAACTGGAATATAACTTCAACTCCCATAACGTTTACATCACC
GCAGACAAACAGAAGAACGGTATCAAAGCTAACTTCAAAATTCGCCATAA
CGTTGAAGACGGTAGCGTACAGCTGGCGGACCACTACCAGCAGAACACTC
CGATCGGTGATGGTCCGGTTCTGCTGCCGGATAACCACTACCTGTCCACCC
AGTCTaaaCTGTCCAAAGACCCGAACGAAAAGCGCGACCACATGGTGCTGC
TGGAGTTCGTTACTGCAGCAGGTATCACGCACGGCATGGATGAACTCTAC
AAATCTGGCGCGCCGGGCGGTGGCGTACCAGGCGCAATTCCTGGGGGTGT

CCCAGGCGGTGTTTTTATCCGGGCGCCGGTCTTGGCGCACTGGGTGGCGG
TGCACTGGGCCCGGGCGGCAAACCGCTGAAACCGGTACCAGGTGGTTTAG
CAGGCGCCGGCTTAGGCGCAGGTCTGGGAGCATTTCCGGCAGTTACCTTT
CCAGGGGCACTGGTTCCTGGAGGTGTGGCCGATGCAGCCGCGGCATATAA
AGCCGCTAAAGCCGGTGCGGGTTTAGGAGGCGTCCCAGGTGTCGGTGGCC
TGGGTGTTAGCGCCGGTGCAGTTGTTCCGCAGCCGGGAGCAGGGGTTAAA
CCTGGTAAAGTGCCGGGAGTAGGTCTGCCAGGCGTTTATCCTGGTGGTGTT
TTGCCGGGTGCCCGTTTTCCGGGCGTTGGTGTTCTTCCAGGCGTGCCGACC
GGAGCCGGTGTTAAACCGAAAGCCCCCGGTGTTGGAGGTGCATTTGCAGG
CATCCCGGGAGTTGGCCCGTTTGGTGGTCCGCAACCTGGGGTTCCGTTAGG
TTATCCGATTAAAGCACCGAAACTGCCCGGCGGTTATGGTCTGCCGTACA
CAACCGGTAAACTGCCGTATGGTTATGGCCCGGGTGGAGTTGCGGGTGCA
GCAGGTAAAGCGGGTTATCCTACCGGAACCGGTGTAGGTCCGCAGGCCGC
TGCTGCCGCCGCCGCAAAAGCAGCGGCTAAATTTGGCGCCGGAGCAGCGG
GTGTTCTGCCTGGAGTTGGTGGTGCGGGCGTGCCAGGGGTACCTGGTGCA
ATTCCGGGTATTGGTGGTATTGCCGGTGTCGGCACCCCGGCCGCGGCAGC
TGCGGCAGCGGCGGCTGCCAAAGCTGCTAAATACGGTGCCGCGGCGGGTC
TGGTGCCAGGAGGTCCGGGTTTTGGTCCGGGAGTGGTTGGCGTGCCTGGC
GCAGGCGTTCCTGGTGTGGGCGTTCCAGGTGCAGGGATTCCTGTTGTGCCT
GGTGCCGGTATTCCCGGCGCGGCCGTTCCGGGGGTGGTTAGCCCGGAAGC
CGCAGCGAAGGCTGCGGCAAAGGCAGCAAAGTATGGCGCACGCCCAGGA
GTCGGCGTGGGTGGTATCCCGACCTATGGGGTGGGCGCAGGGGGTTTTCC
TGGTTTCGGCGTAGGTGTAGGAGGTATACCGGGCGTGGCCGGTGTACCAG
GGGTTGGTGGCGTCCCTGGTGTTGGCGGTGTGCCAGGTGTTGGTATTTCAC
CGGAAGCACAGGCAGCAGCCGCAGCTAAGGCAGCGAAATATGGTGCCGC
CGGCGCAGGAGTTTTAGGTGGGCTGGTTCCGGGCCCGCAGGCAGCTGTGC
CGGGGGTTCCAGGCACCGGTGGTGTCCCTGGAGTCGGTACGCCGGCTGCA
GCGGCAGCCAAAGCGGCTGCGAAAGCAGCACAGTTTGGCTTAGTACCGGG
TGTGGGAGTTGCCCCCGGCGTTGGCGTTGCTCCAGGGGTGGGTGTTGCTCC
TGGCGTCGGTCTGGCTCCTGGAGTGGGCGTAGCACCCGGTGTGGGGGTGG
CCCCGGGTGTTGGGGTTGCACCGGGTATCGGTCCGGGCGGTGTCGCAGCA
GCAGCTAAAAGCGCGGCGAAAGTTGCGGCCAAAGCCCAACTGCGCGCCG
CCGCGGGCCTCGGTGCAGGTATTCCGGGGCTGGGTGTCGGAGTTGGAGTC
CCGGGTTTGGGCGTGGGCGCGGGAGTTCCGGGACTGGGAGTGGGTGCCGG

AGTTCCTGGCTTTGGTGCAGGCGCAGATGAAGGTGTTCGTCGTAGCCTGA
GTCCGGAACTGCGTGAAGGTGATCCGAGTAGCAGCCAGCATCTGCCGAGC
ACCCCGAGCAGCCCGCGTGTTCCGGGTGCATTAGCTGCAGCAAAGCCGC
CAAGTATGGTGCAGCCGTGCCGGGCGTCTTAGGTGGTCTGGGCGCCCTGG
GTGGTGTAGGCATTCCGGGAGGTGTTGTGGGTGCAGGACCGGCCGCCGCA
GCTGCGGCCGCCAAAGCAGCTGCAAAAGCGGCCCAGTTTGGTTTAGTGGG
CGCCGCAGGTTTAGGCGGTTTAGGTGTGGGTGGACTGGGTGTACCTGGCG
TAGGCGGTCTGGGTGGAATTCCGCCCGCAGCGGCCGCGAAAGCGGCAAA
ATATGGCGCGGCAGGCCTGGGCGGCGTGCTGGGTGGGGCAGGTCAGTTTC
CGCTGGGCGGGGTTGCCGCACGTCCGGGATTTGGTCTGAGCCCGATTTTCC
CTGGCGGCGCATGTCTGGGTAAAGCATGTGGTCGTAAACGTAAAcacccagaa
acgctggtgaaagtaaaagatgctgaagatcagttgggtgcacgagtgggttacatcgaactggatctcaacagcggtaag
atccttgagagttttcgccccgaagaacgttttccaatgatgagcacttttaaagttctgctatgtggcgcggtattatcccgtat
tgacgccgggcaagagcaactcggtcgccgcatacactattctcagaatgacttggttgagtactcaccagtcacagaaaa
gcatcttacggatggcatgacagtaagagaattatgcagtgctgccataaccatgagtgataacactgcggccaacttactt
ctgacaacgatcggaggaccgaaggagctaaccgcttttttgcacaacatgggggatcatgtaactcgccttgatcgttggga
accggagctgaatgaagccataccaaacgacgagcgtgacaccacgatgcctgtagcaatggcaacaacgttgcgcaaa
ctattaactggcgaactacttactctagcttcccggcaacaattaatagactggatggaggcggataaagttgcaggaccact
tctgcgctcggcccttccggctggctggtttattgctgataaatctggagccggtgagcgtgggtctcgcggtatcattgcag
cactggggccagatggtaagccctcccgtatcgtagttatctacacgacggggagtcaggcaactatggatgaacgaaata
gacagatcgctgagataggtgcctcactgattaagcattggtaa (SEQ ID NO: 27)

MKKIWLALAGLVLAFSASAAQYEDHHHHHHHHHHSGSSLVPRGSHMSGSSSK
GEELFTGVVPILVELDGDVNGHKFSVRGEGEGDATNGKLTLKFICTTGKLPVP
WPTLVTTLTYGVQCFSRYPDHMKQHDFFKSAMPEGYVQERTISFKDDGTYK
TRAEVKFEGDTLVNRIELKGIDFKEDGNILGHKLEYNFNSHNVYITADKQKNG
IKANFKIRHNVEDGSVQLADHYQQNTPIGDGPVLLPDNHYLSTQSKLSKDPNE
KRDHMVLLEFVTAAGITHGMDELYKSGAPGGGVPGAIPGGVPGGVFYPGAG
LGALGGGALGPGGKPLKPVPGGLAGAGLGAGLGAFPAVTFPGALVPGGVAD
AAAAYKAAKAGAGLGGVPGVGGLGVSAGAVVPQPGAGVKPGKVPGVGLPG
VYPGGVLPGARFPGVGVLPGVPTGAGVKPKAPGVGGAFAGIPGVGPFGGPQP
GVPLGYPIKAPKLPGGYGLPYTTGKLPYGYGPGGVAGAAGKAGYPTGTGVG
PQAAAAAAAKAAAKFGAGAAGVLPGVGGAGVPGVPGAIPGIGGIAGVGTPA
AAAAAAAAAKAAKYGAAAGLVPGGPGFGPGVVGVPGAGVPGVGVPGAGIP
VVPGAGIPGAAVPGVVSPEAAAKAAAKAAKYGARPGVGVGGIPTYGVGAGG

FPGFGVGVGGIPGVAGVPGVGGVPGVGGVPGVGISPEAQAAAAKAAKYGA
AGAGVLGGLVPGPQAAVPGVPGTGGVPGVGTPAAAAKAAAKAAQFGLVP
GVGVAPGVGVAPGVGVAPGVGLAPGVGVAPGVGVAPGVGVAPGIGPGGVA
AAAKSAAKVAAKAQLRAAAGLGAGIPGLGVGVGVPGLGVGAGVPGLGVGA
GVPGFGAGADEGVRRSLSPELREGDPSSSQHLPSTPSSPRVPGALAAAKAAKY
GAAVPGVLGGLGALGGVGIPGGVVGAGPAAAAAAKAAAKAAQFGLVGAA
GLGGLGVGGLGVPGVGGLGGIPPAAAAKAAKYGAAGLGGVLGGAGQFPLG
GVAARPGFGLSPIFPGGACLGKACGRKRKHPETLVKVKDAEDQLGARVGYIE
LDLNSGKILESFRPEERFPMMSTFKVLLCGAVLSRIDAGQEQLGRRIHYSQND
LVEYSPVTEKHLTDGMTVRELCSAAITMSDNTAANLLLTTIGGPKELTAFLHN
MGDHVTRLDRWEPELNEAIPNDERDTTMPVAMATTLRKLLTGELLTLASRQ
QLIDWMEADKVAGPLLRSALPAGWFIADKSGAGERGSRGIIAALGPDGKPSRI
VVIYTTGSQATMDERNRQIAEIGASLIKHW (SEQ ID NO: 28)

### Example 5: Production of Truncated Elastin

Truncated human elastin is produced using the expression system as described in Example 4. The full length amino acid sequence lacking the native secretion tag is disclosed in SEQ ID NO: 29.

GGVPGAIPGGVPGGVFYPGAGLGALGGGALGPGGKPLKPVPGGLAGAGLGA
GLGAFPAVTFPGALVPGGVADAAAAYKAAKAGAGLGGVPGVGGLGVSAGA
VVPQPGAGVKPGKVPGVGLPGVYPGGVLPGARFPGVGVLPGVPTGAGVKPK
APGVGGAFAGIPGVGPFGGPQPGVPLGYPIKAPKLPGGYGLPYTTGKLPYGY
GPGGVAGAAGKAGYPTGTGVGPQAAAAAAKAAAKFGAGAAGVLPGVGG
AGVPGVPGAIPGIGGIAGVGTPAAAAAAAAAKAAKYGAAAGLVPGGPGFG
PGVVGVPGAGVPGVGVPGAGIPVVPGAGIPGAAVPGVVSPEAAAKAAAKAA
KYGARPGVGVGGIPTYGVGAGGFPGFGVGVGGIPGVAGVPGVGGVPGVGGV
PGVGISPEAQAAAAKAAKYGAAGAGVLGGLVPGPQAAVPGVPGTGGVPG
VGTPAAAAKAAAKAAQFGLVPGVGVAPGVGVAPGVGVAPGVGLAPGVGV
APGVGVAPGVGVAPGIGPGGVAAAAKSAAKVAAKAQLRAAAGLGAGIPGL
GVGVGVPGLGVGAGVPGLGVGAGVPGFGAGADEGVRRSLSPELREGDPSSS
QHLPSTPSSPRVPGALAAAKAAKYGAAVPGVLGGLGALGGVGIPGGVVGAG
PAAAAAAAKAAAKAAQFGLVGAAGLGGLGVGGLGVPGVGGLGGIPPAAAA
KAAKYGAAGLGGVLGGAGQFPLGGVAARPGFGLSPIFPGGACLGKACGRKR
K (SEQ ID NO: 29)

The codon optimized polynucleotide sequence encoding the full length human elastin lacking the native secretion tag is disclosed in SEQ ID NO: 30.

GGTGGCGTACCAGGCGCAATTCCTGGGGTGTCCCAGGCGGTGTTTTTAT
CCGGGCGCCGGTCTTGGCGCACTGGGTGGCGGTGCACTGGGCCCGGGCGG
CAAACCGCTGAAACCGGTACCAGGTGGTTTAGCAGGCGCCGGCTTAGGCG
CAGGTCTGGGAGCATTTCCGGCAGTTACCTTTCCAGGGGCACTGGTTCCTG
GAGGTGTGGCCGATGCAGCCGCGGCATATAAAGCCGCTAAAGCCGGTGCG
GGTTTAGGAGGCGTCCCAGGTGTCGGTGGCCTGGGTGTTAGCGCCGGTGC
AGTTGTTCCGCAGCCGGGAGCAGGGGTTAAACCTGGTAAAGTGCCGGGAG
TAGGTCTGCCAGGCGTTTATCCTGGTGGTGTTTTGCCGGGTGCCCGTTTTC
CGGGCGTTGGTGTTCTTCCAGGCGTGCCGACCGGAGCCGGTGTTAAACCG
AAAGCCCCCGGTGTTGGAGGTGCATTTGCAGGCATCCCGGGAGTTGGCCC
GTTTGGTGGTCCGCAACCTGGGGTTCCGTTAGGTTATCCGATTAAAGCACC
GAAACTGCCCGGCGGTTATGGTCTGCCGTACACAACCGGTAAACTGCCGT
ATGGTTATGGCCCGGGTGGAGTTGCGGGTGCAGCAGGTAAAGCGGGTTAT
CCTACCGGAACCGGTGTAGGTCCGCAGGCCGCTGCTGCCGCCGCCGCAAA
AGCAGCGGCTAAATTTGGCGCCGGAGCAGCGGGTGTTCTGCCTGGAGTTG
GTGGTGCGGGCGTGCCAGGGGTACCTGGTGCAATTCCGGGTATTGGTGGT
ATTGCCGGTGTCGGCACCCCGGCCGCGGCAGCTGCGGCAGCGGCGGCTGC
CAAAGCTGCTAAATACGGTGCCGCGGCGGGTCTGGTGCCAGGAGGTCCGG
GTTTTGGTCCGGGAGTGGTTGGCGTGCCTGGCGCAGGCGTTCCTGGTGTGG
GCGTTCCAGGTGCAGGGATTCCTGTTGTGCCTGGTGCCGGTATTCCCGGCG
CGGCCGTTCCGGGGGTGGTTAGCCCGGAAGCCGCAGCGAAGGCTGCGGC
AAAGGCAGCAAAGTATGGCGCACGCCCAGGAGTCGGCGTGGGTGGTATC
CCGACCTATGGGGTGGGCGCAGGGGGTTTTCCTGGTTTCGGCGTAGGTGT
AGGAGGTATACCGGGCGTGGCCGGTGTACCAGGGGTTGGTGGCGTCCCTG
GTGTTGGCGGTGTGCCAGGTGTTGGTATTTCACCGGAAGCACAGGCAGCA
GCCGCAGCTAAGGCAGCGAAATATGGTGCCGCCGGCGCAGGAGTTTAGG
TGGGCTGGTTCCGGGCCCGCAGGCAGCTGTGCCGGGGGTTCCAGGCACCG
GTGGTGTCCCTGGAGTCGGTACGCCGGCTGCAGCGGCAGCCAAAGCGGCT
GCGAAAGCAGCACAGTTTGGCTTAGTACCGGGTGTGGGAGTTGCCCCCGG
CGTTGGCGTTGCTCCAGGGGTGGGTGTTGCTCCTGGCGTCGGTCTGGCTCC
TGGAGTGGGCGTAGCACCCGGTGTGGGGGTGGCCCCGGGTGTTGGGGTTG
CACCGGGTATCGGTCCGGGCGGTGTCGCAGCAGCAGCTAAAAGCGCGGC

GAAAGTTGCGGCCAAAGCCCAACTGCGCGCCGCCGCGGGCCTCGGTGCAG
GTATTCCGGGGCTGGGTGTCGGAGTTGGAGTCCCGGGTTTGGGCGTGGGC
GCGGGAGTTCCGGGACTGGGAGTGGGTGCCGGAGTTCCTGGCTTTGGTGC
AGGCGCAGATGAAGGTGTTCGTCGTAGCCTGAGTCCGGAACTGCGTGAAG
GTGATCCGAGTAGCAGCCAGCATCTGCCGAGCACCCCGAGCAGCCCGCGT
GTTCCGGGTGCATTAGCTGCAGCAAAAGCCGCCAAGTATGGTGCAGCCGT
GCCGGGCGTCTTAGGTGGTCTGGGCGCCCTGGGTGGTGTAGGCATTCCGG
GAGGTGTTGTGGGTGCAGGACCGGCCGCCGCAGCTGCGGCCGCCAAAGC
AGCTGCAAAAGCGGCCCAGTTTGGTTTAGTGGGCGCCGCAGGTTTAGGCG
GTTTAGGTGTGGGTGGACTGGGTGTACCTGGCGTAGGCGGTCTGGGTGGA
ATTCCGCCCtaa (SEQ ID NO: 30)

The amino acid sequence of a 60.7 kDa human elastin truncated at the C-terminal is disclosed in SEQ ID NO: 31. The 60.7 kDa truncated elastin has amino acids 706-761 deleted from the full length elastin.

GGVPGAIPGGVPGGVFYPGAGLGALGGGALGPGGKPLKPVPGGLAGAGLGA
GLGAFPAVTFPGALVPGGVADAAAAYKAAKAGAGLGGVPGVGGLGVSAGA
VVPQPGAGVKPGKVPGVGLPGVYPGGVLPGARFPGVGVLPGVPTGAGVKPK
APGVGGAFAGIPGVGPFGGPQPGVPLGYPIKAPKLPGGYGLPYTTGKLPYGY
GPGGVAGAAGKAGYPTGTGVGPQAAAAAAKAAAKFGAGAAGVLPGVGG
AGVPGVPGAIPGIGGIAGVGTPAAAAAAAAAKAAKYGAAAGLVPGGPGFG
PGVVGVPGAGVPGVGVPGAGIPVVPGAGIPGAAVPGVVSPEAAAKAAAKAA
KYGARPGVGVGGIPTYGVGAGGFPGFGVGVGGIPGVAGVPGVGGVPGVGGV
PGVGISPEAQAAAAKAAKYGAAGAGVLGGLVPGPQAAVPGVPGTGGVPG
VGTPAAAAKAAAKAAQFGLVPGVGVAPGVGVAPGVGVAPGVGLAPGVGV
APGVGVAPGVGVAPGIGPGGVAAAAKSAAKVAAKAQLRAAAGLGAGIPGL
GVGVGVPGLGVGAGVPGLGVGAGVPGFGAGADEGVRRSLSPELREGDPSSS
QHLPSTPSSPRVPGALAAAKAAKYGAAVPGVLGGLGALGGVGIPGGVVGAG
PAAAAAAKAAAKAAQFGLVGAAGLGGLGVGGLGVPGVGGLGGIPP (SEQ
ID NO: 31)

The codon optimized polynucleotide sequence encoding the truncated 60.7 kDa human elastin is disclosed in SEQ ID NO: 32.

GGTGGCGTACCAGGCGCAATTCCTGGGGGTGTCCCAGGCGGTGTTTTTAT
CCGGGCGCCGGTCTTGGCGCACTGGGTGGCGGTGCACTGGGCCCGGGCGG
CAAACCGCTGAAACCGGTACCAGGTGGTTTAGCAGGCGCCGGCTTAGGCG

CAGGTCTGGGAGCATTTCCGGCAGTTACCTTTCCAGGGGCACTGGTTCCTG
GAGGTGTGGCCGATGCAGCCGCGGCATATAAAGCCGCTAAAGCCGGTGCG
GGTTTAGGAGGCGTCCCAGGTGTCGGTGGCCTGGGTGTTAGCGCCGGTGC
AGTTGTTCCGCAGCCGGGAGCAGGGGTTAAACCTGGTAAAGTGCCGGGAG
TAGGTCTGCCAGGCGTTTATCCTGGTGGTGTTTTGCCGGGTGCCCGTTTTC
CGGGCGTTGGTGTTCTTCCAGGCGTGCCGACCGGAGCCGGTGTTAAACCG
AAAGCCCCCGGTGTTGGAGGTGCATTTGCAGGCATCCCGGGAGTTGGCCC
GTTTGGTGGTCCGCAACCTGGGGTTCCGTTAGGTTATCCGATTAAAGCACC
GAAACTGCCCGGCGGTTATGGTCTGCCGTACACAACCGGTAAACTGCCGT
ATGGTTATGGCCCGGGTGGAGTTGCGGGTGCAGCAGGTAAAGCGGGTTAT
CCTACCGGAACCGGTGTAGGTCCGCAGGCCGCTGCTGCCGCCGCCGCAAA
AGCAGCGGCTAAATTGGCGCCGGAGCAGCGGGTGTTCTGCCTGGAGTTG
GTGGTGCGGGCGTGCCAGGGGTACCTGGTGCAATTCCGGGTATTGGTGGT
ATTGCCGGTGTCGGCACCCCGGCCGCGGCAGCTGCGGCAGCGGCGGCTGC
CAAAGCTGCTAAATACGGTGCCGCGGCGGGTCTGGTGCCAGGAGGTCCGG
GTTTTGGTCCGGGAGTGGTTGGCGTGCCTGGCGCAGGCGTTCCTGGTGTGG
GCGTTCCAGGTGCAGGGATTCCTGTTGTGCCTGGTGCCGGTATTCCCGGCG
CGGCCGTTCCGGGGGTGGTTAGCCCGGAAGCCGCAGCGAAGGCTGCGGC
AAAGGCAGCAAAGTATGGCGCACGCCCAGGAGTCGGCGTGGGTGGTATC
CCGACCTATGGGGTGGGCGCAGGGGGTTTTCCTGGTTTCGGCGTAGGTGT
AGGAGGTATACCGGGCGTGGCCGGTGTACCAGGGGTTGGTGGCGTCCCTG
GTGTTGGCGGTGTGCCAGGTGTTGGTATTTCACCGGAAGCACAGGCAGCA
GCCGCAGCTAAGGCAGCGAAATATGGTGCCGCCGGCGCAGGAGTTTTAGG
TGGGCTGGTTCCGGGCCCGCAGGCAGCTGTGCCGGGGGTTCCAGGCACCG
GTGGTGTCCCTGGAGTCGGTACGCCGGCTGCAGCGGCAGCCAAAGCGGCT
GCGAAAGCAGCACAGTTTGGCTTAGTACCGGGTGTGGGAGTTGCCCCCGG
CGTTGGCGTTGCTCCAGGGGTGGGTGTTGCTCCTGGCGTCGGTCTGGCTCC
TGGAGTGGGCGTAGCACCCGGTGTGGGGGTGGCCCCGGGTGTTGGGGTTG
CACCGGGTATCGGTCCGGGCGGTGTCGCAGCAGCAGCTAAAAGCGCGGC
GAAAGTTGCGGCCAAAGCCCAACTGCGCGCCGCCGCGGGCCTCGGTGCAG
GTATTCCGGGGCTGGGTGTCGGAGTTGGAGTCCCGGGTTTGGGCGTGGGC
GCGGGAGTTCCGGGACTGGGAGTGGGTGCCGGAGTTCCTGGCTTTGGTGC
AGGCGCAGATGAAGGTGTTCGTCGTAGCCTGAGTCCGGAACTGCGTGAAG
GTGATCCGAGTAGCAGCCAGCATCTGCCGAGCACCCCGAGCAGCCCGCGT

GTTCCGGGTGCATTAGCTGCAGCAAAAGCCGCCAAGTATGGTGCAGCCGT
GCCGGGCGTCTTAGGTGGTCTGGGCGCCCTGGGTGGTGTAGGCATTCCGG
GAGGTGTTGTGGGTGCAGGACCGGCCGCCGCAGCTGCGGCCGCCAAAGC
AGCTGCAAAAGCGGCCCAGTTTGGTTTAGTGGGCGCCGCAGGTTTAGGCG
GTTTAGGTGTGGGTGGACTGGGTGTACCTGGCGTAGGCGGTCTGGGTGGA
ATTCCGCCCtaa (SEQ ID NO: 32)

The amino acid sequence of a 58.8 kDa human elastin truncated at the N-terminal is disclosed in SEQ ID NO: 33. The 58.8 kDa truncated elastin has amino acids 2-85 deleted from the full length elastin.

GLGGVPGVGGLGVSAGAVVPQPGAGVKPGKVPGVGLPGVYPGGVLPGARFP
GVGVLPGVPTGAGVKPKAPGVGGAFAGIPGVGPFGGPQPGVPLGYPIKAPKL
PGGYGLPYTTGKLPYGYGPGGVAGAAGKAGYPTGTGVGPQAAAAAAKAA
AKFGAGAAGVLPGVGGAGVPGVPGAIPGIGGIAGVGTPAAAAAAAAAKAA
KYGAAAGLVPGGPGFGPGVVGVPGAGVPGVGVPGAGIPVVPGAGIPGAAVP
GVVSPEAAAKAAAKAAKYGARPGVGVGGIPTYGVGAGGFPGFGVGVGGIPG
VAGVPGVGGVPGVGGVPGVGISPEAQAAAAKAAKYGAAGAGVLGGLVPG
PQAAVPGVPGTGGVPGVGTPAAAAKAAAKAAQFGLVPGVGVAPGVGVAP
GVGVAPGVGLAPGVGVAPGVGVAPGVGVAPGIGPGGVAAAAKSAAKVAAK
AQLRAAAGLGAGIPGLGVGVGVPGLGVGAGVPGLGVGAGVPGFGAGADEG
VRRSLSPELREGDPSSSQHLPSTPSSPRVPGALAAAKAAKYGAAVPGVLGGLG
ALGGVGIPGGVVGAGPAAAAAAAKAAAKAAQFGLVGAAGLGGLGVGGLG
VPGVGGLGGIPPAAAAKAAKYGAAGLGGVLGGAGQFPLGGVAARPGFGLSP
IFPGGACLGKACGRKRK (SEQ ID NO: 33)

The codon optimized polynucleotide sequence encoding the 58.8 kDa truncated human elastin is disclosed in SEQ ID NO: 34.

GGTTTAGGAGGCGTCCCAGGTGTCGGTGGCCTGGGTGTTAGCGCCGGTGC
AGTTGTTCCGCAGCCGGGAGCAGGGGTTAAACCTGGTAAAGTGCCGGGAG
TAGGTCTGCCAGGCGTTTATCCTGGTGGTGTTTTGCCGGGTGCCCGTTTC
CGGGCGTTGGTGTTCTTCCAGGCGTGCCGACCGGAGCCGGTGTTAAACCG
AAAGCCCCCGGTGTTGGAGGTGCATTTGCAGGCATCCCGGGAGTTGGCCC
GTTTGGTGGTCCGCAACCTGGGGTTCCGTTAGGTTATCCGATTAAAGCACC
GAAACTGCCCGGCGGTTATGGTCTGCCGTACACAACCGGTAAACTGCCGT
ATGGTTATGGCCCGGGTGGAGTTGCGGGTGCAGCAGGTAAAGCGGGTTAT
CCTACCGGAACCGGTGTAGGTCCGCAGGCCGCTGCTGCCGCCGCCGCAAA

AGCAGCGGCTAAATTTGGCGCCGGAGCAGCGGGTGTTCTGCCTGGAGTTG
GTGGTGCGGGCGTGCCAGGGGTACCTGGTGCAATTCCGGGTATTGGTGGT
ATTGCCGGTGTCGGCACCCCGGCCGCGGCAGCTGCGGCAGCGGCGGCTGC
CAAAGCTGCTAAATACGGTGCCGCGGCGGGTCTGGTGCCAGGAGGTCCGG
GTTTTGGTCCGGGAGTGGTTGGCGTGCCTGGCGCAGGCGTTCCTGGTGTGG
GCGTTCCAGGTGCAGGGATTCCTGTTGTGCCTGGTGCCGGTATTCCGGCG
CGGCCGTTCCGGGGGTGGTTAGCCCGGAAGCCGCAGCGAAGGCTGCGGC
AAAGGCAGCAAAGTATGGCGCACGCCCAGGAGTCGGCGTGGGTGGTATC
CCGACCTATGGGGTGGGCGCAGGGGGTTTTCCTGGTTTCGGCGTAGGTGT
AGGAGGTATACCGGGCGTGGCCGGTGTACCAGGGGTTGGTGGCGTCCCTG
GTGTTGGCGGTGTGCCAGGTGTTGGTATTTCACCGGAAGCACAGGCAGCA
GCCGCAGCTAAGGCAGCGAAATATGGTGCCGCCGGCGCAGGAGTTTAGG
TGGGCTGGTTCCGGGCCCGCAGGCAGCTGTGCCGGGGGTTCCAGGCACCG
GTGGTGTCCCTGGAGTCGGTACGCCGGCTGCAGCGGCAGCCAAAGCGGCT
GCGAAAGCAGCACAGTTTGGCTTAGTACCGGGTGTGGGAGTTGCCCCGG
CGTTGGCGTTGCTCCAGGGGTGGGTGTTGCTCCTGGCGTCGGTCTGGCTCC
TGGAGTGGGCGTAGCACCCGGTGTGGGGGTGGCCCCGGGTGTTGGGGTTG
CACCGGGTATCGGTCCGGGCGGTGTCGCAGCAGCAGCTAAAAGCGCGGC
GAAAGTTGCGGCCAAAGCCCAACTGCGCGCCGCCGCGGGCCTCGGTGCAG
GTATTCCGGGGCTGGGTGTCGGAGTTGGAGTCCCGGGTTTGGGCGTGGGC
GCGGGAGTTCCGGGACTGGGAGTGGGTGCCGGAGTTCCTGGCTTTGGTGC
AGGCGCAGATGAAGGTGTTCGTCGTAGCCTGAGTCCGGAACTGCGTGAAG
GTGATCCGAGTAGCAGCCAGCATCTGCCGAGCACCCCGAGCAGCCCGCGT
GTTCCGGGTGCATTAGCTGCAGCAAAAGCCGCCAAGTATGGTGCAGCCGT
GCCGGGCGTCTTAGGTGGTCTGGGCGCCCTGGGTGGTGTAGGCATTCCGG
GAGGTGTTGTGGGTGCAGGACCGGCCGCCGCAGCTGCGGCCGCCAAAGC
AGCTGCAAAAGCGGCCCAGTTTGGTTTAGTGGGCGCCGCAGGTTTAGGCG
GTTTAGGTGTGGGTGGACTGGGTGTACCTGGCGTAGGCGGTCTGGGTGGA
ATTCCGCCCGCAGCGGCCGCGAAAGCGGCAAAATATGGCGCGGCAGGCC
TGGGCGGCGTGCTGGGTGGGGCAGGTCAGTTTCCGCTGGGCGGGGTTGCC
GCACGTCCGGGATTTGGTCTGAGCCCGATTTTCCCTGGCGGCGCATGTCTG
GGTAAAGCATGTGGTCGTAAACGTAAAtaa (SEQ ID NO: 34)

The amino acid sequence of a 57 kDa human elastin truncated at the C-terminal is disclosed in SEQ ID NO: 35. The 57 kDa truncated elastin has amino acids 661-761 deleted from the full length elastin.

GGVPGAIPGGVPGGVFYPGAGLGALGGGALGPGGKPLKPVPGGLAGAGLGA
GLGAFPAVTFPGALVPGGVADAAAAYKAAKAGAGLGGVPGVGGLGVSAGA
VVPQPGAGVKPGKVPGVGLPGVYPGGVLPGARFPGVGVLPGVPTGAGVKPK
APGVGGAFAGIPGVGPFGGPQPGVPLGYPIKAPKLPGGYGLPYTTGKLPYGY
GPGGVAGAAGKAGYPTGTGVGPQAAAAAAKAAAKFGAGAAGVLPGVGG
AGVPGVPGAIPGIGGIAGVGTPAAAAAAAAAKAAKYGAAAGLVPGGPGFG
PGVVGVPGAGVPGVGVPGAGIPVVPGAGIPGAAVPGVVSPEAAAKAAAKAA
KYGARPGVGVGGIPTYGVGAGGFPGFGVGVGGIPGVAGVPGVGGVPGVGGV
PGVGISPEAQAAAAKAAKYGAAGAGVLGGLVPGPQAAVPGVPGTGGVPG
VGTPAAAAKAAAKAAQFGLVPGVGVAPGVGVAPGVGVAPGVGLAPGVGV
APGVGVAPGVGVAPGIGPGGVAAAAKSAAKVAAKAQLRAAAGLGAGIPGL
GVGVGVPGLGVGAGVPGLGVGAGVPGFGAGADEGVRRSLSPELREGDPSSS
QHLPSTPSSPRVPGALAAAKAAKYGAAVPGVLGGLGALGGVGIPGGVVGAG
P (SEQ ID NO: 35)

The codon optimized polynucleotide sequence encoding the 57 kDa truncated human elastin is disclosed in SEQ ID NO: 36

GGTGGCGTACCAGGCGCAATTCCTGGGGGTGTCCCAGGCGGTGTTTTTAT
CCGGGCGCCGGTCTTGGCGCACTGGGTGGCGGTGCACTGGGCCCGGGCGG
CAAACCGCTGAAACCGGTACCAGGTGGTTTAGCAGGCGCCGGCTTAGGCG
CAGGTCTGGGAGCATTTCCGGCAGTTACCTTTCCAGGGGCACTGGTTCCTG
GAGGTGTGGCCGATGCAGCCGCGGCATATAAAGCCGCTAAAGCCGGTGCG
GGTTTAGGAGGCGTCCCAGGTGTCGGTGGCCTGGGTGTTAGCGCCGGTGC
AGTTGTTCCGCAGCCGGGAGCAGGGGTTAAACCTGGTAAAGTGCCGGGAG
TAGGTCTGCCAGGCGTTTATCCTGGTGGTGTTTTGCCGGGTGCCCGTTTTC
CGGGCGTTGGTGTTCTTCCAGGCGTGCCGACCGGAGCCGGTGTTAAACCG
AAAGCCCCCGGTGTTGGAGGTGCATTTGCAGGCATCCCGGGAGTTGGCCC
GTTTGGTGGTCCGCAACCTGGGGTTCCGTTAGGTTATCCGATTAAAGCACC
GAAACTGCCCGGCGGTTATGGTCTGCCGTACACAACCGGTAAACTGCCGT
ATGGTTATGGCCCGGGTGGAGTTGCGGGTGCAGCAGGTAAAGCGGGTTAT
CCTACCGGAACCGGTGTAGGTCCGCAGGCCGCTGCTGCCGCCGCCGCAAA
AGCAGCGGCTAAATTTGGCGCCGGAGCAGCGGGTGTTCTGCCTGGAGTTG

GTGGTGCGGGCGTGCCAGGGGTACCTGGTGCAATTCCGGGTATTGGTGGT
ATTGCCGGTGTCGGCACCCCGGCCGCGGCAGCTGCGGCAGCGGCGGCTGC
CAAAGCTGCTAAATACGGTGCCGCGGCGGGTCTGGTGCCAGGAGGTCCGG
GTTTTGGTCCGGGAGTGGTTGGCGTGCCTGGCGCAGGCGTTCCTGGTGTGG
GCGTTCCAGGTGCAGGGATTCCTGTTGTGCCTGGTGCCGGTATTCCCGGCG
CGGCCGTTCCGGGGGTGGTTAGCCCGGAAGCCGCAGCGAAGGCTGCGGC
AAAGGCAGCAAAGTATGGCGCACGCCCAGGAGTCGGCGTGGGTGGTATC
CCGACCTATGGGGTGGGCGCAGGGGGTTTTCCTGGTTTCGGCGTAGGTGT
AGGAGGTATACCGGGCGTGGCCGGTGTACCAGGGGTTGGTGGCGTCCCTG
GTGTTGGCGGTGTGCCAGGTGTTGGTATTTCACCGGAAGCACAGGCAGCA
GCCGCAGCTAAGGCAGCGAAATATGGTGCCGCCGGCGCAGGAGTTTTAGG
TGGGCTGGTTCCGGGCCCGCAGGCAGCTGTGCCGGGGGTTCCAGGCACCG
GTGGTGTCCCTGGAGTCGGTACGCCGGCTGCAGCGGCAGCCAAAGCGGCT
GCGAAAGCAGCACAGTTTGGCTTAGTACCGGGTGTGGGAGTTGCCCCCGG
CGTTGGCGTTGCTCCAGGGGTGGGTGTTGCTCCTGGCGTCGGTCTGGCTCC
TGGAGTGGGCGTAGCACCCGGTGTGGGGGTGGCCCCGGGTGTTGGGGTTG
CACCGGGTATCGGTCCGGGCGGTGTCGCAGCAGCAGCTAAAAGCGCGGC
GAAAGTTGCGGCCAAAGCCCAACTGCGCGCCGCCGCGGGCCTCGGTGCAG
GTATTCCGGGGCTGGGTGTCGGAGTTGGAGTCCCGGGTTTGGGCGTGGGC
GCGGGAGTTCCGGGACTGGGAGTGGGTGCCGGAGTTCCTGGCTTTGGTGC
AGGCGCAGATGAAGGTGTTCGTCGTAGCCTGAGTCCGGAACTGCGTGAAG
GTGATCCGAGTAGCAGCCAGCATCTGCCGAGCACCCCGAGCAGCCCGCGT
GTTCCGGGTGCATTAGCTGCAGCAAAAGCCGCCAAGTATGGTGCAGCCGT
GCCGGGCGTCTTAGGTGGTCTGGGCGCCCTGGGTGGTGTAGGCATTCCGG
GAGGTGTTGTGGGTGCAGGACCGtaa (SEQ ID NO: 36)

The amino acid sequence of a 53.9 kDa human elastin truncated at the C-terminal is disclosed in SEQ ID NO: 37. The 53.9 kDa truncated elastin has amino acids 624-761 deleted from the full length elastin.

GGVPGAIPGGVPGGVFYPGAGLGALGGGALGPGGKPLKPVPGGLAGAGLGA
GLGAFPAVTFPGALVPGGVADAAAAYKAAKAGAGLGGVPGVGGLGVSAGA
VVPQPGAGVKPGKVPGVGLPGVYPGGVLPGARFPGVGVLPGVPTGAGVKPK
APGVGGAFAGIPGVGPFGGPQPGVPLGYPIKAPKLPGGYGLPYTTGKLPYGY
GPGGVAGAAGKAGYPTGTGVGPQAAAAAAAKAAAKFGAGAAGVLPGVGG
AGVPGVPGAIPGIGGIAGVGTPAAAAAAAAAAKAAKYGAAAGLVPGGPGFG

PGVVGVPGAGVPGVGVPGAGIPVVPGAGIPGAAVPGVVSPEAAAKAAAKAA
KYGARPGVGVGGIPTYGVGAGGFPGFGVGVGGIPGVAGVPGVGGVPGVGGV
PGVGISPEAQAAAAAKAAKYGAAGAGVLGGLVPGPQAAVPGVPGTGGVPG
VGTPAAAAAKAAAKAAQFGLVPGVGVAPGVGVAPGVGVAPGVGLAPGVGV
APGVGVAPGVGVAPGIGPGGVAAAAKSAAKVAAKAQLRAAAGLGAGIPGL
GVGVGVPGLGVGAGVPGLGVGAGVPGFGAGADEGVRRSLSPELREGDPSSS
QHLPSTPSSPRVPGA (SEQ ID NO: 37)

The codon optimized polynucleotide sequence encoding the 53.9 kDa truncated human elastin is disclosed in SEQ ID NO: 38

GGTGGCGTACCAGGCGCAATTCCTGGGGTGTCCCAGGCGGTGTTTTTAT
CCGGGCGCCGGTCTTGGCGCACTGGGTGGCGGTGCACTGGGCCCGGGCGG
CAAACCGCTGAAACCGGTACCAGGTGGTTTAGCAGGCGCCGGCTTAGGCG
CAGGTCTGGGAGCATTTCCGGCAGTTACCTTTCCAGGGGCACTGGTTCCTG
GAGGTGTGGCCGATGCAGCCGCGGCATATAAAGCCGCTAAAGCCGGTGCG
GGTTTAGGAGGCGTCCCAGGTGTCGGTGGCCTGGGTGTTAGCGCCGGTGC
AGTTGTTCCGCAGCCGGGAGCAGGGGTTAAACCTGGTAAAGTGCCGGGAG
TAGGTCTGCCAGGCGTTTATCCTGGTGGTGTTTTGCCGGGTGCCCGTTTTC
CGGGCGTTGGTGTTCTTCCAGGCGTGCCGACCGGAGCCGGTGTTAAACCG
AAAGCCCCCGGTGTTGGAGGTGCATTTGCAGGCATCCCGGGAGTTGGCCC
GTTTGGTGGTCCGCAACCTGGGGTTCCGTTAGGTTATCCGATTAAAGCACC
GAAACTGCCCGGCGGTTATGGTCTGCCGTACACAACCGGTAAACTGCCGT
ATGGTTATGGCCCGGGTGGAGTTGCGGGTGCAGCAGGTAAAGCGGGTTAT
CCTACCGGAACCGGTGTAGGTCCGCAGGCCGCTGCTGCCGCCGCCGCAAA
AGCAGCGGCTAAATTTGGCGCCGGAGCAGCGGGTGTTCTGCCTGGAGTTG
GTGGTGCGGGCGTGCCAGGGGTACCTGGTGCAATTCCGGGTATTGGTGGT
ATTGCCGGTGTCGGCACCCCGGCCGCGGCAGCTGCGGCAGCGGCGGCTGC
CAAAGCTGCTAAATACGGTGCCGCGGCGGGTCTGGTGCCAGGAGGTCCGG
GTTTTGGTCCGGGAGTGGTTGGCGTGCCTGGCGCAGGCGTTCCTGGTGTGG
GCGTTCCAGGTGCAGGGATTCCTGTTGTGCCTGGTGCCGGTATTCCCGGCG
CGGCCGTTCCGGGGGTGGTTAGCCCGGAAGCCGCAGCGAAGGCTGCGGC
AAAGGCAGCAAAGTATGGCGCACGCCCAGGAGTCGGCGTGGGTGGTATC
CCGACCTATGGGGTGGGCGCAGGGGGTTTTCCTGGTTTCGGCGTAGGTGT
AGGAGGTATACCGGGCGTGGCCGGTGTACCAGGGGTTGGTGGCGTCCCTG
GTGTTGGCGGTGTGCCAGGTGTTGGTATTTCACCGGAAGCACAGGCAGCA

GCCGCAGCTAAGGCAGCGAAATATGGTGCCGCCGGCGCAGGAGTTTTAGG
TGGGCTGGTTCCGGGCCCGCAGGCAGCTGTGCCGGGGGTTCCAGGCACCG
GTGGTGTCCCTGGAGTCGGTACGCCGGCTGCAGCGGCAGCCAAAGCGGCT
GCGAAAGCAGCACAGTTTGGCTTAGTACCGGGTGTGGGAGTTGCCCCCGG
CGTTGGCGTTGCTCCAGGGGTGGGTGTTGCTCCTGGCGTCGGTCTGGCTCC
TGGAGTGGGCGTAGCACCCGGTGTGGGGGTGGCCCCGGGTGTTGGGGTTG
CACCGGGTATCGGTCCGGGCGGTGTCGCAGCAGCAGCTAAAAGCGCGGC
GAAAGTTGCGGCCAAAGCCCAACTGCGCGCCGCCGCGGGCCTCGGTGCAG
GTATTCCGGGGCTGGGTGTCGGAGTTGGAGTCCCGGGTTTGGGCGTGGGC
GCGGGAGTTCCGGGACTGGGAGTGGGTGCCGGAGTTCCTGGCTTTGGTGC
AGGCGCAGATGAAGGTGTTCGTCGTAGCCTGAGTCCGGAACTGCGTGAAG
GTGATCCGAGTAGCAGCCAGCATCTGCCGAGCACCCCGAGCAGCCCGCGT
GTTCCGGGTGCAtaa (SEQ ID NO: 38)

The amino acid sequence of a 45.3 kDa human elastin truncated at the C-terminal is disclosed in SEQ ID NO: 39. The 45.3 kDa truncated elastin has amino acids 529-761 deleted from the full length elastin.

GGVPGAIPGGVPGGVFYPGAGLGALGGGALGPGGKPLKPVPGGLAGAGLGA
GLGAFPAVTFPGALVPGGVADAAAAYKAAKAGAGLGGVPGVGGLGVSAGA
VVPQPGAGVKPGKVPGVGLPGVYPGGVLPGARFPGVGVLPGVPTGAGVKPK
APGVGGAFAGIPGVGPFGGPQPGVPLGYPIKAPKLPGGYGLPYTTGKLPYGY
GPGGVAGAAGKAGYPTGTGVGPQAAAAAAKAAKFGAGAAGVLPGVGG
AGVPGVPGAIPGIGGIAGVGTPAAAAAAAAAKAAKYGAAGLVPGGPGFG
PGVVGVPGAGVPGVGVPGAGIPVVPGAGIPGAAVPGVVSPEAAAKAAAKAA
KYGARPGVGVGGIPTYGVGAGGFPGFGVGVGGIPGVAGVPGVGGVPGVGGV
PGVGISPEAQAAAAKAAKYGAAGAGVLGGLVPGPQAAVPGVPGTGGVPG
VGTPAAAAKAAAKAAQFGLVPGVGVAPGVGVAPGVGVAPGVGLAPGVGV
APGVGVAPGVGVAPGIGPGGV (SEQ ID NO: 39)

The codon optimized polynucleotide sequence encoding the 45.3 kDa truncated human elastin is disclosed in SEQ ID NO: 40

GGTGGCGTACCAGGCGCAATTCCTGGGGGTGTCCCAGGCGGTGTTTTTAT
CCGGGCGCCGGTCTTGGCGCACTGGGTGGCGGTGCACTGGGCCCGGGCGG
CAAACCGCTGAAACCGGTACCAGGTGGTTTAGCAGGCGCCGGCTTAGGCG
CAGGTCTGGGAGCATTTCCGGCAGTTACCTTTCCAGGGGCACTGGTTCCTG
GAGGTGTGGCCGATGCAGCCGCGGCATATAAAGCCGCTAAAGCCGGTGCG

GGTTTAGGAGGCGTCCCAGGTGTCGGTGGCCTGGGTGTTAGCGCCGGTGC
AGTTGTTCCGCAGCCGGGAGCAGGGGTTAAACCTGGTAAAGTGCCGGGAG
TAGGTCTGCCAGGCGTTTATCCTGGTGGTGTTTTGCCGGGTGCCCGTTTTC
CGGGCGTTGGTGTTCTTCCAGGCGTGCCGACCGGAGCCGGTGTTAAACCG
AAAGCCCCCGGTGTTGGAGGTGCATTTGCAGGCATCCCGGGAGTTGGCCC
GTTTGGTGGTCCGCAACCTGGGGTTCCGTTAGGTTATCCGATTAAAGCACC
GAAACTGCCCGGCGGTTATGGTCTGCCGTACACAACCGGTAAACTGCCGT
ATGGTTATGGCCCGGGTGGAGTTGCGGGTGCAGCAGGTAAAGCGGGTTAT
CCTACCGGAACCGGTGTAGGTCCGCAGGCCGCTGCTGCCGCCGCCGCAAA
AGCAGCGGCTAAATTTGGCGCCGGAGCAGCGGGTGTTCTGCCTGGAGTTG
GTGGTGCGGGCGTGCCAGGGGTACCTGGTGCAATTCCGGGTATTGGTGGT
ATTGCCGGTGTCGGCACCCCGGCCGCGGCAGCTGCGGCAGCGGCGGCTGC
CAAAGCTGCTAAATACGGTGCCGCGGCGGGTCTGGTGCCAGGAGGTCCGG
GTTTTGGTCCGGGAGTGGTTGGCGTGCCTGGCGCAGGCGTTCCTGGTGTGG
GCGTTCCAGGTGCAGGGATTCCTGTTGTGCCTGGTGCCGGTATTCCCGGCG
CGGCCGTTCCGGGGGTGGTTAGCCCGGAAGCCGCAGCGAAGGCTGCGGC
AAAGGCAGCAAAGTATGGCGCACGCCCAGGAGTCGGCGTGGGTGGTATC
CCGACCTATGGGGTGGGCGCAGGGGGTTTTCCTGGTTTCGGCGTAGGTGT
AGGAGGTATACCGGGCGTGGCCGGTGTACCAGGGGTTGGTGGCGTCCCTG
GTGTTGGCGGTGTGCCAGGTGTTGGTATTTCACCGGAAGCACAGGCAGCA
GCCGCAGCTAAGGCAGCGAAATATGGTGCCGCCGGCGCAGGAGTTTAGG
TGGGCTGGTTCCGGGCCCGCAGGCAGCTGTGCCGGGGGTTCCAGGCACCG
GTGGTGTCCCTGGAGTCGGTACGCCGGCTGCAGCGGCAGCCAAAGCGGCT
GCGAAAGCAGCACAGTTTGGCTTAGTACCGGGTGTGGGAGTTGCCCCCGG
CGTTGGCGTTGCTCCAGGGGTGGGTGTTGCTCCTGGCGTCGGTCTGGCTCC
TGGAGTGGGCGTAGCACCCGGTGTGGGGGTGGCCCCGGGTGTTGGGGTTG
CACCGGGTATCGGTCCGGGCGGTGTCtaa (SEQ ID NO: 40)

The amino acid sequence of a 44.4 kDa human elastin truncated at the N-terminal is disclosed in SEQ ID NO: 41. The 44.4 kDa truncated elastin has amino acids 2-246 deleted from the full length elastin.

GVLPGVGGAGVPGVPGAIPGIGGIAGVGTPAAAAAAAAAKAAKYGAAAGL
VPGGPGFGPGVVGVPGAGVPGVGVPGAGIPVVPGAGIPGAAVPGVVSPEAAA
KAAAKAAKYGARPGVGVGGIPTYGVGAGGFPGFGVGVGGIPGVAGVPGVG
GVPGVGGVPGVGISPEAQAAAAAKAAKYGAAGAGVLGGLVPGPQAAVPGV

PGTGGVPGVGTPAAAAAKAAAKAAQFGLVPGVGVAPGVGVAPGVGVAPGV
GLAPGVGVAPGVGVAPGVGVAPGIGPGGVAAAAKSAAKVAAKAQLRAAAG
LGAGIPGLGVGVGVPGLGVGAGVPGLGVGAGVPGFGAGADEGVRRSLSPEL
REGDPSSSQHLPSTPSSPRVPGALAAAKAAKYGAAVPGVLGGLGALGGVGIP
GGVVGAGPAAAAAAAKAAAKAAQFGLVGAAGLGGLGVGGLGVPGVGGLG
GIPPAAAAKAAKYGAAGLGGVLGGAGQFPLGGVAARPGFGLSPIFPGGACLG
KACGRKRK (SEQ ID NO: 41)

The codon optimized polynucleotide sequence encoding the 44.4 kDa truncated human elastin is disclosed in SEQ ID NO: 42

GGTGTTCTGCCTGGAGTTGGTGGTGCGGCGTGCCAGGGGTACCTGGTGC
AATTCCGGGTATTGGTGGTATTGCCGGTGTCGGCACCCCGGCCGCGGCAG
CTGCGGCAGCGGCGGCTGCCAAAGCTGCTAAATACGGTGCCGCGGCGGGT
CTGGTGCCAGGAGGTCCGGGTTTTGGTCCGGGAGTGGTTGGCGTGCCTGG
CGCAGGCGTTCCTGGTGTGGGCGTTCCAGGTGCAGGGATTCCTGTTGTGCC
TGGTGCCGGTATTCCCGGCGCGGCCGTTCCGGGGGTGGTTAGCCCGGAAG
CCGCAGCGAAGGCTGCGGCAAAGGCAGCAAAGTATGGCGCACGCCCAGG
AGTCGGCGTGGGTGGTATCCCGACCTATGGGGTGGGCGCAGGGGGTTTTC
CTGGTTTCGGCGTAGGTGTAGGAGGTATACCGGGCGTGGCCGGTGTACCA
GGGGTTGGTGGCGTCCCTGGTGTTGGCGGTGTGCCAGGTGTTGGTATTTCA
CCGGAAGCACAGGCAGCAGCCGCAGCTAAGGCAGCGAAATATGGTGCCG
CCGGCGCAGGAGTTTTAGGTGGGCTGGTTCCGGGCCCGCAGGCAGCTGTG
CCGGGGGTTCCAGGCACCGGTGGTGTCCCTGGAGTCGGTACGCCGGCTGC
AGCGGCAGCCAAAGCGGCTGCGAAAGCAGCACAGTTTGGCTTAGTACCGG
GTGTGGGAGTTGCCCCCGGCGTTGGCGTTGCTCCAGGGGTGGGTGTTGCTC
CTGGCGTCGGTCTGGCTCCTGGAGTGGGCGTAGCACCCGGTGTGGGGGTG
GCCCCGGGTGTTGGGGTTGCACCGGGTATCGGTCCGGGCGGTGTCGCAGC
AGCAGCTAAAAGCGCGGCGAAAGTTGCGGCCAAAGCCCAACTGCGCGCC
GCCGCGGGCCTCGGTGCAGGTATTCCGGGGCTGGGTGTCGGAGTTGGAGT
CCCGGGTTTGGGCGTGGGCGCGGGAGTTCCGGGACTGGGAGTGGGTGCCG
GAGTTCCTGGCTTTGGTGCAGGCGCAGATGAAGGTGTTCGTCGTAGCCTG
AGTCCGGAACTGCGTGAAGGTGATCCGAGTAGCAGCCAGCATCTGCCGAG
CACCCCGAGCAGCCCGCGTGTTCCGGGTGCATTAGCTGCAGCAAAAGCCG
CCAAGTATGGTGCAGCCGTGCCGGGCGTCTTAGGTGGTCTGGGCGCCCTG
GGTGGTGTAGGCATTCCGGGAGGTGTTGTGGGTGCAGGACCGGCCGCCGC

AGCTGCGGCCGCCAAAGCAGCTGCAAAAGCGGCCCAGTTTGGTTTAGTGG
GCGCCGCAGGTTTAGGCGGTTTAGGTGTGGGTGGACTGGGTGTACCTGGC
GTAGGCGGTCTGGGTGGAATTCCGCCCGCAGCGGCCGCGAAAGCGGCAA
AATATGGCGCGGCAGGCCTGGGCGGCGTGCTGGGTGGGGCAGGTCAGTTT
CCGCTGGGCGGGGTTGCCGCACGTCCGGGATTTGGTCTGAGCCCGATTTC
CCTGGCGGCGCATGTCTGGGTAAAGCATGTGGTCGTAAACGTAAAtaa
(SEQ ID NO: 42)

The amino acid sequence of a 40.4 kDa human elastin truncated at the N-terminal is disclosed in SEQ ID NO: 43. The 40.4 kDa truncated elastin has amino acids 2-295 deleted from the full length elastin.

GLVPGGPGFGPGVVGVPGAGVPGVGVPGAGIPVVPGAGIPGAAVPGVVSPEA
AAKAAAKAAKYGARPGVGVGGIPTYGVGAGGFPGFGVGVGGIPGVAGVPG
VGGVPGVGGVPGVGISPEAQAAAAKAAKYGAAGAGVLGGLVPGPQAAVP
GVPGTGGVPGVGTPAAAAKAAAKAAQFGLVPGVGVAPGVGVAPGVGVAP
GVGLAPGVGVAPGVGVAPGVGVAPGIGPGGVAAAAKSAAKVAAKAQLRAA
AGLGAGIPGLGVGVGVPGLGVGAGVPGLGVGAGVPGFGAGADEGVRRSLSP
ELREGDPSSSQHLPSTPSSPRVPGALAAAKAAKYGAAVPGVLGGLGALGGVG
IPGGVVGAGPAAAAAAAKAAAKAAQFGLVGAAGLGGLGVGGLGVPGVGGL
GGIPPAAAAKAAKYGAAGLGGVLGGAGQFPLGGVAARPGFGLSPIFPGGACL
GKACGRKRK (SEQ ID NO: 43)

The codon optimized polynucleotide sequence encoding the 40.4 kDa truncated human elastin is disclosed in SEQ ID NO: 44

GGTCTGGTGCCAGGAGGTCCGGGTTTTGGTCCGGGAGTGGTTGGCGTGCC
TGGCGCAGGCGTTCCTGGTGTGGGCGTTCCAGGTGCAGGGATTCCTGTTGT
GCCTGGTGCCGGTATTCCCGGCGCGGCCGTTCCGGGGGTGGTTAGCCCGG
AAGCCGCAGCGAAGGCTGCGGCAAAGGCAGCAAAGTATGGCGCACGCCC
AGGAGTCGGCGTGGGTGGTATCCCGACCTATGGGGTGGGCGCAGGGGGTT
TTCCTGGTTTCGGCGTAGGTGTAGGAGGTATACCGGGCGTGGCCGGTGTA
CCAGGGGTTGGTGGCGTCCCTGGTGTTGGCGGTGTGCCAGGTGTTGGTATT
TCACCGGAAGCACAGGCAGCAGCCGCAGCTAAGGCAGCGAAATATGGTG
CCGCCGGCGCAGGAGTTTTAGGTGGGCTGGTTCCGGGCCCGCAGGCAGCT
GTGCCGGGGGTTCCAGGCACCGGTGGTGTCCCTGGAGTCGGTACGCCGGC
TGCAGCGGCAGCCAAAGCGGCTGCGAAAGCAGCACAGTTTGGCTTAGTAC
CGGGTGTGGGAGTTGCCCCCGGCGTTGGCGTTGCTCCAGGGGTGGGTGTT

GCTCCTGGCGTCGGTCTGGCTCCTGGAGTGGGCGTAGCACCCGGTGTGGG
GGTGGCCCCGGGTGTTGGGGTTGCACCGGGTATCGGTCCGGGCGGTGTCG
CAGCAGCAGCTAAAAGCGCGGCGAAAGTTGCGGCCAAAGCCCAACTGCG
CGCCGCCGCGGGCCTCGGTGCAGGTATTCCGGGGCTGGGTGTCGGAGTTG
GAGTCCCGGGTTTGGGCGTGGGCGCGGGAGTTCCGGGACTGGGAGTGGGT
GCCGGAGTTCCTGGCTTTGGTGCAGGCGCAGATGAAGGTGTTCGTCGTAG
CCTGAGTCCGGAACTGCGTGAAGGTGATCCGAGTAGCAGCCAGCATCTGC
CGAGCACCCCGAGCAGCCCGCGTGTTCCGGGTGCATTAGCTGCAGCAAAA
GCCGCCAAGTATGGTGCAGCCGTGCCGGGCGTCTTAGGTGGTCTGGGCGC
CCTGGGTGGTGTAGGCATTCCGGGAGGTGTTGTGGGTGCAGGACCGGCCG
CCGCAGCTGCGGCCGCCAAAGCAGCTGCAAAAGCGGCCCAGTTTGGTTTA
GTGGGCGCCGCAGGTTTAGGCGGTTTAGGTGTGGGTGGACTGGGTGTACC
TGGCGTAGGCGGTCTGGGTGGAATTCCGCCCGCAGCGGCCGCGAAAGCGG
CAAAATATGGCGCGGCAGGCCTGGGCGGCGTGCTGGGTGGGGCAGGTCA
GTTTCCGCTGGGCGGGGTTGCCGCACGTCCGGGATTTGGTCTGAGCCCGAT
TTTCCCTGGCGGCGCATGTCTGGGTAAAGCATGTGGTCGTAAACGTAAAtaa
(SEQ ID NO: 44)

The amino acid sequence of a 39.8 kDa human elastin truncated at the C-terminal is disclosed in SEQ ID NO: 45. The 39.8 kDa truncated elastin has amino acids 462-761 deleted from the full length elastin.

GGVPGAIPGGVPGGVFYPGAGLGALGGGALGPGGKPLKPVPGGLAGAGLGA
GLGAFPAVTFPGALVPGGVADAAAAYKAAKAGAGLGGVPGVGGLGVSAGA
VVPQPGAGVKPGKVPGVGLPGVYPGGVLPGARFPGVGVLPGVPTGAGVKPK
APGVGGAFAGIPGVGPFGGPQPGVPLGYPIKAPKLPGGYGLPYTTGKLPYGY
GPGGVAGAAGKAGYPTGTGVGPQAAAAAAKAAAKFGAGAAGVLPGVGG
AGVPGVPGAIPGIGGIAGVGTPAAAAAAAAAKAAKYGAAAGLVPGGPGFG
PGVVGVPGAGVPGVGVPGAGIPVVPGAGIPGAAVPGVVSPEAAAKAAAKAA
KYGARPGVGVGGIPTYGVGAGGFPGFGVGVGGIPGVAGVPGVGGVPGVGGV
PGVGISPEAQAAAAAKAAKYGAAGAGVLGGLVPGPQAAVPGVPGTGGVPG
VGTP (SEQ ID NO: 45)

The codon optimized polynucleotide sequence encoding the 39.8 kDa truncated human elastin is disclosed in SEQ ID NO: 46

GGTGGCGTACCAGGCGCAATTCCTGGGGGTGTCCCAGGCGGTGTTTTTAT
CCGGGCGCCGGTCTTGGCGCACTGGGTGGCGGTGCACTGGGCCCGGGCGG

CAAACCGCTGAAACCGGTACCAGGTGGTTTAGCAGGCGCCGGCTTAGGCG
CAGGTCTGGGAGCATTTCCGGCAGTTACCTTTCCAGGGGCACTGGTTCCTG
GAGGTGTGGCCGATGCAGCCGCGGCATATAAAGCCGCTAAAGCCGGTGCG
GGTTTAGGAGGCGTCCCAGGTGTCGGTGGCCTGGGTGTTAGCGCCGGTGC
AGTTGTTCCGCAGCCGGGAGCAGGGGTTAAACCTGGTAAAGTGCCGGGAG
TAGGTCTGCCAGGCGTTTATCCTGGTGGTGTTTTGCCGGGTGCCCGTTTTC
CGGGCGTTGGTGTTCTTCCAGGCGTGCCGACCGGAGCCGGTGTTAAACCG
AAAGCCCCCGGTGTTGGAGGTGCATTTGCAGGCATCCCGGGAGTTGGCCC
GTTTGGTGGTCCGCAACCTGGGGTTCCGTTAGGTTATCCGATTAAAGCACC
GAAACTGCCCGGCGGTTATGGTCTGCCGTACACAACCGGTAAACTGCCGT
ATGGTTATGGCCCGGGTGGAGTTGCGGGTGCAGCAGGTAAAGCGGGTTAT
CCTACCGGAACCGGTGTAGGTCCGCAGGCCGCTGCTGCCGCCGCCGCAAA
AGCAGCGGCTAAATTTGGCGCCGGAGCAGCGGGTGTTCTGCCTGGAGTTG
GTGGTGCGGGCGTGCCAGGGGTACCTGGTGCAATTCCGGGTATTGGTGGT
ATTGCCGGTGTCGGCACCCCGGCCGCGGCAGCTGCGGCAGCGGCGGCTGC
CAAAGCTGCTAAATACGGTGCCGCGGCGGGTCTGGTGCCAGGAGGTCCGG
GTTTTGGTCCGGGAGTGGTTGGCGTGCCTGGCGCAGGCGTTCCTGGTGTGG
GCGTTCCAGGTGCAGGGATTCCTGTTGTGCCTGGTGCCGGTATTCCCGGCG
CGGCCGTTCCGGGGGTGGTTAGCCCGGAAGCCGCAGCGAAGGCTGCGGC
AAAGGCAGCAAAGTATGGCGCACGCCCAGGAGTCGGCGTGGGTGGTATC
CCGACCTATGGGGTGGGCGCAGGGGGTTTTCCTGGTTTCGGCGTAGGTGT
AGGAGGTATACCGGGCGTGGCCGGTGTACCAGGGGTTGGTGGCGTCCCTG
GTGTTGGCGGTGTGCCAGGTGTTGGTATTTCACCGGAAGCACAGGCAGCA
GCCGCAGCTAAGGCAGCGAAATATGGTGCCGCCGGCGCAGGAGTTTAGG
TGGGCTGGTTCCGGGCCCGCAGGCAGCTGTGCCGGGGGTTCCAGGCACCG
GTGGTGTCCCTGGAGTCGGTACGCCGtaa (SEQ ID NO: 46)

The amino acid sequence of a 36.1 kDa human elastin truncated at the C-terminal is disclosed in SEQ ID NO: 47. The 36.1 kDa truncated elastin has amino acids 418-761 deleted from the full length elastin.

GGVPGAIPGGVPGGVFYPGAGLGALGGGALGPGGKPLKPVPGGLAGAGLGA
GLGAFPAVTFPGALVPGGVADAAAAYKAAKAGAGLGGVPGVGGLGVSAGA
VVPQPGAGVKPGKVPGVGLPGVYPGGVLPGARFPGVGVLPGVPTGAGVKPK
APGVGGAFAGIPGVGPFGGPQPGVPLGYPIKAPKLPGGYGLPYTTGKLPYGY
GPGGVAGAAGKAGYPTGTGVGPQAAAAAAAKAAAKFGAGAAGVLPGVGG

AGVPGVPGAIPGIGGIAGVGTPAAAAAAAAAAKAAKYGAAAGLVPGGPGFG
PGVVGVPGAGVPGVGVPGAGIPVVPGAGIPGAAVPGVVSPEAAAKAAAKAA
KYGARPGVGVGGIPTYGVGAGGFPGFGVGVGGIPGVAGVPGVGGVPGVGGV
PGVGISPEAQ (SEQ ID NO: 47)

The codon optimized polynucleotide sequence encoding the 36.1 kDa truncated human elastin is disclosed in SEQ ID NO: 48

GGTGGCGTACCAGGCGCAATTCCTGGGGGTGTCCCAGGCGGTGTTTTTAT
CCGGGCGCCGGTCTTGGCGCACTGGGTGGCGGTGCACTGGGCCCGGGCGG
CAAACCGCTGAAACCGGTACCAGGTGGTTTAGCAGGCGCCGGCTTAGGCG
CAGGTCTGGGAGCATTTCCGGCAGTTACCTTTCCAGGGGCACTGGTTCCTG
GAGGTGTGGCCGATGCAGCCGCGGCATATAAAGCCGCTAAAGCCGGTGCG
GGTTTAGGAGGCGTCCCAGGTGTCGGTGGCCTGGGTGTTAGCGCCGGTGC
AGTTGTTCCGCAGCCGGGAGCAGGGGTTAAACCTGGTAAAGTGCCGGGAG
TAGGTCTGCCAGGCGTTTATCCTGGTGGTGTTTTGCCGGGTGCCCGTTTTC
CGGGCGTTGGTGTTCTTCCAGGCGTGCCGACCGGAGCCGGTGTTAAACCG
AAAGCCCCCGGTGTTGGAGGTGCATTTGCAGGCATCCCGGGAGTTGGCCC
GTTTGGTGGTCCGCAACCTGGGGTTCCGTTAGGTTATCCGATTAAAGCACC
GAAACTGCCCGGCGGTTATGGTCTGCCGTACACAACCGGTAAACTGCCGT
ATGGTTATGGCCCGGGTGGAGTTGCGGGTGCAGCAGGTAAAGCGGGTTAT
CCTACCGGAACCGGTGTAGGTCCGCAGGCCGCTGCTGCCGCCGCCGCAAA
AGCAGCGGCTAAATTTGGCGCCGGAGCAGCGGGTGTTCTGCCTGGAGTTG
GTGGTGCGGGCGTGCCAGGGGTACCTGGTGCAATTCCGGGTATTGGTGGT
ATTGCCGGTGTCGGCACCCCGGCCGCGGCAGCTGCGGCAGCGGCGGCTGC
CAAAGCTGCTAAATACGGTGCCGCGGCGGGTCTGGTGCCAGGAGGTCCGG
GTTTTGGTCCGGGAGTGGTTGGCGTGCCTGGCGCAGGCGTTCCTGGTGTGG
GCGTTCCAGGTGCAGGGATTCCTGTTGTGCCTGGTGCCGGTATTCCCGGCG
CGGCCGTTCCGGGGGTGGTTAGCCCGGAAGCCGCAGCGAAGGCTGCGGC
AAAGGCAGCAAAGTATGGCGCACGCCCAGGAGTCGGCGTGGGTGGTATC
CCGACCTATGGGGTGGGCGCAGGGGGTTTTCCTGGTTTCGGCGTAGGTGT
AGGAGGTATACCGGGCGTGGCCGGTGTACCAGGGGTTGGTGGCGTCCCTG
GTGTTGGCGGTGTGCCAGGTGTTGGTATTTCACCGGAAGCACAGtaa (SEQ
ID NO: 48)

The amino acid sequence of a 34.9 kDa human elastin truncated at the N-terminal is disclosed in SEQ ID NO: 49. The 34.9 kDa truncated elastin has amino acids 2-360 deleted from the full length elastin.

RPGVGVGGIPTYGVGAGGFPGFGVGVGGIPGVAGVPGVGGVPGVGGVPGVG
ISPEAQAAAAAKAAKYGAAGAGVLGGLVPGPQAAVPGVPGTGGVPGVGTPA
AAAAKAAAKAAQFGLVPGVGVAPGVGVAPGVGVAPGVGLAPGVGVAPGV
GVAPGVGVAPGIGPGGVAAAAKSAAKVAAKAQLRAAAGLGAGIPGLGVGV
GVPGLGVGAGVPGLGVGAGVPGFGAGADEGVRRSLSPELREGDPSSSQHLPS
TPSSPRVPGALAAAKAAKYGAAVPGVLGGLGALGGVGIPGGVVGAGPAAAA
AAAKAAAKAAQFGLVGAAGLGGLGVGGLGVPGVGGLGGIPPAAAKAAKY
GAAGLGGVLGGAGQFPLGGVAARPGFGLSPIFPGGACLGKACGRKRK (SEQ
ID NO: 49)

The codon optimized polynucleotide sequence encoding the 34.9 kDa truncated human elastin is disclosed in SEQ ID NO: 50

CGCCCAGGAGTCGGCGTGGTGGTATCCCGACCTATGGGGTGGGCGCAGG
GGGTTTTCCTGGTTTCGGCGTAGGTGTAGGAGGTATACCGGGCGTGGCCG
GTGTACCAGGGGTTGGTGGCGTCCCTGGTGTTGGCGGTGTGCCAGGTGTT
GGTATTTCACCGGAAGCACAGGCAGCAGCCGCAGCTAAGGCAGCGAAAT
ATGGTGCCGCCGGCGCAGGAGTTTTAGGTGGGCTGGTTCCGGGCCCGCAG
GCAGCTGTGCCGGGGGTTCCAGGCACCGGTGGTGTCCCTGGAGTCGGTAC
GCCGGCTGCAGCGGCAGCCAAAGCGGCTGCGAAAGCAGCACAGTTTGGC
TTAGTACCGGGTGTGGGAGTTGCCCCCGGCGTTGGCGTTGCTCCAGGGGT
GGGTGTTGCTCCTGGCGTCGGTCTGGCTCCTGGAGTGGGCGTAGCACCCG
GTGTGGGGGTGGCCCCGGGTGTTGGGGTTGCACCGGGTATCGGTCCGGGC
GGTGTCGCAGCAGCAGCTAAAAGCGCGGCGAAAGTTGCGGCCAAAGCCC
AACTGCGCGCCGCCGCGGGCCTCGGTGCAGGTATTCCGGGGCTGGGTGTC
GGAGTTGGAGTCCCGGGTTTGGGCGTGGGCGCGGGAGTTCCGGGACTGGG
AGTGGGTGCCGGAGTTCCTGGCTTTGGTGCAGGCGCAGATGAAGGTGTTC
GTCGTAGCCTGAGTCCGGAACTGCGTGAAGGTGATCCGAGTAGCAGCCAG
CATCTGCCGAGCACCCCGAGCAGCCCGCGTGTTCCGGGTGCATTAGCTGC
AGCAAAAGCCGCCAAGTATGGTGCAGCCGTGCCGGGCGTCTTAGGTGGTC
TGGGCGCCCTGGGTGGTGTAGGCATTCCGGGAGGTGTTGTGGGTGCAGGA
CCGGCCGCCGCAGCTGCGGCCGCCAAAGCAGCTGCAAAAGCGGCCCAGTT
TGGTTTAGTGGGCGCCGCAGGTTTAGGCGGTTTAGGTGTGGGTGGACTGG

GTGTACCTGGCGTAGGCGGTCTGGGTGGAATTCCGCCCGCAGCGGCCGCG
AAAGCGGCAAAATATGGCGCGGCAGGCCTGGGCGGCGTGCTGGGTGGGG
CAGGTCAGTTTCCGCTGGGCGGGGTTGCCGCACGTCCGGGATTTGGTCTG
AGCCCGATTTTCCCTGGCGGCGCATGTCTGGGTAAAGCATGTGGTCGTAA
ACGTAAAtaa (SEQ ID NO: 50)

The amino acid sequence of a 32 kDa human elastin truncated at the C-terminal is disclosed in SEQ ID NO: 51. The 32 kDa truncated elastin has amino acids 373-761 deleted from the full length elastin.

GGVPGAIPGGVPGGVFYPGAGLGALGGGALGPGGKPLKPVPGGLAGAGLGA
GLGAFPAVTFPGALVPGGVADAAAAYKAAKAGAGLGGVPGVGGLGVSAGA
VVPQPGAGVKPGKVPGVGLPGVYPGGVLPGARFPGVGVLPGVPTGAGVKPK
APGVGGAFAGIPGVGPFGGPQPGVPLGYPIKAPKLPGGYGLPYTTGKLPYGY
GPGGVAGAAGKAGYPTGTGVGPQAAAAAAKAAAKFGAGAAGVLPGVGG
AGVPGVPGAIPGIGGIAGVGTPAAAAAAAAAKAAKYGAAAGLVPGGPGFG
PGVVGVPGAGVPGVGVPGAGIPVVPGAGIPGAAVPGVVSPEAAAKAAAKAA
KYGARPGVGVGGIPTY (SEQ ID NO: 51)

The codon optimized polynucleotide sequence encoding the 32 kDa truncated human elastin is disclosed in SEQ ID NO: 52

GGTGGCGTACCAGGCGCAATTCCTGGGGTGTCCCAGGCGGTGTTTTTAT
CCGGGCGCCGGTCTTGGCGCACTGGGTGGCGGTGCACTGGGCCCGGGCGG
CAAACCGCTGAAACCGGTACCAGGTGGTTTAGCAGGCGCCGGCTTAGGCG
CAGGTCTGGGAGCATTTCCGGCAGTTACCTTTCCAGGGGCACTGGTTCCTG
GAGGTGTGGCCGATGCAGCCGCGGCATATAAAGCCGCTAAAGCCGGTGCG
GGTTTAGGAGGCGTCCCAGGTGTCGGTGGCCTGGGTGTTAGCGCCGGTGC
AGTTGTTCCGCAGCCGGGAGCAGGGGTTAAACCTGGTAAAGTGCCGGGAG
TAGGTCTGCCAGGCGTTTATCCTGGTGGTGTTTTGCCGGGTGCCCGTTTC
CGGGCGTTGGTGTTCTTCCAGGCGTGCCGACCGGAGCCGGTGTTAAACCG
AAAGCCCCCGGTGTTGGAGGTGCATTTGCAGGCATCCCGGGAGTTGGCCC
GTTTGGTGGTCCGCAACCTGGGGTTCCGTTAGGTTATCCGATTAAAGCACC
GAAACTGCCCGGCGGTTATGGTCTGCCGTACACAACCGGTAAACTGCCGT
ATGGTTATGGCCCGGGTGGAGTTGCGGGTGCAGCAGGTAAAGCGGGTTAT
CCTACCGGAACCGGTGTAGGTCCGCAGGCCGCTGCTGCCGCCGCCGCAAA
AGCAGCGGCTAAATTTGGCGCCGGAGCAGCGGGTGTTCTGCCTGGAGTTG
GTGGTGCGGGCGTGCCAGGGGTACCTGGTGCAATTCCGGGTATTGGTGGT

ATTGCCGGTGTCGGCACCCCGGCCGCGGCAGCTGCGGCAGCGGCGGCTGC
CAAAGCTGCTAAATACGGTGCCGCGGCGGGTCTGGTGCCAGGAGGTCCGG
GTTTTGGTCCGGGAGTGGTTGGCGTGCCTGGCGCAGGCGTTCCTGGTGTGG
GCGTTCCAGGTGCAGGGATTCCTGTTGTGCCTGGTGCCGGTATTCCCGGCG
CGGCCGTTCCGGGGGTGGTTAGCCCGGAAGCCGCAGCGAAGGCTGCGGC
AAAGGCAGCAAAGTATGGCGCACGCCCAGGAGTCGGCGTGGGTGGTATC
CCGACCTATtaa (SEQ ID NO: 52)

The amino acid sequence of a 29.9 kDa human elastin truncated at the C-terminal is disclosed in SEQ ID NO: 53. The 60.7 kDa truncated elastin has amino acids 347-761 deleted from the full length elastin.

GGVPGAIPGGVPGGVFYPGAGLGALGGGALGPGGKPLKPVPGGLAGAGLGA
GLGAFPAVTFPGALVPGGVADAAAAYKAAKAGAGLGGVPGVGGLGVSAGA
VVPQPGAGVKPGKVPGVGLPGVYPGGVLPGARFPGVGVLPGVPTGAGVKPK
APGVGGAFAGIPGVGPFGGPQPGVPLGYPIKAPKLPGGYGLPYTTGKLPYGY
GPGGVAGAAGKAGYPTGTGVGPQAAAAAAKAAAKFGAGAAGVLPGVGG
AGVPGVPGAIPGIGGIAGVGTPAAAAAAAAAKAAKYGAAAGLVPGGPGFG
PGVVGVPGAGVPGVGVPGAGIPVVPGAGIPGAAVPGVVSPE (SEQ ID NO:
53)

The codon optimized polynucleotide sequence encoding the 29.9 kDa truncated human elastin is disclosed in SEQ ID NO: 54

GGTGGCGTACCAGGCGCAATTCCTGGGGGTGTCCCAGGCGGTGTTTTTAT
CCGGGCGCCGGTCTTGGCGCACTGGGTGGCGGTGCACTGGGCCCGGGCGG
CAAACCGCTGAAACCGGTACCAGGTGGTTTAGCAGGCGCCGGCTTAGGCG
CAGGTCTGGGAGCATTTCCGGCAGTTACCTTTCCAGGGGCACTGGTTCCTG
GAGGTGTGGCCGATGCAGCCGCGGCATATAAAGCCGCTAAAGCCGGTGCG
GGTTTAGGAGGCGTCCCAGGTGTCGGTGGCCTGGGTGTTAGCGCCGGTGC
AGTTGTTCCGCAGCCGGGAGCAGGGGTTAAACCTGGTAAAGTGCCGGGAG
TAGGTCTGCCAGGCGTTTATCCTGGTGGTGTTTTGCCGGGTGCCCGTTTTC
CGGGCGTTGGTGTTCTTCCAGGCGTGCCGACCGGAGCCGGTGTTAAACCG
AAAGCCCCCGGTGTTGGAGGTGCATTTGCAGGCATCCCGGGAGTTGGCCC
GTTTGGTGGTCCGCAACCTGGGGTTCCGTTAGGTTATCCGATTAAAGCACC
GAAACTGCCCGGCGGTTATGGTCTGCCGTACACAACCGGTAAACTGCCGT
ATGGTTATGGCCCGGGTGGAGTTGCGGGTGCAGCAGGTAAAGCGGGTTAT
CCTACCGGAACCGGTGTAGGTCCGCAGGCCGCTGCTGCCGCCGCCGCAAA

AGCAGCGGCTAAATTTGGCGCCGGAGCAGCGGGTGTTCTGCCTGGAGTTG
GTGGTGCGGGCGTGCCAGGGGTACCTGGTGCAATTCCGGGTATTGGTGGT
ATTGCCGGTGTCGGCACCCCGGCCGCGGCAGCTGCGGCAGCGGCGGCTGC
CAAAGCTGCTAAATACGGTGCCGCGGCGGGTCTGGTGCCAGGAGGTCCGG
GTTTTGGTCCGGGAGTGGTTGGCGTGCCTGGCGCAGGCGTTCCTGGTGTGG
GCGTTCCAGGTGCAGGGATTCCTGTTGTGCCTGGTGCCGGTATTCCCGGCG
CGGCCGTTCCGGGGGTGGTTAGCCCGGAAtaa (SEQ ID NO: 54)

The amino acid sequence of a 29.4 kDa human elastin truncated at the N-terminal is disclosed in SEQ ID NO: 55. The 29.4 kDa truncated elastin has amino acids 2-425 deleted from the full length elastin.

KYGAAGAGVLGGLVPGPQAAVPGVPGTGGVPGVGTPAAAAAKAAAKAAQF
GLVPGVGVAPGVGVAPGVGVAPGVGLAPGVGVAPGVGVAPGVGVAPGIGP
GGVAAAAKSAAKVAAKAQLRAAAGLGAGIPGLGVGVGVPGLGVGAGVPGL
GVGAGVPGFGAGADEGVRRSLSPELREGDPSSSQHLPSTPSSPRVPGALAAAK
AAKYGAAVPGVLGGLGALGGVGIPGGVVGAGPAAAAAAKAAAKAAQFGL
VGAAGLGGLGVGGLGVPGVGGLGGIPPAAAAKAAKYGAAGLGGVLGGAGQ
FPLGGVAARPGFGLSPIFPGGACLGKACGRKRK (SEQ ID NO: 55)

The codon optimized polynucleotide sequence encoding the 29.4 kDa truncated human elastin is disclosed in SEQ ID NO: 56

AAATATGGTGCCGCCGGCGCAGGAGTTTTAGGTGGGCTGGTTCCGGCCC
GCAGGCAGCTGTGCCGGGGGTTCCAGGCACCGGTGGTGTCCCTGGAGTCG
GTACGCCGGCTGCAGCGGCAGCCAAAGCGGCTGCGAAAGCAGCACAGTT
TGGCTTAGTACCGGGTGTGGGAGTTGCCCCCGGCGTTGGCGTTGCTCCAG
GGGTGGGTGTTGCTCCTGGCGTCGGTCTGGCTCCTGGAGTGGGCGTAGCA
CCCGGTGTGGGGGTGGCCCCGGGTGTTGGGGTTGCACCGGGTATCGGTCC
GGGCGGTGTCGCAGCAGCAGCTAAAAGCGCGGCGAAAGTTGCGGCCAAA
GCCCAACTGCGCGCCGCCGCGGGCCTCGGTGCAGGTATTCCGGGGCTGGG
TGTCGGAGTTGGAGTCCCGGGTTTGGGCGTGGGCGCGGGAGTTCCGGGAC
TGGGAGTGGGTGCCGGAGTTCCTGGCTTTGGTGCAGGCGCAGATGAAGGT
GTTCGTCGTAGCCTGAGTCCGGAACTGCGTGAAGGTGATCCGAGTAGCAG
CCAGCATCTGCCGAGCACCCCGAGCAGCCCGCGTGTTCCGGGTGCATTAG
CTGCAGCAAAAGCCGCCAAGTATGGTGCAGCCGTGCCGGGCGTCTTAGGT
GGTCTGGGCGCCCTGGGTGGTGTAGGCATTCCGGGAGGTGTTGTGGGTGC
AGGACCGGCCGCCGCAGCTGCGGCCGCCAAAGCAGCTGCAAAAGCGGCC

CAGTTTGGTTTAGTGGGCGCCGCAGGTTTAGGCGGTTTAGGTGTGGGTGG
ACTGGGTGTACCTGGCGTAGGCGGTCTGGGTGGAATTCCGCCCGCAGCGG
CCGCGAAAGCGGCAAAATATGGCGCGGCAGGCCTGGGCGGCGTGCTGGG
TGGGGCAGGTCAGTTTCCGCTGGGCGGGGTTGCCGCACGTCCGGGATTTG
GTCTGAGCCCGATTTTCCCTGGCGGCGCATGTCTGGGTAAAGCATGTGGTC
GTAAACGTAAAtaa (SEQ ID NO: 56)

The amino acid sequence of a 25.3 kDa human elastin truncated at the N-terminal is disclosed in SEQ ID NO: 57. The 25.3 kDa truncated elastin has amino acids 2-473 deleted from the full length elastin.

QFGLVPGVGVAPGVGVAPGVGVAPGVGLAPGVGVAPGVGVAPGVGVAPGI
GPGGVAAAAKSAAKVAAKAQLRAAAGLGAGIPGLGVGVGVPGLGVGAGVP
GLGVGAGVPGFGAGADEGVRRSLSPELREGDPSSSQHLPSTPSSPRVPGALAA
AKAAKYGAAVPGVLGGLGALGGVGIPGGVVGAGPAAAAAAKAAAKAAQ
FGLVGAAGLGGLGVGGLGVPGVGGLGGIPPAAAAKAAKYGAAGLGGVLGG
AGQFPLGGVAARPGFGLSPIFPGGACLGKACGRKRK (SEQ ID NO: 57)

The codon optimized polynucleotide sequence encoding the 25.3 kDa truncated human elastin is disclosed in SEQ ID NO: 58

CAGTTTGGCTTAGTACCGGGTGTGGAGTTGCCCCCGGCGTTGGCGTTGCT
CCAGGGGTGGGTGTTGCTCCTGGCGTCGGTCTGGCTCCTGGAGTGGGCGT
AGCACCCGGTGTGGGGGTGGCCCCGGGTGTTGGGGTTGCACCGGGTATCG
GTCCGGGCGGTGTCGCAGCAGCAGCTAAAAGCGCGGCGAAAGTTGCGGC
CAAAGCCCAACTGCGCGCCGCCGCGGGCCTCGGTGCAGGTATTCCGGGGC
TGGGTGTCGGAGTTGGAGTCCCGGGTTTGGGCGTGGGCGCGGGAGTTCCG
GGACTGGGAGTGGGTGCCGGAGTTCCTGGCTTTGGTGCAGGCGCAGATGA
AGGTGTTCGTCGTAGCCTGAGTCCGGAACTGCGTGAAGGTGATCCGAGTA
GCAGCCAGCATCTGCCGAGCACCCCGAGCAGCCCGCGTGTTCCGGGTGCA
TTAGCTGCAGCAAAAGCCGCCAAGTATGGTGCAGCCGTGCCGGGCGTCTT
AGGTGGTCTGGGCGCCCTGGGTGGTGTAGGCATTCCGGGAGGTGTTGTGG
GTGCAGGACCGGCCGCCGCAGCTGCGGCCGCCAAAGCAGCTGCAAAAGC
GGCCCAGTTTGGTTTAGTGGGCGCCGCAGGTTTAGGCGGTTTAGGTGTGG
GTGGACTGGGTGTACCTGGCGTAGGCGGTCTGGGTGGAATTCCGCCCGCA
GCGGCCGCGAAAGCGGCAAAATATGGCGCGGCAGGCCTGGGCGGCGTGC
TGGGTGGGGCAGGTCAGTTTCCGCTGGGCGGGGTTGCCGCACGTCCGGGA

TTTGGTCTGAGCCCGATTTTCCCTGGCGGCGCATGTCTGGGTAAAGCATGT
GGTCGTAAACGTAAAtaa (SEQ ID NO: 58)

The amino acid sequence of a 24.1 kDa human elastin truncated at the C-terminal is disclosed in SEQ ID NO: 59. The 24.1 kDa truncated elastin has amino acids 277-761 deleted from the full length elastin.

GGVPGAIPGGVPGGVFYPGAGLGALGGGALGPGGKPLKPVPGGLAGAGLGA
GLGAFPAVTFPGALVPGGVADAAAAYKAAKAGAGLGGVPGVGGLGVSAGA
VVPQPGAGVKPGKVPGVGLPGVYPGGVLPGARFPGVGVLPGVPTGAGVKPK
APGVGGAFAGIPGVGPFGGPQPGVPLGYPIKAPKLPGGYGLPYTTGKLPYGY
GPGGVAGAAGKAGYPTGTGVGPQAAAAAAKAAAKFGAGAAGVLPGVGG
AGVPGVPGAIPGIGGIAGVGTP (SEQ ID NO: 59)

The codon optimized polynucleotide sequence encoding the 24.1 kDa truncated human elastin is disclosed in SEQ ID NO: 60

GGTGGCGTACCAGGCGCAATTCCTGGGGTGTCCCAGGCGGTGTTTTTAT
CCGGGCGCCGGTCTTGGCGCACTGGGTGGCGGTGCACTGGGCCCGGGCGG
CAAACCGCTGAAACCGGTACCAGGTGGTTTAGCAGGCGCCGGCTTAGGCG
CAGGTCTGGGAGCATTTCCGGCAGTTACCTTTCCAGGGGCACTGGTTCCTG
GAGGTGTGGCCGATGCAGCCGCGGCATATAAAGCCGCTAAAGCCGGTGCG
GGTTTAGGAGGCGTCCCAGGTGTCGGTGGCCTGGGTGTTAGCGCCGGTGC
AGTTGTTCCGCAGCCGGGAGCAGGGGTTAAACCTGGTAAAGTGCCGGGAG
TAGGTCTGCCAGGCGTTTATCCTGGTGGTGTTTTGCCGGGTGCCCGTTTC
CGGGCGTTGGTGTTCTTCCAGGCGTGCCGACCGGAGCCGGTGTTAAACCG
AAAGCCCCCGGTGTTGGAGGTGCATTTGCAGGCATCCCGGGAGTTGGCCC
GTTTGGTGGTCCGCAACCTGGGGTTCCGTTAGGTTATCCGATTAAAGCACC
GAAACTGCCCGGCGGTTATGGTCTGCCGTACACAACCGGTAAACTGCCGT
ATGGTTATGGCCCGGGTGGAGTTGCGGGTGCAGCAGGTAAAGCGGGTTAT
CCTACCGGAACCGGTGTAGGTCCGCAGGCCGCTGCTGCCGCCGCCGCAAA
AGCAGCGGCTAAATTTGGCGCCGGAGCAGCGGGTGTTCTGCCTGGAGTTG
GTGGTGCGGGCGTGCCAGGGGTACCTGGTGCAATTCCGGGTATTGGTGGT
ATTGCCGGTGTCGGCACCCCGtaa (SEQ ID NO:60)

The amino acid sequence of a 20.3 kDa human elastin truncated at the C-terminal is disclosed in SEQ ID NO: 61. The 20.3 kDa truncated elastin has amino acids 229-761 deleted from the full length elastin.

GGVPGAIPGGVPGGVFYPGAGLGALGGGALGPGGKPLKPVPGGLAGAGLGA
GLGAFPAVTFPGALVPGGVADAAAAYKAAKAGAGLGGVPGVGGLGVSAGA
VVPQPGAGVKPGKVPGVGLPGVYPGGVLPGARFPGVGVLPGVPTGAGVKPK
APGVGGAFAGIPGVGPFGGPQPGVPLGYPIKAPKLPGGYGLPYTTGKLPYGY
GPGGVAGAAGKAGYPTGTGVGPQ (SEQ ID NO: 61)

The codon optimized polynucleotide sequence encoding the 20.3 kDa truncated human elastin is disclosed in SEQ ID NO: 62

GGTGGCGTACCAGGCGCAATTCCTGGGGGTGTCCCAGGCGGTGTTTTTAT
CCGGGCGCCGGTCTTGGCGCACTGGGTGGCGGTGCACTGGGCCCGGGCGG
CAAACCGCTGAAACCGGTACCAGGTGGTTTAGCAGGCGCCGGCTTAGGCG
CAGGTCTGGGAGCATTTCCGGCAGTTACCTTTCCAGGGGCACTGGTTCCTG
GAGGTGTGGCCGATGCAGCCGCGGCATATAAAGCCGCTAAAGCCGGTGCG
GGTTTAGGAGGCGTCCCAGGTGTCGGTGGCCTGGGTGTTAGCGCCGGTGC
AGTTGTTCCGCAGCCGGGAGCAGGGGTTAAACCTGGTAAAGTGCCGGGAG
TAGGTCTGCCAGGCGTTTATCCTGGTGGTGTTTTGCCGGGTGCCCGTTTTC
CGGGCGTTGGTGTTCTTCCAGGCGTGCCGACCGGAGCCGGTGTTAAACCG
AAAGCCCCCGGTGTTGGAGGTGCATTTGCAGGCATCCCGGGAGTTGGCCC
GTTTGGTGGTCCGCAACCTGGGGTTCCGTTAGGTTATCCGATTAAAGCACC
GAAACTGCCCGGCGGTTATGGTCTGCCGTACACAACCGGTAAACTGCCGT
ATGGTTATGGCCCGGGTGGAGTTGCGGGTGCAGCAGGTAAAGCGGGTTAT
CCTACCGGAACCGGTGTAGGTCCGCAGtaa (SEQ ID NO: 62)

The amino acid sequence of a19.6 kDa human elastin truncated at the N-terminal is disclosed in SEQ ID NO: 63. The 19.6 kDa truncated elastin has amino acids 2-542 deleted from the full length elastin.

QLRAAAGLGAGIPGLGVGVGVPGLGVGAGVPGLGVGAGVPGFGAGADEGV
RRSLSPELREGDPSSSQHLPSTPSSPRVPGALAAAKAAKYGAAVPGVLGGLGA
LGGVGIPGGVVGAGPAAAAAAKAAAKAAQFGLVGAAGLGGLGVGGLGVP
GVGGLGGIPPAAAAKAAKYGAAGLGGVLGGAGQFPLGGVAARPGFGLSPIFP
GGACLGKACGRKRK (SEQ ID NO: 63)

The codon optimized polynucleotide sequence encoding the 19.6 kDa truncated human elastin is disclosed in SEQ ID NO: 64

CAACTGCGCGCCGCCGCGGGCCTCGGTGCAGGTATTCCGGGGCTGGGTGT
CGGAGTTGGAGTCCCGGGTTTGGGCGTGGGCGCGGAGTTCCGGACTGG
GAGTGGGTGCCGGAGTTCCTGGCTTTGGTGCAGGCGCAGATGAAGGTGTT

CGTCGTAGCCTGAGTCCGGAACTGCGTGAAGGTGATCCGAGTAGCAGCCA
GCATCTGCCGAGCACCCCGAGCAGCCCGCGTGTTCCGGGTGCATTAGCTG
CAGCAAAAGCCGCCAAGTATGGTGCAGCCGTGCCGGGCGTCTTAGGTGGT
CTGGGCGCCCTGGGTGGTGTAGGCATTCCGGGAGGTGTTGTGGGTGCAGG
ACCGGCCGCCGCAGCTGCGGCCGCCAAAGCAGCTGCAAAAGCGGCCCAG
TTTGGTTTAGTGGGCGCCGCAGGTTTAGGCGGTTTAGGTGTGGGTGGACTG
GGTGTACCTGGCGTAGGCGGTCTGGGTGGAATTCCGCCCGCAGCGGCCGC
GAAAGCGGCAAAATATGGCGCGGCAGGCCTGGGCGGCGTGCTGGGTGGG
GCAGGTCAGTTTCCGCTGGGCGGGGTTGCCGCACGTCCGGGATTTGGTCT
GAGCCCGATTTTCCCTGGCGGCGCATGTCTGGGTAAAGCATGTGGTCGTA
AACGTAAAtaa (SEQ ID NO: 64)

The amino acid sequence of a 11 kDa human elastin truncated at the N-terminal is disclosed in SEQ ID NO: 65. The 11 kDa truncated elastin has amino acids 2-635 deleted from the full length elastin.

VPGVLGGLGALGGVGIPGGVVGAGPAAAAAAAKAAAKAAQFGLVGAAGLG
GLGVGGLGVPGVGGLGGIPPAAAAKAAKYGAAGLGGVLGGAGQFPLGGVA
ARPGFGLSPIFPGGACLGKACGRKRK (SEQ ID NO: 65)

The codon optimized polynucleotide sequence encoding the 11 kDa truncated human elastin is disclosed in SEQ ID NO: 66

GTGCCGGGCGTCTTAGGTGGTCTGGGCGCCCTGGGTGGTGTAGGCATTCC
GGGAGGTGTTGTGGGTGCAGGACCGGCCGCCGCAGCTGCGGCCGCCAAA
GCAGCTGCAAAAGCGGCCCAGTTTGGTTTAGTGGGCGCCGCAGGTTTAGG
CGGTTTAGGTGTGGGTGGACTGGGTGTACCTGGCGTAGGCGGTCTGGGTG
GAATTCCGCCCGCAGCGGCCGCGAAAGCGGCAAAATATGGCGCGGCAGG
CCTGGGCGGCGTGCTGGGTGGGGCAGGTCAGTTTCCGCTGGGCGGGGTTG
CCGCACGTCCGGGATTTGGTCTGAGCCCGATTTTCCCTGGCGGCGCATGTC
TGGGTAAAGCATGTGGTCGTAAACGTAAAtaa (SEQ ID NO: 66)

The amino acid sequence of a 7.9 kDa human elastin truncated at the N-terminal is disclosed in SEQ ID NO: 67. The 7.9 kDa truncated elastin has amino acids 2-674 deleted from the full length elastin.

QFGLVGAAGLGGLGVGGLGVPGVGGLGGIPPAAAAKAAKYGAAGLGGVLG
GAGQFPLGGVAARPGFGLSPIFPGGACLGKACGRKRK (SEQ ID NO: 67)

The codon optimized polynucleotide sequence encoding the 7.9 kDa truncated human elastin is disclosed in SEQ ID NO: 68

CAGTTTGGTTTAGTGGGCGCCGCAGGTTTAGGCGGTTTAGGTGTGGGTGG
ACTGGGTGTACCTGGCGTAGGCGGTCTGGGTGGAATTCCGCCCGCAGCGG
CCGCGAAAGCGGCAAAATATGGCGCGGCAGGCCTGGGCGGCGTGCTGGG
TGGGGCAGGTCAGTTTCCGCTGGGCGGGGTTGCCGCACGTCCGGGATTTG
GTCTGAGCCCGATTTTCCCTGGCGGCGCATGTCTGGGTAAAGCATGTGGTC
GTAAACGTAAAtaa (SEQ ID NO: 68)

The amino acid sequence of a 6.3 kDa human elastin truncated at the C-terminal is disclosed in SEQ ID NO: 69. The 6.3 kDa truncated elastin has amino acids 74-761 deleted from the full length elastin.

GGVPGAIPGGVPGGVFYPGAGLGALGGGALGPGGKPLKPVPGGLAGAGLGA
GLGAFPAVTFPGALVPGGVAD (SEQ ID NO: 69)

The codon optimized polynucleotide sequence encoding the 6.3 kDa truncated human elastin is disclosed in SEQ ID NO: 70:

GGTGGCGTACCAGGCGCAATTCCTGGGGGTGTCCCAGGCGGTGTTTTTAT
CCGGGCGCCGGTCTTGGCGCACTGGGTGGCGGTGCACTGGGCCCGGGCGG
CAAACCGCTGAAACCGGTACCAGGTGGTTTAGCAGGCGCCGGCTTAGGCG
CAGGTCTGGGAGCATTTCCGGCAGTTACCTTTCCAGGGGCACTGGTTCCTG
GAGGTGTGGCCGATtaa (SEQ ID NO: 70)

The amino acid sequence of a 4.3 kDa human elastin truncated at the N-terminal is disclosed in SEQ ID NO: 71. The 4.3 kDa truncated elastin has amino acids 2-717 deleted from the full length elastin.
GLGGVLGGAGQFPLGGVAARPGFGLSPIFPGGACLGKACGRKRK (SEQ ID NO: 71)

The codon optimized polynucleotide sequence encoding the 4.3 kDa truncated human elastin is disclosed in SEQ ID NO: 72

GGCCTGGGCGGCGTGCTGGGTGGGGCAGGTCAGTTTCCGCTGGGCGGGGT
TGCCGCACGTCCGGGATTTGGTCTGAGCCCGATTTTCCCTGGCGGCGCATG
TCTGGGTAAAGCATGTGGTCGTAAACGTAAAtaa (SEQ ID NO: 72)

### Truncated Human Elastin 1 with DsbA secretion and FLAG tag

The amino acid sequence of truncated human elastin 1 with DsbA secretion and FLAG tag is disclosed in SEQ ID NO: 98. The DsbA secretion tag is encoded by nucleotides 1-57 of SEQ ID NO: 99 and the amino acid sequences are amino acids 1-19 of SEQ ID NO: 98. The elastin nucleotide sequences are nucleotides 58-657 of SEQ ID NO: 99 and the amino acid sequences are amino acids 20-219 of SEQ ID NO: 98. The FLAG nucleotide sequences are nucleotides 658-684 of SEQ ID NO: 99 and the amino acid sequences are amino acids 220-228 of SEQ ID NO: 98.

MKKIWLALAGLVLAFSASAGGVPGAIPGGVPGGVFYPGAGLGALGGGALGP
GGKPLKPVPGGLAGAGLGAGLGAFPAVTFPGALVPGGVADAAAAYKAAKA
GAGLGGVPGVGGLGVSAGAVVPQPGAGVKPGKVPGVGLPGVYPGGVLPGA
RFPGVGVLPGVPTGAGVKPKAPGVGGAFAGIPGVGPFGGPQPGVPLGYPIKA
PKLPGGYGLPYTTGKLGDYKDDDDK (SEQ ID NO: 98)

The nucleic acid sequence of truncated human elastin 1 with DsbA secretion and FLAG tag is disclosed in SEQ ID NO: 99.

ATGAAAAAGATTTGGCTGGCGCTGGCTGGTTTAGTTTTAGCGTTTAGCGCA
TCGGCGGGTGGCGTACCAGGCGCAATTCCTGGGGGTGTCCCAGGCGGTGT
TTTTTATCCGGGCGCCGGTCTTGGCGCACTGGGTGGCGGTGCACTGGGCCC
GGGCGGCAAACCGCTGAAACCGGTACCAGGTGGTTTAGCAGGCGCCGGCT
TAGGCGCAGGTCTGGGAGCATTTCCGGCAGTTACCTTTCCAGGGGCACTG
GTTCCTGGAGGTGTGGCCGATGCAGCCGCGGCATATAAAGCCGCTAAAGC
CGGTGCGGGTTTAGGAGGCGTCCCAGGTGTCGGTGGCCTGGGTGTTAGCG
CCGGTGCAGTTGTTCCGCAGCCGGGAGCAGGGGTTAAACCTGGTAAAGTG
CCGGGAGTAGGTCTGCCAGGCGTTTATCCTGGTGGTGTTTTGCCGGGTGCC
CGTTTTCCGGGCGTTGGTGTTCTTCCAGGCGTGCCGACCGGAGCCGGTGTT
AAACCGAAAGCCCCCGGTGTTGGAGGTGCATTTGCAGGCATCCCGGGAGT
TGGCCCGTTTGGTGGTCCGCAACCTGGGGTTCCGTTAGGTTATCCGATTAA
AGCACCGAAACTGCCCGGCGGTTATGGTCTGCCGTACACAACCGGTAAAC
TGGGTGACTACAAAGACGACGACGACAAAtaa (SEQ ID NO: 99)

The polynucleotide of SEQ ID NO: 99 was subcloned into vector pET28a, expressed host *E. coli* cells and the truncated elastin was purified as described herein. The purified elastin produced a clear band on SDS-PAGE and an anti-FLAG western was observed at around 25 kilodaltons. There were no existing bands that appear at that location on the gel in the absence of expression of this protein.

### Truncated Human Elastin 2 with DsbA secretion and FLAG tag

The amino acid sequence of truncated human elastin type 2 with DsbA secretion and FLAG tag is disclosed in SEQ ID NO: 100. The DsbA secretion tag is encoded by nucleotides 1-57 of SEQ ID NO: 101 and the amino acid sequences are amino acids 1-19 of SEQ ID NO: 100. The elastin nucleotide sequences are nucleotides 58-657 of SEQ ID NO: 101 and the amino acid sequences are amino acids 20-219 of SEQ ID NO: 100. The FLAG nucleotide sequences are nucleotides 658-684 of SEQ ID NO: 101 and the amino acid sequences are amino acids 220-228 of SEQ ID NO: 100.

MKKIWLALAGLVLAFSASAPYGYGPGGVAGAAGKAGYPTGTGVGPQAAAA
AAAKAAAKFGAGAAGVLPGVGGAGVPGVPGAIPGIGGIAGVGTPAAAAAA
AAAKAAKYGAAAGLVPGGPGFGPGVVGVPGAGVPGVGVPGAGIPVVPGAGI
PGAAVPGVVSPEAAAKAAAKAAKYGARPGVGVGGIPTYGVGAGGFPGFGV
GVGGIPGVAGVPGVGGVGDYKDDDDK (SEQ ID NO: 100)

The nucleic acid sequence of truncated human elastin 2 with DsbA secretion and FLAG tag is disclosed in SEQ ID NO: 101.

ATGAAAAAGATTTGGCTGGCGCTGGCTGGTTTAGTTTTAGCGTTTAGCGCA
TCGGCGCCGTATGGTTATGGCCCGGGTGGAGTTGCGGGTGCAGCAGGTAA
AGCGGGTTATCCTACCGGAACCGGTGTAGGTCCGCAGGCCGCTGCTGCCG
CCGCCGCAAAAGCAGCGGCTAAATTTGGCGCCGGAGCAGCGGGTGTTCTG
CCTGGAGTTGGTGGTGCGGGCGTGCCAGGGGTACCTGGTGCAATTCCGGG
TATTGGTGGTATTGCCGGTGTCGGCACCCCGGCCGCGGCAGCTGCGGCAG
CGGCGGCTGCCAAAGCTGCTAAATACGGTGCCGCGGCGGGTCTGGTGCCA
GGAGGTCCGGGTTTTGGTCCGGGAGTGGTTGGCGTGCCTGGCGCAGGCGT
TCCTGGTGTGGGCGTTCCAGGTGCAGGGATTCCTGTTGTGCCTGGTGCCGG
TATTCCCGGCGCGGCCGTTCCGGGGGTGGTTAGCCCGGAAGCCGCAGCGA
AGGCTGCGGCAAAGGCAGCAAAGTATGGCGCACGCCCAGGAGTCGGCGT
GGGTGGTATCCCGACCTATGGGGTGGGCGCAGGGGGTTTTCCTGGTTTCG
GCGTAGGTGTAGGAGGTATACCGGGCGTGGCCGGTGTACCAGGGGTTGGT
GGCGTCGGTGACTACAAAGACGACGACGACAAAtaa (SEQ ID NO: 101)

The polynucleotide of SEQ ID NO: 101 was subcloned into vector pET28a, expressed host *E. coli* cells and the truncated elastin was purified as described herein. The purified elastin produced a clear band on SDS-PAGE and an anti-FLAG western was observed at around 25 kilodaltons. There were no existing bands that appear at that location on the gel in the absence of expression of this protein.

### Example 6: Effect of Truncated Collagen on fibroblast Cell Viability,

### Procollagen Synthesis, and Elastin Synthesis

A human fibroblast cell culture was used to assess the ability of the truncated jellyfish collagen molecule of Example 2 to determine its effect on procollagen, and elastin synthesis. The human fibroblast cell culture was also used to determine the increased viability of the human fibroblast cells after exposure to the truncated jellyfish collagen.

A stock solution of 2% w/w truncated collagen was prepared from the histidine tagged truncated collagen of example 3. Aliquots from the 2% stock truncated collagen solution were then used in the experiments described below.

### Preparation of Fibroblasts

Fibroblasts were seeded into the individual wells of a 24-well plate in 0.5 ml of Fibroblast Growth Media (FGM) and incubated overnight at 37 ± 2° C and 5 ± 1% CO₂. On the following day the media was removed via aspiration to eliminate any non-adherent cells and replaced with 0.5 ml of fresh FGM. The cells were grown until confluent, with a media change every 48 to 72 hours. Upon reaching confluency the cells were treated for 24 hours with DMEM supplemented with 1.5% FBS to wash out any effects from the growth factors included in the normal culture media. After the 24-hour wash out period the cells were treated with the truncated jellyfish collagen at specified concentrations dissolved in FGM with 1.5% FBS. Transforming Growth Factor Beta (TGF-β) (20 ng/ml) was used as a positive control for collagen and elastin synthesis. Untreated cells (negative controls) just received DMEM with 1.5% FBS. The cells were incubated for 48 hours and at the end of the incubation period cell culture medium was collected and either stored frozen (-75°C) or assayed immediately. Materials were tested in triplicate.

### MTT Assay

The MTT (3-(4,5-Dimethylthiazol-2-yl)-2,5-diphenyltetrazolium bromide, a tetrazole) assay is a colorimetric assay used to determine the metabolic activity of cells. Changes in cell number were assessed via an MTT assay. When cells are exposed to MTT, reduction of MTT by mitochondria in viable cells results in the formation of insoluble purple formazin crystals that are extracted from the cells with isopropanol and quantified spectrophotometrically. Non-living cells cannot reduce MTT and therefore cannot produce the purple formazin crystals. The intensity of the purple color is directly proportional to the number of living cells (metabolically active cells). The intensity of the purple color is directly proportional to the metabolic activity of the cells and is inversely proportional to the toxicity of the test material.

After the 2-day incubation discussed above, the cell culture medium was removed (see above) and the fibroblasts were washed twice with PBS to remove any remaining jellyfish glycogen molecules. After the final wash, 500 µl of DMEM supplemented with 0.5 mg/ml MTT was added to each well and the cells were incubated for 1 hour at 37 ± 2° C and 5 ± 1% CO₂. After the incubation, the DMEM/MTT solution was removed and the cells were washed again once with PBS and then 0.5 ml of isopropyl alcohol was added to the well to extract the purple formazin crystals. Two hundred microliters of the isopropyl extracts was transferred to a 96-well plate and the plate was read at 540 nm using isopropyl alcohol as a blank.

The mean MTT absorbance value for the negative control cells was calculated and used to represent 100% cell viability. The individual MTT absorbance values from the cells undergoing the various treatments were then divided by the mean value for the negative control cells and expressed as a percent to determine the change in cell viability caused by each treatment.

In Tables 1, 2 and 3 of this example, the experiments were performed by using the designated aliquots of the 2% stock truncated collagen solution in the assays. For example, in the samples that tested the "10% Collagen Solution," an aliquot of the 2% truncated collagens in an amount sufficient to provide 10% of the assay volume was used. For a total assay volume of 1.0 ml, 100µl of the 2% stock truncated collagen solution was used. In Tables 1, 2 and 3, "10% Collagen Solution" is 0.2% collagen, "5% Collagen Solution" is 0.1% collagen, "1% Collagen Solution" is 0.02% collagen, "0.5% Collagen Solution" is 0.01% collagen, "0.1% Collagen Solution" is 0.002% collagen, "0.05% Collagen Solution" is 0.001% collagen, "0.01% Collagen Solution" is 0.0002% collagen, "0.005% Collagen Solution" is 0.0001% collagen,. f

The results for the MTT assay are presented in Table 3. The values are presented as the mean percent viability ± the deviation from the mean.

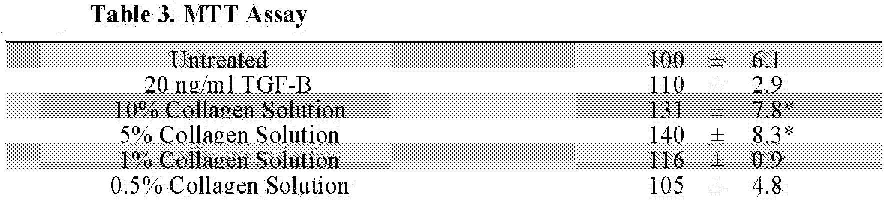

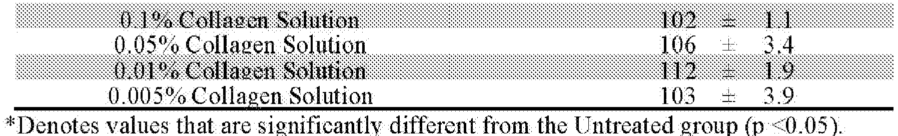

The histidine tagged truncated jellyfish collagen showed protective effect by increasing the cell viability of human fibroblast cells. As can be seen in Table 3, the highest value in the MTT assay was observed when the fibroblast cells were exposed to 0.02% to 0.2% truncated jellyfish collagen.

### Procollagen Synthesis

Fibroblasts are the main source of the extracellular matrix peptides, including the structural proteins collagen and elastin. Procollagen is a large peptide synthesized by fibroblasts in the dermal layer of the skin and is the precursor for collagen. As the peptide is processed to form a mature collagen protein, the propeptide portion is cleaved off (type I C-peptide). Both the mature collagen protein and the type I C-peptide fragment are then released into the extracellular environment. As collagen is synthesized the type I C-peptide fragment accumulates into the tissue culture medium. Since there is a 1:1 stoichiometric ratio between the two parts of the procollagen peptide, assaying for type I C-peptide will reflect the amount of collagen synthesized. Type 1 C-peptide can be assayed via an ELISA based method.

A series of type I C-peptide standards was prepared ranging from 0 ng/ml to 640 ng/ml. Next, an ELISA microplate was prepared by removing any unneeded strips from the plate frame followed by the addition of 100 µl of peroxidase-labeled anti procollagen type I-C peptide antibody to each well used in the assay. Twenty (20) µl of either sample (collected tissue culture media) or standard was then added to appropriate wells and the microplate was covered and allowed to incubate for 3 ± 0.25 hours at 37°C. After the incubation the wells were aspirated and washed three times with 400 µl of wash buffer. After the last wash was removed 100 µl of peroxidase substrate solution (hydrogen peroxide + tetramethylbenzidine as a chromagen) was added to each well and the plate was incubated for 15 ± 5 minutes at room temperature. After the incubation 100 µl of stop solution (1 N sulfuric acid) was added to each well and the plate was read using a microplate reader at 450 nm.

To quantify the amount of each substance present, a standard curve was generated using known concentrations of each substance. A regression analysis was performed to establish the line that best fits these data points. Absorbance values for the test materials and untreated samples were used to estimate the amount of each substance present in each sample.

The results for the ELISA assays are presented in Table 4.

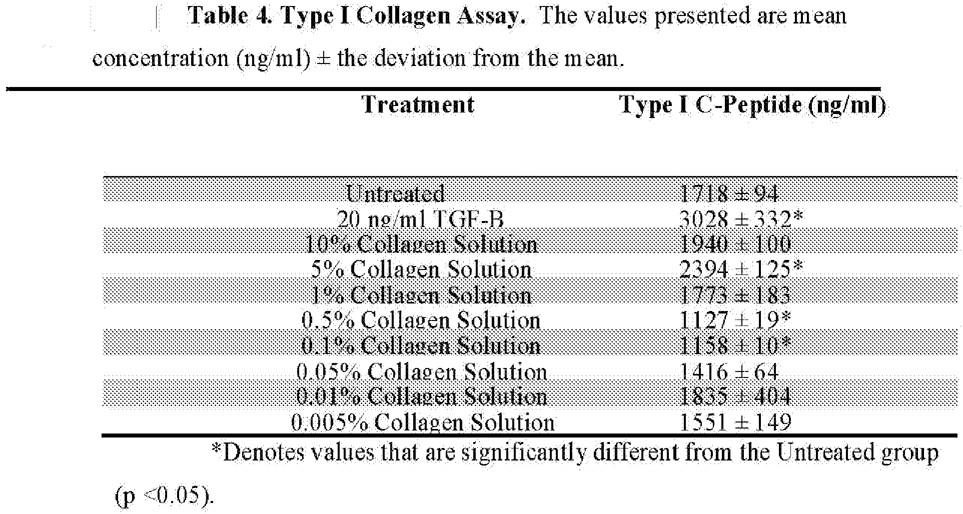

The truncated histidine tagged jellyfish collagen was observed to have a biphasic effect on collagen synthesis. At the 1%, 5% and the 10% levels, collagen synthesis increased. At the 5% concentration truncated jellyfish collagen significantly increased collagen synthesis with a p-value of less than 0.05.

### Elastin Synthesis

Elastin is the main component of a network of elastic fibers that give tissues their ability to recoil after a transient stretch. This protein is released by fibroblasts (soluble elastin) into the extracellular space where it is then cross-linked to other elastin proteins to form an extensive network of fibers and sheets (insoluble elastin). Soluble elastin can be readily measured from cell culture medium via an ELISA based method.

Soluble α-elastin was dissolved in 0.1 M sodium carbonate (pH 9.0) at a concentration of 1.25 µg/ml. 150 µl of this solution was then applied to the wells of a 96-well maxisorp Nunc plate and the plate was incubated overnight at 4°C. On the following day the wells were saturated with PBS containing 0.25% BSA and 0.05% Tween 20. The plate was then incubated with this blocking solution for 1 hour at 37°C and then washed two times with PBS containing 0.05% Tween 20.

A set of α-elastin standards was generated ranging from 0 to 100 ng/ml. 180 µl of either standard or truncated jellyfish collagen was then transferred to a 650 µl microcentrifuge tube. An anti-elastin antibody solution was prepared (the antibody was diluted 1:100 in PBS containing 0.25% BSA and 0.05% Tween 20) and 20 µl of the solution was added to the tube. The tubes were then incubated overnight at 4 ± 2°C. On the following day, 150 µl was transferred from each tube to the 96-well elastin ELISA plate, and the plate was incubated for 1 hour at room temperature. The plate was then washed 3 times with PBS containing 0.05% Tween 20. After washing, 200 µl of a solution containing a peroxidase linked secondary antibody diluted in PBS containing 0.25% BSA and 0.05% Tween 20 was added, and the plate was incubated for 1 hour at room temperature. After washing the plate three times, 200 µl of a substrate solution was added and the plate was incubated for 10 to 30 minutes in the dark at room temperature. After this final incubation the plate was read at 460 nm using a plate reader.

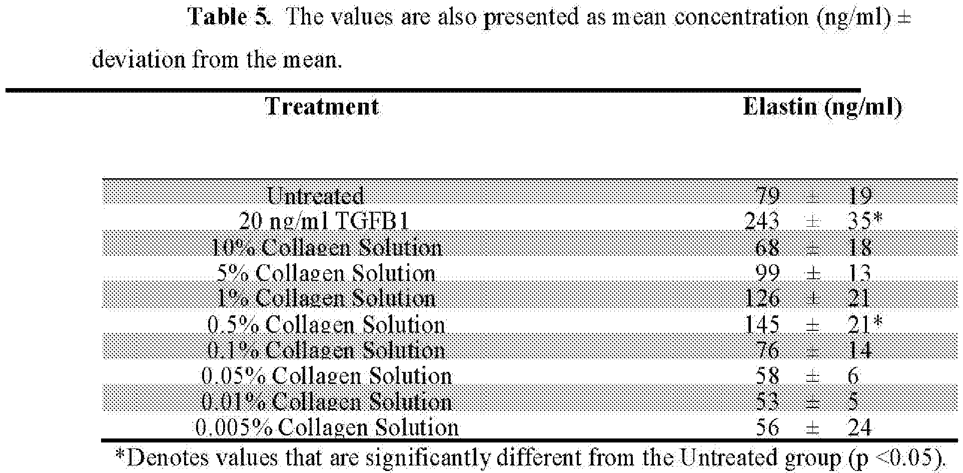

Truncated his-tagged jellyfish collagen significantly increased elastin production when it was used at the 0.5% concentration as shown in Table 5.

### Example 7: Effect of Truncated Collagen on Keratinocyte Proliferation and UVB Protection

A human keratinocyte cell culture model was used to assess the ability of the test materials to exert an effect on cell proliferation. In addition, the impact of the test materials on the cell viability after an exposure to UVB was also assessed.

A stock solution of 2% w/w truncated collagen was prepared from the truncated collagen of example 1. Aliquots from the 2% stock truncated collagen solution was then used in the experiments described below.

This study was conducted in two parts. In the first part cultured keratinocytes were incubated with the test materials for 48 hours, after which changes in the number of viable cells were assessed using an MTT assay. In the second part of the study cultured keratinocytes were irradiated with UVB and then treated with the test materials for 48 hours. At the end of the 48 hour period the number of viable cells was again assessed via an MTT assay.

Changes in cell number of viable cells can be determined using an MTT (3-(4,5-Dimethylthiazol-2-yl)-2,5- diphenyltetrazolium bromide, a tetrazole) assay. The MTT assay is a colorimetric analysis of the metabolic activity of the cell, which is a reflection of the number of viable cells. Reduction of MTT by mitochondria in viable cells results in the formation of insoluble purple formazin crystals that are extracted from the cells with isopropanol and quantified spectrophotometrically. The intensity of the purple color is directly proportional to the number of metabolically active cells.

### Proliferation Assay

For the proliferation assay the keratinocytes were seeded into 96-welll plates using without growth factors and incubated for 24 hours at 37±2°C and 5±1% CO2. After this initial incubation the media was replaced with media supplemented with the test materials. Normal media (with growth factors) was used as a positive control. After the addition of the test materials the cells were cultured for 48 hours as described above. At the end of the incubation period changes in the number of viable cells was determined using an MTT assay.

### UVB Protection Assay

For the UVB protection assay the keratinocytes were seeded into 96-welll plates using normal media and incubated for 24 hours at 37±2°C and 5±1% CO2. After this initial incubation the media was replaced with 100 µl of phosphate buffered saline (PBS) and the cells were exposed to UVB (40 mJ/cm2). After the UVB exposure, the PBS was replaced with fresh media supplemented with the test materials (ascorbic acid at 100 µg/ml served as the positive control) and the cells were cultured for 48 hours at 37±2°C and 5±1% CO2. At the end of the 48 hour incubation cell viability was determined using an MTT assay.

### MTT Assay

After the 48-hour incubation the cell culture medium was removed and replaced with 200 µl of culture media supplemented with 0.5 mg/ml MTT, The well plates were incubated for 1 hour at 37±2oC and 5±1% CO2. After the incubation, the MTT solution was removed and the cells were washed once with phosphate buffered saline and then 200 µl of isopropyl alcohol was added to the well to extract the purple formazin crystals. The 96-well plate was read at 540 nm using isopropyl alcohol as a blank.

The mean absorbance value for the cells not treated with test materials (proliferation assay: Untreated Group) or not exposed to UVB (UVB protection assay: Non-UVB Exposed Group) was calculated and used to represent 100% cell viability. The individual absorbance values from the cells undergoing the various treatments were then divided by the mean absorbance value representing 100% cell viability and expressed as a percent to determine the change in cell viability caused by each treatment.

The results for the Proliferation assay using the his-tagged truncated jellyfish collagen are presented in Table 6. The results for the UVB Protection assay using the his-tagged truncated jellyfish collagen are presented in Table 7. The values for both assays are presented as mean viability ± standard deviation.

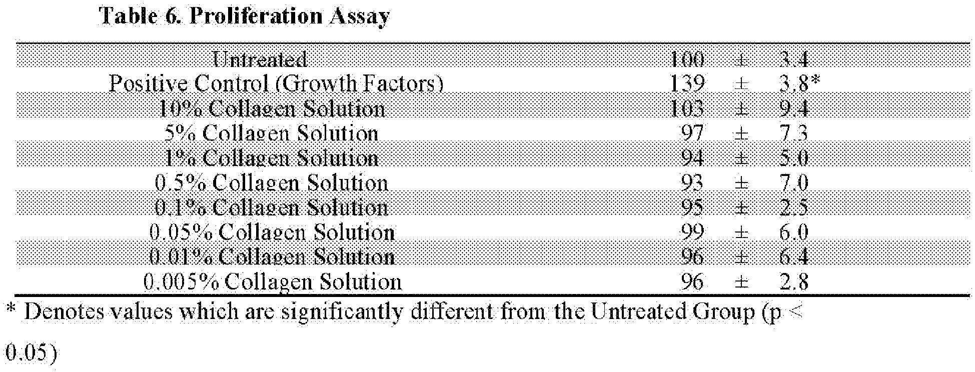

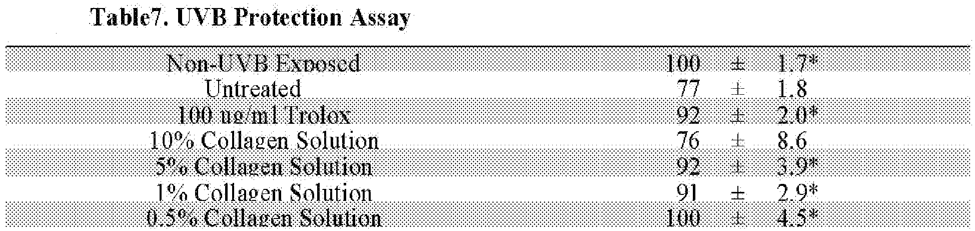

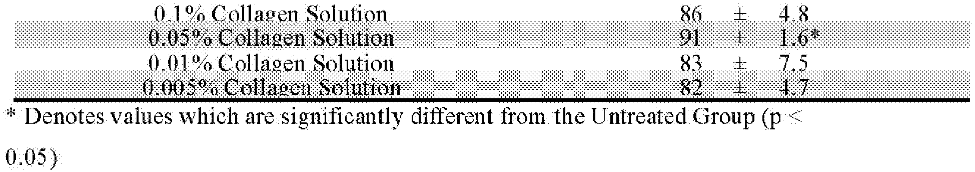

For the proliferation assay the Untreated group was used to represent 100% cell viability. Values above 100% reflect an increase in the number of viable cells and hence are indicative of cell proliferation. In this study, the test material was not observed to promote cell proliferation.

In addition, the keratinocyte proliferation assay was performed using the truncated collagen of SEQ ID NO: 91. A 1% and 0.5% collagen solution of a 5% stock solution was prepared according to Example 8 and tested. The truncated collagen of SEQ ID NO: 91 had keratinocyte cell viability assay values of 102 ± 2.9 and 102 ± 2.0, respectively. The observed values were statistically significant (p <0.05).

In addition to its effects on cell proliferation, the test material was also screened to determine if it had an impact on cell recovery after UVB exposure. In this study, exposure to UVB was observed to result in a significant reduction in the number of viable cells 48 hours post exposure. However, treatment with the test material prevented this decrease in cell viability. The effect was evident within a concentration range between 0.05% and 5% of the test material, with an optimal effect at a concentration of 0.05%. Within this range of concentrations cell viability was significantly greater than the untreated group (with the lone exception of the 0.01% concentration), demonstrating that the material has UVB protective effect. Since this material was added after the UVB exposure it could be acting to reduce the damaging effects of the UVB irradiation, or it could be helping the damaged cells to recover at a faster rate. With respect to the latter, then truncated collagen is beneficial when applied topically to the skin and has a regenerative effect on skin cells damaged by UVB.

In addition, the UVB protection assay was performed using the truncated collagen of SEQ ID NO: 91. A 1% and 0.5% collagen solution of SEQ ID NO: 91 had keratinocyte cell viability assay values of 80 ± 4.6 and 78 ± 2.5, respectively. The observed values were statistically significant (p <0.05).

### Example 8: Effect of Truncated Collagen on Thymine Dimer Formation

Upon exposure to ultraviolet radiation the thymine dimer (TT dimer) content in DNA present in cells increases. Increases in TT dimer formation are correlated with skin damage and certain types of cell proliferative diseases including skin cancer.

The polynucleotide of SEQ ID NO: 11 was expressed in the expression system of Example 1 and purified as described in this example. The encoded polypeptide includes the DsbA secretion tag. As the polypeptide is processed through the secretion pathway, the DsbA tag, amino acids 1-24 of SEQ ID NO: 12 is cleaved by the host cell. The truncated collagen without the DsbA secretion tag is provided in SEQ ID NO: 91.

The truncated collagen of SEQ ID NO: 91 was tested to determine if it could reduce TT dimer formation is human epidermal keratinocytes. For this study, the cells were exposed to UVB (25 mJ/cm2). Following the exposures cells were treated with the test materials or Trolox (100 ug/ml) and incubated overnight. On the following day cellular DNA was extracted and assayed for thymine dimer content using an ELISA based method.

Human keratinocytes were seeded into 12-well plates using normal media and incubated for 24 hours at 37±2°C and 5±1% CO2. After this initial incubation the media was replaced with 100 µl of phosphate buffered saline (PBS) and the cells were exposed to UVB (25 mJ/cm2). After the UVB exposure, the PBS was replaced with fresh media supplemented with the test materials or Trolox (100 µg/ml, this served as the positive control) and the cells were cultured overnight at 37±2°C and 5±1% CO2. At the end of the incubation cellular DNA was extracted.

After the overnight incubation the cell culture media was removed from the wells and replaced with 200 µl of PBS and 20 µl of Proteinase K. After swirling the plate to mix the PBS and Proteinase K, 200 µl of buffer AL was added to each well. After again swirling the plate to mix the reagents, the plates were incubated for 10 minutes at 55±2°C. After cooling the plate to room temperature, the DNA was precipitated by the addition of 200 µl of 100% ethanol. The precipitated DNA mixtures were then transferred to DNEasy Spin Columns in 2 ml collection tubes and centrifuged at 8,000 RPM for 1 minute. The flow through and collection tubes were discarded, and 500 µl of Wash Buffer One was added to the spin column and the column was placed into a new collection tube and centrifuged at 8,000 RPM for 1 minute. The flow through and collection tube were again discarded, and 500 µl of Wash Buffer Two was added to the spin column and the column was placed into a new collection tube and centrifuged at 14,000 RPM for 3 minutes. The spin column was then placed into a new 1.5 ml centrifuge tube and 110 µl of ultrapure water was added to the column. The column was incubated for 1 minute at room temperature and then centrifuged at 8,000 RPM for 1 minute.

Extracted DNA was quantified via a fluorometric assay. A 2 µl aliquot of the DNA sample was mixed with 100 µl TE buffer in a 96-well plate. A series of DNA standards was also transferred to wells in a 96-well plate (in duplicate). Finally, 100 µl of dilute Cyquant Green dye was added to each well and the fluorescence intensity of each well was determined using an excitation wavelength of 480 nm and an emission wavelength of 520 nm.

Thymine Dimer Detection was determined using an OxiSelect^{™} UV-Induced DNA Damage ELISA Kit)

Aliquots of genomic DNA samples or standards were converted to single stranded DNA by incubating the samples at 95°C for 10 minutes and then chilled on ice. 100 µl or each sample or standard was transferred to a DNA binding ELISA plate and incubated overnight at 4°C. On the following day the wells were rinsed once with 100 µl of PBS and then blocked with 150 µl of Assay Diluent for one hour at room temperature. After removing the Assay Diluent, 100 µl of anti-CPD antibody was added to each well and the plate was incubated for one hour at room temperature. After this incubation, the plate was washed three times with 250 µl of wash buffer per well, and then 150 µl of Blocking Reagent was added to the plate. The plate will be blocked again for one hour at room temperature, and then washed three times as described before. 100 µl of Secondary Antibody was then added to each well and the plate was incubated for 1 hour at room temperature. After washing the plate again, 100 µl of substrate was added to each well and the plate was incubated for 5-20 minutes to allow for color generation in the plate. The color generation reaction was stopped by the addition of 100 µl of stop solution and the plate was read at 460 nm using a plate reader.

To quantify the amount of DNA present, a standard curve was generated using known concentrations of DNA and their respective fluorescence intensity (measured in RFUs or relative fluorescence units). A regression analysis was performed to establish the line that best fits these data points. The Relative Fluorescence Units (RFU) for each unknown sample was then used to estimate the amount of DNA.

A series of DNA standards with known amounts of thymine dimer content were used to generate a standard curve. This standard curve was used to determine the amount of DNA damage in the sample DNA. Means for each treatment group were calculated and compared using an ANOVA. In table 8 and Fig. 5, a standard 5% collagen solution (5g truncated collagen in 95 ml deionized water was further diluted with phosphate buffered saline (PBS) to the indicated percent solution.

**Table 8. Thymine Dimer Assay**

| Treatment | Thymine Dimer, ng/ml |
|---|---|
| Non-UVB Exposed | 0.1 ± 0.2* |
| Untreated | 10.7 ± 1.4* |
| 100 µg/ml Trolox | 1.6 ± 0.3* |
| 5% Truncated Collagen | 3.5 ± 0.3* |
| 1% Truncated Collagen | 5.5 ± 1.0* |
| 0.5% Truncated Collagen | 8.9 ± 0.8 |
| 0.1% Truncated Collagen | 9.2 ± 0.9 |
| 0.05% Truncated Collagen | 9.2 ± 0.3 |

| | |
|---|---|
| *Denotes values which are statistically significantly different from untreated with P<0.05 | |

Table 8 shows that the 5% and the 1% truncated collagen solution reduced TT dimer formation with statistical significance (p < 0.05). The data is presented graphically in Fig. 5.

The experiment was repeated with a different lot of truncated collagen (SEQ ID NO: 91). The amount of TT dimer in ng/ml in non-UVB exposed cells was 1.3 ± 1.2, untreated cells was 18.1 ± 0.4, 100 µg/ml Trolox treated cells was 7.9 ± 0.3, 5% collagen was 13.1 ± 0.2, and 1% collagen was 17.4 ±0.7. The reduction in TT dimer formation for non-UVB exposed cells, Trolox treated cells, 5% collagen treated cells and 1% collagen treated cells was statistically significant compared to untreated cells (p< 0.05).

### Example 9: Human Collagens

### Truncated Human Collagen Type 21 alpha 1

A truncated human collagen type 21 alpha 1 without a His tag, linker, and thrombin cleavage site is disclosed below. The codon-optimized nucleotide sequence encoding this collagen and the amino acid sequence are disclosed below. In SEQ ID NOs: 73 and 74, the DsbA secretion tag is encoded by nucleotides 1-72 and encodes amino acids 1-24. In SEQ ID NOs: 73 and 74, the truncated collagen sequence is encoded by nucleotides 73-633 and encodes amino acids 25-211.

The codon-optimized nucleotide sequence encoding this collagen is provided in SEQ ID NO: 73.

ATGAAAAAGATTTGGCTGGCGCTGGCTGGTTTAGTTTTAGCGTTTAGCGCA
TCGGCGGCGCAGTATGAAGATGCAGGTTTTCCGGGTCTGCCTGGTCCGGC
AGGCGAACCGGGTCGTCATGGTAAAGATGGTCTGATGGGTAGTCCGGGTT
TTAAAGGTGAAGCAGGTTCACCGGGTGCACCTGGTCAGGATGGCACCCGT
GGTGAACCGGGTATTCCGGGATTTCCGGGTAATCGTGGCCTGATGGGTCA
GAAAGGTGAAATTGGTCCGCCTGGTCAGCAGGGTAAAAAAGGCGCACCG
GGTATGCCAGGACTGATGGGTTCAAATGGCAGTCCGGGTCAGCCAGGCAC
ACCGGGTTCAAAAGGTAGCAAAGGCGAACCTGGTATTCAGGGTATGCCTG
GTGCAAGCGGTCTGAAAGGCGAGCCAGGTGCCACCGGTTCTCCGGGTGAA
CCAGGTTATATGGGTCTGCCAGGTATCCAAGGCAAAAAAGGTGATAAAGG
TAATCAGGGCGAAAAAGGCATTCAGGGCCAGAAAGGCGAAAATGGCCGT
CAGGGTATTCCAGGCCAGCAGGGCATCCAGGGTCATCATGGTGCAAAAGG
TGAACGTGGTGAAAAGGGCGAACCAGGTGTTCGTtaa (SEQ ID NO: 73)

The amino acid sequence is disclosed in SEQ ID NO: 74.

MKKIWLALAGLVLAFSASAAQYEDAGFPGLPGPAGEPGRHGKDGLMGSPGF
KGEAGSPGAPGQDGTRGEPGIPGFPGNRGLMGQKGEIGPPGQQGKKGAPGM
PGLMGSNGSPGQPGTPGSKGSKGEPGIQGMPGASGLKGEPGATGSPGEPGYM
GLPGIQGKKGDKGNQGEKGIQGQKGENGRQGIPGQQGIQGHHGAKGERGEK
GEPGVR (SEQ ID NO: 74)

The codon-optimized nucleotide sequence encoding the truncated human collagen type 21 alpha 1 without the DsbA secretion tag collagen is provided in SEQ ID NO: 75.

TGCAGGTTTTCCGGGTCTGCCTGGTCCGGCAGGCGAACCGGGTCGTCATG
GTAAAGATGGTCTGATGGGTAGTCCGGGTTTTAAAGGTGAAGCAGGTTCA
CCGGGTGCACCTGGTCAGGATGGCACCCGTGGTGAACCGGGTATTCCGGG
ATTTCCGGGTAATCGTGGCCTGATGGGTCAGAAAGGTGAAATTGGTCCGC

CTGGTCAGCAGGGTAAAAAGGCGCACCGGGTATGCCAGGACTGATGGG
TTCAAATGGCAGTCCGGGTCAGCCAGGCACACCGGGTTCAAAAGGTAGCA
AAGGCGAACCTGGTATTCAGGGTATGCCTGGTGCAAGCGGTCTGAAAGGC
GAGCCAGGTGCCACCGGTTCTCCGGGTGAACCAGGTTATATGGGTCTGCC
AGGTATCCAAGGCAAAAAAGGTGATAAAGGTAATCAGGGCGAAAAAGGC
ATTCAGGGCCAGAAAGGCGAAAATGGCCGTCAGGGTATTCCAGGCCAGC
AGGGCATCCAGGGTCATCATGGTGCAAAAGGTGAACGTGGTGAAAAGGG
CGAACCAGGTGTTCGTtaa (SEQ ID NO: 75)

The amino acid sequence of truncated human collagen type 21 alpha 1 without the DsbA secretion tag is disclosed in SEQ ID NO: 76.

AGFPGLPGPAGEPGRHGKDGLMGSPGFKGEAGSPGAPGQDGTRGEPGIPGFP
GNRGLMGQKGEIGPPGQQGKKGAPGMPGLMGSNGSPGQPGTPGSKGSKGEP
GIQGMPGASGLKGEPGATGSPGEPGYMGLPGIQGKKGDKGNQGEKGIQGQK
GENGRQGIPGQQGIQGHHGAKGERGEKGEPGVR (SEQ ID NO: 76)

### Truncated Human Collagen type 1 alpha 2 (1)

A truncated human collagen type 1 alpha 2 without a His tag, linker, and thrombin cleavage site is disclosed below. The codon-optimized nucleotide sequence and the amino acid sequences are disclosed below. In SEQ ID NOs: 78 and 79, The DsbA secretion tag is encoded by nucleotides 1-72 and encodes amino acids 1-24. The truncated collagen sequence is encoded by nucleotides 73-636 and encodes amino acids 25-212.

The codon-optimized nucleotide sequence encoding this collagen is provided in SEQ ID NO: 77.

ATGAAAAAGATTTGGCTGGCGCTGGCTGGTTTAGTTTTAGCGTTTAGCGCA
TCGGCGGCGCAGTATGAAGATATGGGTCCGCCTGGTAGCCGTGGTGCAAG
TGGTCCGGCAGGCGTTCGTGGTCCGAATGGTGATGCAGGTCGTCCGGGTG
AACCGGGTCTGATGGGTCCTCGTGGTCTGCCTGGTTCACCGGGTAATATTG
GTCCTGCAGGTAAAGAAGGTCCGGTTGGTCTGCCAGGTATTGATGGCCGT
CCGGGTCCGATTGGTCCAGCCGGTGCACGTGGTGAACCTGGCAATATTGG
TTTTCCGGGTCCTAAAGGTCCGACCGGTGATCCGGGTAAAAATGGTGATA
AAGGTCATGCAGGTCTGGCAGGCGCACGCGGTGCACCTGGTCCGGATGGT
AATAATGGTGCACAGGGTCCACCGGGTCCGCAGGGTGTTCAAGGTGGTAA
AGGCGAACAGGGTCCTGCCGGTCCTCCGGGTTTCAGGGACTGCCTGGTC
CGAGCGGTCCTGCGGGTGAAGTTGGTAAACCTGGTGAACGCGGTCTGCAT

GGTGAATTTGGCCTGCCTGGGCCTGCAGGTCCGCGTGGCGAACGTGGTCC
GCCAGGTGAAAGCGGTGCAGCAGGTCCGACAGGTtaa (SEQ ID NO: 77)

The amino acid sequence is disclosed in SEQ ID NO: 78.

MKKIWLALAGLVLAFSASAAQYEDMGPPGSRGASGPAGVRGPNGDAGRPGE
PGLMGPRGLPGSPGNIGPAGKEGPVGLPGIDGRPGPIGPAGARGEPGNIGFPGP
KGPTGDPGKNGDKGHAGLAGARGAPGPDGNNGAQGPPGPQGVQGGKGEQG
PAGPPGFQGLPGPSGPAGEVGKPGERGLHGEFGLPGPAGPRGERGPPGESGAA
GPTG (SEQ ID NO: 78)

The nucleic acid sequence of truncated human collagen type 1 alpha 2(1) without the DsbA secretion tag is disclosed in SEQ ID NO: 79.

ATGGGTCCGCCTGGTAGCCGTGGTGCAAGTGGTCCGGCAGGCGTTCGTGG
TCCGAATGGTGATGCAGGTCGTCCGGGTGAACCGGGTCTGATGGGTCCTC
GTGGTCTGCCTGGTTCACCGGGTAATATTGGTCCTGCAGGTAAAGAAGGT
CCGGTTGGTCTGCCAGGTATTGATGGCCGTCCGGGTCCGATTGGTCCAGCC
GGTGCACGTGGTGAACCTGGCAATATTGGTTTTCCGGGTCCTAAAGGTCC
GACCGGTGATCCGGGTAAAAATGGTGATAAAGGTCATGCAGGTCTGGCAG
GCGCACGCGGTGCACCTGGTCCGGATGGTAATAATGGTGCACAGGGTCCA
CCGGGTCCGCAGGGTGTTCAAGGTGGTAAAGGCGAACAGGGTCCTGCCGG
TCCTCCGGGTTTTCAGGGACTGCCTGGTCCGAGCGGTCCTGCGGGTGAAGT
TGGTAAACCTGGTGAACGCGGTCTGCATGGTGAATTTGGCCTGCCTGGGC
CTGCAGGTCCGCGTGGCGAACGTGGTCCGCCAGGTGAAAGCGGTGCAGCA
GGTCCGACAGGTtaa (SEQ ID NO: 79)

The amino acid sequence of truncated human collagen type 1 alpha 2(1) without the DsbA secretion tag is disclosed in SEQ ID NO: 80.

MGPPGSRGASGPAGVRGPNGDAGRPGEPGLMGPRGLPGSPGNIGPAGKEGPV
GLPGIDGRPGPIGPAGARGEPGNIGFPGPKGPTGDPGKNGDKGHAGLAGARG
APGPDGNNGAQGPPGPQGVQGGKGEQGPAGPPGFQGLPGPSGPAGEVGKPG
ERGLHGEFGLPGPAGPRGERGPPGESGAAGPTG (SEQ ID NO: 80)

### Truncated Human Collagen type 1 alpha 2 (2)

A truncated human collagen type 1 alpha 2 without a His tag, linker, and thrombin cleavage site is disclosed below. The codon-optimized nucleotide sequence and the amino acid sequences are disclosed below. in SEQ ID NO: 82 and 83, the DsbA secretion tag is encoded by nucleotides 1-72 and encodes amino acids 1-24. The truncated collagen sequence is encoded by nucleotides 73-609 and encodes amino acids 25-203.

The codon-optimized nucleotide sequence encoding this collagen is provided in SEQ ID NO: 81.

ATGAAAAAGATTTGGCTGGCGCTGGCTGGTTTAGTTTTAGCGTTTAGCGCA
TCGGCGGCGCAGTATGAAGATGGTTTTCAGGGTCCTGCCGGTGAACCGGG
TGAACCTGGTCAGACAGGTCCGGCAGGCGCACGTGGTCCTGCAGGTCCTC
CTGGTAAAGCCGGTGAAGATGGTCATCCGGGTAAACCGGGTCGTCCTGGT
GAACGTGGTGTTGTTGGTCCGCAGGGTGCCCGTGGTTTTCCGGGTACTCCG
GGTCTGCCAGGTTTTAAAGGTATTCGTGGTCATAATGGTCTGGATGGTCTG
AAAGGTCAGCCTGGTGCACCGGGTGTTAAAGGTGAACCAGGTGCTCCGGG
TGAAAATGGCACACCGGGTCAGACCGGTGCGCGTGGTCTGCCTGGCGAAC
GCGGTCGTGTTGGTGCACCTGGTCCAGCCGGTGCACGCGGTAGTGATGGT
AGCGTTGGTCCGGTTGGTCCAGCGGGTCCGATTGGTAGCGCAGGTCCACC
GGGTTTTCCAGGCGCACCGGGTCCGAAAGGTGAAATTGGTGCAGTTGGTA
ATGCAGGCCCTGCCGGTCCAGCAGGACCGCGTGGTGAAGTTGGCCTGCCT
GGTCTGtaa (SEQ ID NO: 81)

The amino acid sequence is disclosed in SEQ ID NO: 82.

MKKIWLALAGLVLAFSASAAQYEDGFQGPAGEPGEPGQTGPAGARGPAGPP
GKAGEDGHPGKPGRPGERGVVGPQGARGFPGTPGLPGFKGIRGHNGLDGLK
GQPGAPGVKGEPGAPGENGTPGQTGARGLPGERGRVGAPGPAGARGSDGSV
GPVGPAGPIGSAGPPGFPGAPGPKGEIGAVGNAGPAGPAGPRGEVGLPGL
(SEQ ID NO: 82)

The nucleic acid sequence of truncated human collagen type 1 alpha 2(2) without the DsbA secretion tag is disclosed in SEQ ID NO: 83.

GGTTTTCAGGGTCCTGCCGGTGAACCGGGTGAACCTGGTCAGACAGGTCC
GGCAGGCGCACGTGGTCCTGCAGGTCCTCCTGGTAAAGCCGGTGAAGATG
GTCATCCGGGTAAACCGGGTCGTCCTGGTGAACGTGGTGTTGTTGGTCCGC
AGGGTGCCCGTGGTTTTCCGGGTACTCCGGGTCTGCCAGGTTTAAAGGTA
TTCGTGGTCATAATGGTCTGGATGGTCTGAAAGGTCAGCCTGGTGCACCG
GGTGTTAAAGGTGAACCAGGTGCTCCGGGTGAAAATGGCACACCGGGTCA
GACCGGTGCGCGTGGTCTGCCTGGCGAACGCGGTCGTGTTGGTGCACCTG
GTCCAGCCGGTGCACGCGGTAGTGATGGTAGCGTTGGTCCGGTTGGTCCA
GCGGGTCCGATTGGTAGCGCAGGTCCACCGGGTTTTCCAGGCGCACCGGG

TCCGAAAGGTGAAATTGGTGCAGTTGGTAATGCAGGCCCTGCCGGTCCAG
CAGGACCGCGTGGTGAAGTTGGCCTGCCTGGTCTGtaa (SEQ ID NO: 83)

The amino acid sequence of truncated human collagen type 1 alpha 2(2) without the DsbA secretion tag is disclosed in SEQ ID NO: 84.

GFQGPAGEPGEPGQTGPAGARGPAGPPGKAGEDGHPGKPGRPGERGVVGPQ
GARGFPGTPGLPGFKGIRGHNGLDGLKGQPGAPGVKGEPGAPGENGTPGQTG
ARGLPGERGRVGAPGPAGARGSDGSVGPVGPAGPIGSAGPPGFPGAPGPKGEI
GAVGNAGPAGPAGPRGEVGLPGL (SEQ ID NO: 84)

The polynucleotides of SEQ ID NO: 73, 77 or 81 were subcloned in vector pET28a as described herein to prepare a transformation vector. Host cells were transformed with the vector the polynucleotides were expressed as described in Example 2.

After the fermentation was completed, the truncated human collagen was purified from the fermentation broth using the procedures disclosed in Example 3. The purified truncated human collagens were analyzed using SDS-PAGE and HPLC as disclosed in Example 3.

All three truncated human collagens ran at the expected molecular weights in the SDS-PAGE analysis. In analyzing the truncated human collagens using HPLC, a standard curve using the jellyfish collagen of Example 3 was utilized. The retention times of the human collagens were slightly different than the jellyfish collagen. The retention time of SEQ ID NO: 76 was 5.645 minutes, the retention time of SEQ ID NO: 80 was 5.631 minutes, and SEQ ID NO: 84 ran at two peaks and the retention times were5.531 and 5.7 minutes.

### Truncated Human Collagen Type 1 alpha 2 truncation 5 with DsbA secretion and FLAG tag

The amino acid sequence of truncated human collagen type 1 alpha 2 truncation 5 with DsbA secretion and FLAG tag is disclosed in SEQ ID NO: 92. The DsbA secretion tag is encoded by nucleotides 1-57 of SEQ ID NO: 93 and the amino acid sequences are amino acids 1-19 of SEQ ID NO: 92. The collagen nucleotide sequences are nucleotides 58-657 of SEQ ID NO: 93 and the amino acid sequences are amino acids 20-219 of SEQ ID NO: 92. The FLAG nucleotide sequences are nucleotides 658-684 of SEQ ID NO: 93 and the amino acid sequences are amino acids 220-228.

MKKIWLALAGLVLAFSASAGDQGPVGRTGEVGAVGPPGFAGEKGPSGEAGT
AGPPGTPGPQGLLGAPGILGLPGSRGERGLPGVAGAVGEPGPLGIAGPPGARG
PPGAVGSPGVNGAPGEAGRDGNPGNDGPPGRDGQPGHKGERGYPGNIGPVG
AAGAPGPHGPVGPAGKHGNRGETGPSGPVGPAGAVGPRGPSGPQGIRGDKG
EPGEKGPRGLPGLGDYKDDDDK (SEQ ID NO: 92)

The nucleic acid sequence of truncated human collagen type 1 alpha 2 truncation 5 with DsbA secretion and FLAG tag is disclosed in SEQ ID NO: 93.

ATGAAAAAGATTTGGCTGGCGCTGGCTGGTTTAGTTTTAGCGTTTAGCGCA
TCGGCGGGTGATCAGGGTCCGGTTGGTCGTACCGGTGAAGTTGGTGCAGT
CGGGCCGCCGGGTTTTGCGGGTGAAAAAGGCCCGTCAGGTGAAGCAGGC
ACCGCTGGCCCTCCTGGCACGCCTGGCCCACAGGGTTTACTGGGCGCACC
TGGAATTCTGGGACTGCCGGGCAGCCGTGGAGAACGCGGTTTACCAGGTG
TTGCCGGTGCCGTTGGTGAACCTGGTCCACTGGGCATTGCAGGGCCGCCT
GGCGCACGGGGACCGCCTGGTGCTGTTGGTAGTCCGGGTGTGAATGGTGC
TCCGGGTGAAGCCGGTCGTGACGGTAATCCGGGAAATGACGGCCCGCCAG
GCCGCGATGGTCAGCCGGGTCATAAAGGTGAGCGTGGTTACCCAGGTAAT
ATTGGTCCAGTCGGTGCCGCCGGTGCGCCGGGTCCTCATGGCCCTGTCGGT
CCAGCCGGTAAACATGGTAATCGCGGTGAGACAGGTCCGTCAGGACCAGT
GGGCCCTGCTGGCGCAGTCGGTCCGCGCGGCCGAGTGGCCCTCAGGGTA
TTCGTGGCGATAAAGGGGAACCGGGCGAAAAAGGCCGCGGGTCTGCC
AGGCCTGGGTGACTACAAAGACGACGACGACAAAtaa (SEQ ID NO: 93)

The polynucleotide of SEQ ID NO: 93 was subcloned into vector pET28a, expressed host *E. coli* cells and the truncated collagen was purified as described herein. The purified collagen produced a clear band on SDS-PAGE and an anti-FLAG western was observed at around 100 kilodaltons. There were no existing bands that appear at that location on the gel in the absence of expression of this protein.

### Truncated Human Collagen Type 1 alpha 2 truncation 6 with DsbA secretion and FLAG tag

The amino acid sequence of truncated human collagen type 1 alpha 2 truncation 6 with DsbA secretion and FLAG tag is disclosed in SEQ ID NO: 94. The DsbA secretion tag is encoded by nucleotides 1-57 of SEQ ID NO: 95 and the amino acid sequences are amino acids 1-19 of SEQ ID NO: 94. The collagen nucleotide sequences are nucleotides 58-657 of SEQ ID NO: 95 and the amino acid sequences are amino acids 20-219 of SEQ ID NO: 94. The FLAG nucleotide sequences are nucleotides 658-684 of SEQ ID NO: 95 and the amino acid sequences are amino acids 220-228 of SEQ ID NO: 94.

MKKIWLALAGLVLAFSASAKGHNGLQGLPGIAGHHGDQGAPGSVGPAGPRG
PAGPSGPAGKDGRTGHPGTVGPAGIRGPQGHQGPAGPPGPPGPPGVSGG
GYDFGYDGDFYRADQPRSAPSLRPKDYEVDATLKSLNNQIETLLTPEGSRKN
PARTCRDLRLSHPEWSSGYYWIDPNQGCTMDAIKVYCDFSTGETCIRAQPENI
PAKNWYRSSKDGDYKDDDDK (SEQ ID NO: 94)

The nucleic acid sequence of truncated human collagen type 1 alpha 2 truncation 6 with DsbA secretion and FLAG tag is disclosed in SEQ ID NO: 95.

ATGAAAAAGATTTGGCTGGCGCTGGCTGGTTTAGTTTTAGCGTTTAGCGCA
TCGGCGAAAGGTCACAATGGACTGCAAGGCCTGCCAGGTATTGCAGGTCA
TCATGGTGATCAAGGTGCCCCGGGAAGCGTTGGTCCGGCGGGCCGAGAG
GCCCTGCGGGACCTTCAGGTCCGGCAGGCAAAGATGGTCGGACAGGCCAT
CCGGGCACCGTTGGCCCTGCAGGAATTCGTGGACCGCAGGGTCATCAGGG
ACCTGCTGGTCCGCCAGGTCCCCCGGGCCCTCCGGGACCACCGGGTGTTA
GTGGTGGTGGTTATGATTTTGGCTATGATGGTGATTTTTATCGTGCAGATC
AGCCGCGTAGCGCACCGAGCCTGCGTCCTAAAGATTATGAAGTTGATGCA
ACCCTGAAAAGCCTGAATAATCAGATTGAAACACTGCTGACACCGGAAGG
TAGCCGTAAAAATCCGGCCCGTACCTGTCGTGATCTGCGTCTGAGCCACC
CGGAATGGAGCAGCGGTTATTATTGGATTGATCCGAATCAAGGTTGTACC
ATGGATGCAATTAAAGTTTATTGTGATTTTAGCACAGGTGAAACATGTATC
CGTGCACAGCCGGAAAATATTCCGGCCAAAAATTGGTATCGTAGTAGCAA
AGATGGTGACTACAAAGACGACGACGACAAAtaa (SEQ ID NO: 95)

The polynucleotide of SEQ ID NO: 94 was subcloned into vector pET28a, expressed host *E. coli* cells and the truncated collagen was purified as described herein. The purified collagen produced a clear band on SDS-PAGE and an anti-FLAG western was observed at around 25 kilodaltons. There were no existing bands that appear at that location on the gel in the absence of expression of this protein.

### Truncated Human Collagen Type 1 alpha 2 truncation 7 with DsbA secretion and FLAG tag

The amino acid sequence of truncated human collagen type 1 alpha 2 truncation 7 with DsbA secretion and FLAG tag is disclosed in SEQ ID NO: 96. The DsbA secretion tag is encoded by nucleotides 1-57 of SEQ ID NO: 97 and the amino acid sequences are amino acids 1-19 of SEQ ID NO: 96. The collagen nucleotide sequences are nucleotides 58-759 of SEQ ID NO: 96 and the amino acid sequences are amino acids 20-253 of SEQ ID NO: 96. The FLAG nucleotide sequences are nucleotides 760-786 of SEQ ID NO: 97 and the amino acid sequences are amino acids 254-262 of SEQ ID NO: 96.

MKKIWLALAGLVLAFSASAYEVDATLKSLNNQIETLLTPEGSRKNPARTCRD
LRLSHPEWSSGYYWIDPNQGCTMDAIKVYCDFSTGETCIRAQPENIPAKNWY
RSSKDKKHVWLGETINAGSQFEYNVEGVTSKEMATQLAFMRLLANYASQNI
TYHCKNSIAYMDEETGNLKKAVILQGSNDVELVAEGNSRFTYTVLVDGCSKK
TNEWGKTIIEYKTNKPSRLPFLDIAPLDIGGADQEFFVDIGPVCFKGDYKDDD
DK (SEQ ID NO: 96)

The nucleic acid sequence of truncated human collagen type 1 alpha 2 truncation 7 with DsbA secretion and FLAG tag is disclosed in SEQ ID NO: 97.

TGAAAAAGATTTGGCTGGCGCTGGCTGGTTTAGTTTTAGCGTTTAGCGCAT
CGGCGTATGAAGTTGATGCAACCCTGAAAAGCCTGAATAATCAGATTGAA
ACACTGCTGACACCGGAAGGTAGCCGTAAAAATCCGGCCCGTACCTGTCG
TGATCTGCGTCTGAGCCACCCGGAATGGAGCAGCGGTTATTATTGGATTG
ATCCGAATCAAGGTTGTACCATGGATGCAATTAAAGTTTATTGTGATTTTA
GCACAGGTGAAACATGTATCCGTGCACAGCCGGAAAATATTCCGGCCAAA
AATTGGTATCGTAGTAGCAAAGATAAAAAACATGTGTGGCTGGGTGAAAC
CATTAATGCAGGTAGCCAGTTTGAATACAATGTTGAAGGTGTTACCAGCA
AAGAAATGGCAACACAGCTGGCATTTATGCGTCTGCTGGCAAATTATGCA
AGCCAGAATATTACATATCATTGTAAAAATAGCATTGCATATATGGATGA
AGAAACCGGTAATCTGAAAAAAGCAGTTATTCTGCAGGGTAGCAATGATG
TTGAACTGGTTGCCGAAGGTAATAGCCGTTTTACATATACCGTTCTGGTTG
ATGGTTGTAGCAAAAAAACCAATGAATGGGGTAAAACCATCATTGAATAT
AAAACCAACAAACCGAGCCGTCTGCCGTTTCTGGATATCGCTCCGCTGGA
TATTGGTGGTGCCGATCAGGAATTTTTTGTCGATATCGGTCCTGTGTGTTTT
AAAGGTGACTACAAAGACGACGACGACAAAtaa (SEQ ID NO: 97)

The polynucleotide of SEQ ID NO: 97 was subcloned into vector pET28a, expressed host *E. coli* cells and the truncated collagen was purified as described herein. The purified collagen produced a clear band on SDS-PAGE and an anti-FLAG western was observed at around 30 kilodaltons. There were no existing bands that appear at that location on the gel in the absence of expression of this protein.

### Example 10: Protective Effect of Truncated Human Collagen on Fibroblasts

The effect of truncated human collagen on fibroblast cell viability, procollagen synthesis, and elastin Synthesis is determined according to the methods of Example 6.

The effect of truncated human collagen on Keratinocyte proliferation and UVB protection is determined according to the methods of Example 7.

The effect of truncated collagen on thymine dimer formation after exposure to UV radiation is determined according to the methods of Example 8.

It is understood that the examples and embodiments described herein are for illustrative purposes only and that various modifications or changes in light thereof will be suggested to persons skilled in the art and are to be included within the spirit and purview of this application and scope of the appended claims.

### Example 11: Effect of Truncated Collagen on Inflammatory Cytokines

Keratinocytes and dermal fibroblasts play an important role in the immune response of the skin. In response to irritating chemicals or UV radiation (pro-inflammatory/pro-irriation stimuli), keratinocytes can release a vast array of cytokines. These cytokines are thought to help engage immune cells to the site of inflammation. Cytokines released by the keratinocytes include TNFα, IL-1α, IL-1β, IL-3, IL-6, IL-7, IL-8, IL-10, IL-18, and IL-1RA.

The testing model used for this study was the MatTek EpiDerm. This skin model co nsists of normal human-derived epidermal keratinocytes that have been cultured to form a mu ltilayered, highly differentiated model of the human epidermis. Ultrastructural analysis has revealed the presence of keratohyalin granules, tonofilament bundles, desmosomes, and a multi-layered stratum corneum containing intercellular lamellar lipid layers arranged in patterns characteristic of in vivo epidermis. Markers of mature epidermis specific differentiation such as pro-filaggrin, the K1/K10 cytokeratin pair, involucrin, and type I epidermal transglutaminase have been localized in this model. The MatTek EpiDerm is also mitotically and metabolically active.

The MatTek EpiDerm tissues were used to assess the ability of various test materials to inhibit the release the inflammatory mediator IL-1α. Test materials were compared to an over the counter topical hydrocortisone preparation (positive control) as well as to untreated tissues (negative control 1) and untreated, non-inflamed tissues (negative control 2). This test was also used to assess the viability of the tissues after exposure to the test materials.

IL-1α, IL-6 and IL-8 are synthesized and stored in keratinocytes and have been identified as a mediators of skin irritation and inflammation. Release of these cytokines can be directly measured in tissue culture media via a colorimetric based enzyme linked immunosorbent assay (ELISA). Briefly, antibodies covalently linked to a solid support will bind IL-1a, IL-6 or IL-8 present in spent culture media samples. A second antibody that is covalently attached to an acetylcholinesterase enzyme will in turn detect the specific bound cytokines. Upon addition of an appropriate color substrate the acetylcholinesterase enzyme will generate a colored end product that can be measured spectrophotometrically.

MatTek EpiDerm Tissues were purchased from MatTek corporation and were stored at 4°C until used. Prior to use, the tissues to be used were removed from the agarose-shipping tray and placed into a 6-well plate containing 0.9 ml of hydrocortisone free assay medium (37±2°C). The tissues were allowed to incubate overnight at 37±2°C and 5±1% CO2. After this initial incubation, the assay medium was replaced with 0.9 ml of fresh hydrocortisone free medium (37±2°C). Three tissues were prepared for each test material.

An inflammatory response in the tissues was initiated via UV irradiation (UVB). A UV lamp was used to give a 300 mJ/cm² dose of UVB radiation to the tissues. Immediately after the application of the inflammatory stimuli 50 µl or mg of test material was applied directly onto the surface of the tissue. An over the counter hydrocortisone cream was used as a positive control. For a negative control tissues were exposed to the inflammatory stimuli but were not treated with any type of anti-inflammatory material. One additional set of tissues was left without exposure to the inflammatory stimuli to provide a baseline measurement for the cytokines. The tissues were incubated at37±2°C and 5±1% CO₂ for 24 hours after exposure to the inflammatory stimuli. After the 24-hour incubation the cell culture medium was collect and stored at -75° C until analyzed for cytokines.

The ELISA plates were prepared by diluting the appropriate capture antibody in PBS. Next, 100 µl of the diluted capture antibody was added to the wells of a 96-well ELISA plate and the plate was incubated overnight at room temperature. On the following day the plate was washed three times with 300 µl wash buffer (0.05% Tween 20 in PBS) and then blocked by adding 300 µl of blocking buffer (1% BSA in PBS) to each well. The plate was incubated with the blocking buffer for at least one hour. After the incubation the blocking buffer was removed and the plate was washed three times as described above.

A series of standards was prepared and 100 µl of each of these standards was dispensed into two wells (duplicates) in the appropriate 96-well plate. Subsequently, 100 µl of each sample was added to additional wells and the plate was incubated for two hours at room temperature. After the incubation the plate was washed three times as described above. Once the last wash was removed, 100 µl of a biotin conjugated detection antibody was added. After incubating the plate for two hours at room temperature the plate was washed again as described above. 100 µl of HRP-streptavidin was then added to each well and the plate was incubated for 20 minutes at room temperature. Once the last wash was removed, 100µl of substrate solution (hydrogen peroxide + tetramethylbenzidine as a chromagen) was added to each well. Once a sufficient level of color development had occurred, 50 µl of stop solution (2N sulfuric acid) was added to each well and the plate was read at 460 nm.

After the 24 hour incubation, the tissues were rinsed twice with at least 100 µl of phosphate buffered saline to remove the test material and then transferred to a 6-well plate containing 1.0 ml of assay medium supplemented with MTT (1 mg/ml) and allowed to incubate for 3 ± 0.25 hours at 37±2°C and 5±1% CO2. After the incubation, the tissues were rinsed at least twice with 100 µl of phosphate buffered saline, blotted dry, and then placed into a 24-well plate containing 2 ml of isopropanol per well. The 24- well plate was covered and allowed to incubate at room temperature for at least 2 hours on a rocking platform to extract the reduced MTT from the tissues. After the extraction, a 200 µl sample of the isopropanol/MTT mixture was transferred to a 96-well plate and the absorbance of the sample was read at 540 nm with a plate reader using 200 µl of isopropanol as the blank. The MTT assay is described in Example 6 herein. The cell viability results of the MTT assay were similar to the results obtained in Example 6

The results of the IL-1a assay are shown in Table 9 below. A 2% stock solution of the jellyfish collagen of SEQ ID NO: 91 is Sample 4 and Sample 3 is a 2% stock solution of the truncated jellyfish collagen of SEQ ID NO: 10. In Table 9 below, the indicated percentage is the percent dilution of the stock solution used for the test. For example, the 1% Sample 4 treatment is a 1% solution of the 2% stock truncated collagen solution. The untreated cells produced 18.2 pg/ml of Il-1a. Upon treatment with truncated collagen, all samples showed decreases in Il-1a production. The 1% Sample 4 treatment reduced IL-1A production to 13.4 pg/ml, which is significant with a p value of less than 0.05. The decrease in IL-1a production indicates that the truncated collagen has anti-inflammatory effects.

**Table 9 IL-1a Assay**

| Treatment | Il-1a pg/ml |
|---|---|
| Non-UVB Exposed | 4.9±2.1 |
| Untreated | 18.2±1.4 |
| 1% Hydrocortisone | 6.7±0.6 |
| 5% Sample 4 | 18.1±1.1 |
| 1% Sample 4 | 13.4±0.9* |
| 0.5% Sample 4 | 15.7±0.7 |
| 0.1% Sample 4 | 13.9±1.7* |
| 5% Sample 3 | 15.8±2.0 |

| | |
|---|---|
| Denotes values that are significantly different from Untreated (p<0.05) | |

### Example 12: Urban Dust Protection by Truncated Collagen

**A keratinocyte cell** culture model was used to assess the ability of truncated collagens to exert a protective effect by promoting cell survival after exposure to urban dust.

Human epidermal keratinocytes were pretreated with the test materials and then exposed to urban dust. At the end of the treatment period changes in cell viability were then determined via an MTT assay.

Keratinocytes were seeded into the individual wells of a 96 well plate in 100 µl of medium and incubated overnight at 37±2°C and 5±1% CO2. On the following day the media was removed via aspiration to eliminate any non-adherent cells and replaced with 100 µl of fresh medium. The cells were grown until confluent, with a media change every 48 to 72 hours.

### Pretreatment with Test Material followed by Urban Dust Treatment

Test materials were prepared at 2x their final desired concentrations in cell culture media. Urban dust (NIST 1649B from Sigma Chemicals) was also prepared at 2x solutions. For the pretreatment, 50 µl of 2x test material was combined with 50 µl of culture media and the cells were incubated for 24 hours. At the end of the pretreatment period the test material containing culture media was removed and replaced with 50µl of 2x urban dust and 50 µl of media. Another set of cells was treated with media alone (non-dust exposed) and used as a reference control to represent 100% cell viability. The cells were then incubated for 24 hours and then subjected to an MTT assay to determine changes in cell viability.

At the end of the treatment period, the cell culture medium was removed and the cells were washed with PBS. After the wash, 100 µl of cell culture media supplemented with 0.5 mg/ml MTT was added to each well and the cells were incubated for 30 minutes at 37+2oC and 5+1% CO2. After the incubation, the media/MTT solution was removed and the cells were washed again once with PBS and then 100 µl of isopropyl alcohol was added to the wells to extract the purple formazin crystals. The 96-well plate was then read at 540 nm using isopropyl alcohol as a blank.

The mean MTT absorbance value for the non-dust exposed cells was calculated and used to represent 100% value for cell number. The individual MTT values from the cells undergoing the various treatments was then divided by the mean value for the non-dust exposed cells and expressed as a percent to determine the change in cell number caused by each treatment.

The MTT results for the pretreatment with the test material then dust treatments are presented in Table 10. Table 10 shows that as the cells were treated with increasing amounts of collagen, cell viability increased upon pretreatment with truncated collagen and subsequent exposure to urban dust. These results show that truncated collagen protects against the decline in cell viability associated with urban dust exposure.

**Table 10 MTT Assay, Truncated Collagen Pretreatment**

| Treatment | Viability (% Non-Dust Exposed) | Viability (% Non-Dust Exposed) |
|---|---|---|
| | 4 mg/ml Urban Dust | 2 mg/ml Urban Dust |
| Non-Dust Exposed | 100±4.4* | 100±1.8 |
| Untreated | 59±3.8 | 70±0.9 |
| 0.1% Collagen | 61±4.7 | 72±3.5 |
| 0.5% Collagen | 59±2.2 | 73±2.2 |
| 1% Collagen | 58±0.5 | 74±2.6 |
| 5% Collagen | 66±1.0 | 83±6.5* |

| | | |
|---|---|---|
| * Denotes values that are significantly different from untreated group (p<0.05) | | |

### Example 13: Truncated Chondrosia reniformis (Kidney sponge) Collagen Truncated Chondrosia reniformis (Kidney sponge) Fibrillar Collagen 1 with DsbA secretion and FLAG tag

The amino acid sequence of truncated *Chondrosia reniformis* fibrillar collagen 1 with DsbA secretion and FLAG tag is disclosed in SEQ ID NO: 102. The DsbA secretion tag is encoded by nucleotides 1-57 of SEQ ID NO: 103 and the amino acid sequences are amino acids 1-19 of SEQ ID NO: 102. The fibrillary collagen nucleotide sequences are nucleotides 58-792 of SEQ ID NO: 103 and the amino acid sequences are amino acids 20-264 of SEQ ID NO: 102. The FLAG nucleotide sequences are nucleotides 793-819 of SEQ ID NO: 103 and the amino acid sequences are amino acids 265-273 of SEQ ID NO: 102.

MKKIWLALAGLVLAFSASAPVGRRGPKGSRGDPGDGGAAGPKGPEGVDGLIGEPGQ
PGPIGAEGSSGLEGFLGDKGSKGARGGPGNRGRPGQDGVPGQDGRAGEKGEGGETG
DRGQQGLRGKVGDPGLVGDLGAQGPQGSQGLVGPPGIPGEPGSGGEPGDQGPRGPE
GPQGSPGVRGGRGERGTPGAVGPKGPPGKNGADGPRGLPGASGPPGSPGNQGPEGS
RGADGNNGFPGDDGENGLVGIPGEPGPKGARGTRGELGKTGDYKDDDDK (SEQ ID
NO: 102)

The nucleic acid sequence of truncated *Chondrosia reniformis* fibrillar collagen 1 with DsbA secretion and FLAG tag is disclosed in SEQ ID NO: 103.

ATGAAAAAGATTTGGCTGGCGCTGGCTGGTTTAGTTTTAGCGTTTAGCGCATCGG
CGCCGGTTGGTCGTCGTGGTCCGAAAGGTAGCCGTGGTGATCCTGGTGATGGTGG
TGCAGCAGGTCCTAAAGGTCCGGAAGGTGTTGATGGTCTGATTGGTGAACCGGG
TCAGCCTGGTCCGATTGGCGCAGAAGGTAGCAGCGGTCTGGAAGGTTTCTGGGT
GATAAAGGTAGCAAAGGTGCACGTGGTGGTCCGGGTAATCGCGGTCGTCCTGGT
CAGGATGGTGTTCCGGGTCAAGATGGTCGTGCCGGTGAAAAAGGTGAAGGTGGT
GAAACCGGTGATCGCGGTCAGCAGGGTCTGCGTGGTAAAGTTGGTGATCCAGGT
CTGGTGGGTGATCTGGGTGCACAGGGTCCGCAGGGTAGCCAAGGTCTGGTTGGT
CCGCCTGGTATTCCGGGTGAACCTGGTAGCGGTGGCGAACCGGGTGATCAGGGT
CCTCGCGGTCC

AGAAGGTCCTCAGGGTTCACCGGGTGTTCGCGGTGGTCGTGGTGAACGTG
GTACACCGGGTGCAGTTGGACCGAAAGGTCCGCCAGGTAAAAATGGTGCA
GATGGTCCGCGTGGTCTGCCTGGTGCAAGCGGTCCTCCGGGTAGTCCTGGT
AACCAGGGTCCTGAAGGTTCTCGTGGTGCCGATGGTAATAATGGTTTTCCA
GGTGATGATGGTGAAAATGGCCTGGTTGGTATCCCTGGCGAACCAGGTCC
AAAAGGCGCACGCGGTACACGCGGTGAACTGGGTAAAACCGGTGACTAC
AAAGACGACGACGACAAAtaa (SEQ ID NO: 103)

The polynucleotide of SEQ ID NO: 103 was subcloned into vector pET28a, expressed host *E. coli* cells and the truncated *Chondrosia reniformis* fibrillar collagen 1 was purified as described herein. The purified fibrillary collagen produced a clear band on SDS-PAGE and an anti-FLAG western was observed at around 40 kilodaltons. There were no existing bands that appear at that location on the gel in the absence of expression of this protein.

### Truncated Chondrosia reniformis (Kidney sponge) Fibrillar Collagen 2 with DsbA secretion and FLAG tag

The amino acid sequence of truncated *Chondrosia reniform is* fibrillar collagen 2 with DsbA secretion and FLAG tag is disclosed in SEQ ID NO: 104. The DsbA secretion tag is encoded by nucleotides 1-57 of SEQ ID NO: 105 and the amino acid sequences are amino acids 1-19 of SEQ ID NO: 104. The fibrillary collagen nucleotide sequences are nucleotides 58-1323 of SEQ ID NO: 105 and the amino acid sequences are amino acids 20-441 of SEQ ID NO: 104. The FLAG nucleotide sequences are nucleotides 1324-1350 of SEQ ID NO: 105 and the amino acid sequences are amino acids 442-450 of SEQ ID NO: 105.

MKKIWLALAGLVLAFSASAGRGGPAGLQGAAGNPGDPGDRGQAGEIGLPGT
EGQRGQGGSRGDDGIGGQSGTDGDPGNDGVAGIRGARGEPGATGPEGAAGQ
KGDRGRFGEQGRPGNDGPPGRRGRVGNLGETGAEGDEGTRGYTGDRGPEGA
IGISGVTGNPGPQGIKGPPGDTGHPGRQGPSGPQGPPGIPGTDGLTIHNLIKPPS
QFFDATSSSDPLTDAVVESILKSFQYAELEIDLTKKPDGTMKYPAISCDDLHK
DYPQLPSGNYTLDPNGGCKNDAFETYCEFNNSVKMCLTPKIPTLLPMGTYKY
YVNSEGYYSPNDFGLNLRFFEYYGSVTQLKFLQTKATRVTQTIRVLCKNYDP
LHKQPVFIGMNDETVMDEPRMEENQCQYFNGLSAHVELELSSNDPSYLPIYE
MRLYLGRKTNEELGIELGDLCFEYGDYKDDDDK (SEQ ID NO: 104)

The nucleic acid sequence of truncated *Chondrosia reniformis* fibrillar collagen 2 with DsbA secretion and FLAG tag is disclosed in SEQ ID NO: 105.

ATGAAAAAGATTTGGCTGGCGCTGGCTGGTTTAGTTTTAGCGTTTAGCGCA
TCGGCGGGTCGTGGCGGTCCGGCAGGTCTGCAGGGTGCTGCAGGTAATCC
TGGCGACCCTGGCGATCGTGGTCAGGCAGGCGAAATTGGTCTGCCAGGCA
CCGAAGGTCAGCGTGGTCAAGGTGGTTCACGTGGTGATGACGGTATTGGT
GGTCAGAGCGGCACCGATGGCGATCCGGGTAACGATGGTGTTGCAGGTAT
TCGTGGTGCACGCGGAGAACCTGGTGCCACCGGACCTGAAGGTGCAGCCG
GTCAGAAAGGTGATCGTGGCCGTTTTGGCGAACAGGGTCGTCCGGGAAAT
GATGGTCCACCGGGTCGCCGTGGCCGTGTGGGCAATCTGGGTGAAACAGG
TGCCGAAGGTGATGAAGGCACCCGTGGTTATACAGGTGACCGTGGACCGG
AAGGCGCAATTGGTATTAGCGGTGTGACCGGTAATCCGGGTCCACAGGGC
ATTAAAGGCCCTCCGGGTGATACGGGTCATCCGGGTCGTCAGGGACCGAG
CGGTCCGCAAGGACCACCGGGTATTCCAGGTACAGATGGCCTGACCATTC
ATAATCTGATTAAACCGCCTAGCCAGTTTTTGATGCAACCAGCAGCAGC
GATCCGCTGACCGATGCAGTTGTTGAAAGCATTCTGAAATCTTTTCAGTAT
GCCGAGCTGGAAATTGACCTGACCAAAAAACCGGATGGCACCATGAAAT
ATCCGGCAATTAGCTGTGATGATCTGCACAAAGATTATCCGCAGCTGCCG
AGCGGTAATTATACCCTGGATCCGAATGGTGGTTGTAAAAATGATGCCTTT
GAAACCTATTGCGAGTTCAACAATAGCGTGAAAATGTGTCTGACCCCGAA
AATTCCGACACTGCTGCCGATGGGCACCTATAAATACTATGTTAATAGCG
AGGGTTACTACAGCCCGAATGATTTTGGTCTGAATCTGCGCTTTTTGAGT
ATTATGGTAGCGTTACCCAGCTGAAATTTCTGCAGACCAAAGCAACCCGT
GTTACCCAGACCATTCGTGTTCTGTGTAAAAACTATGATCCGCTGCATAAA
CAGCCGGTTTTTATTGGTATGAATGACGAAACCGTTATGGATGAACCGCG
TATGGAAGAAAATCAGTGCCAGTATTTTAACGGTCTGAGCGCACATGTTG
AACTGGAACTGAGCAGCAATGATCCGAGCTATCTGCCGATTTATGAAATG
CGTCTGTATCTGGGTCGTAAAACCAATGAAGAACTGGGCATTGAACTGGG
CGATCTGTGTTTTGAATATGGTGACTACAAAGACGACGACGACAAAtaa
(SEQ ID NO:105)

The polynucleotide of SEQ ID NO: 105 was subcloned into vector pET28a, expressed host *E. coli* cells and the truncated *Chondrosia reniformis* fibrillar collagen 2 was purified as described herein. The purified fibrillary collagen produced a clear band on SDS-PAGE and an anti-FLAG western was observed at around 55 kilodaltons. There were no existing bands that appear at that location on the gel in the absence of expression of this protein.

### Truncated Chondrosia reniformis (Kidney sponge) Non-Fibrillar Collagen 1 with DsbA secretion and FLAG tag

The amino acid sequence of truncated *Chondrosia reniformis* non-fibrillar collagen 1 with DsbA secretion and FLAG tag is disclosed in SEQ ID NO: 106. The DsbA secretion tag is encoded by nucleotides 1-57 of SEQ ID NO: 107 and the amino acid sequences are amino acids 1-19 of SEQ ID NO: 106. The non-fibrillar collagen nucleotide sequences are nucleotides 58-831 of SEQ ID NO: 107 and the amino acid sequences are amino acids 20-277 of SEQ ID NO: 106. The FLAG nucleotide sequences are nucleotides 832-858 of SEQ ID NO: 107 and the amino acid sequences are amino acids 278-286 of SEQ ID NO: 106.

MKKIWLALAGLVLAFSASAEKTSSKVALMTVLVVITGALIIEGTSITRGSTHV
NRGLRKRQTSEDNCEAVKVGLPGRDGREGPPGPPGPAGRDGRDAVCSNQTT
GLGAKGDRGPPGTPGFPGEVGRPGPPGADGIPGPQGERGAVGPGGKPGPRGE
VGTPGADGADGATGATGVQGPDGAKGEKGASGTAGLKGEKGDTCIPDSNST
LGMPGTPGAGGSKGQKGESGIVGPKGERGEIGTPGHPGFRGADGEPGHKGVP
GRAGAQGDRGDPGDDGLTGDYKDDDDK (SEQ ID NO: 106)

The nucleic acid sequence of truncated *Chondrosia reniformis* non-fibrillar collagen 1 with DsbA secretion and FLAG tag is disclosed in SEQ ID NO: 107.

ATGAAAAAGATTTGGCTGGCGCTGGCTGGTTTAGTTTTAGCGTTTAGCGCA
TCGGCGGAAAAAACCAGCAGCAAAGTTGCACTGATGACCGTTCTGGTTGT
TATTACCGGTGCACTGATTATTGAAGGCACCAGCATTACCCGTGGTAGCA
CCCATGTTAATCGTGGTCTGCGTAAACGTCAGACCAGCGAAGATAATTGT
GAAGCAGTTAAAGTTGGTCTGCCAGGTCGTGATGGTCGTGAAGGTCCTCC
GGGTCCGCCTGGTCCGGCTGGCAGAGATGGCCGTGATGCAGTTTGTAGCA
ATCAGACCACCGGTCTGGGTGCAAAAGGTGATCGTGGTCCGCCAGGTACA
CCGGGTTTTCCGGGTGAAGTTGGCCGTCCGGGTCCACCGGGTGCAGATGG
TATTCCGGGTCCTCAGGGTGAACGTGGTGCAGTTGGTCCTGGTGGTAAAC
CTGGTCCGCGTGGTGAAGTGGGCACCCCTGGTGCCGATGGCGCAGATGGT
GCAACCGGTGCGACCGGTGTTCAGGGTCCTGATGGTGCCAAAGGCGAAAA
AGGTGCAAGCGGCACCGCAGGTCTGAAAGGTGAGAAAGGCGATACCTGT
ATTCCGGATAGCAATAGCACCCTGGGTATGCCTGGTACACCAGGTGCCGG
TGGTAGCAAAGGCCAGAAAGGTGAAAGTGGTATTGTTGGTCCGAAAGGC
GAACGCGGTGAAATTGGCACACCGGGTCATCCTGGTTTTCGTGGTGCGGA
TGGTGAACCAGGTCATAAAGGTGTTCCGGGTCGTGCCGGTGCGCAGGGTG

ATCGCGGTGATCCGGGTGATGATGGTCTGACCGGTGACTACAAAGACGAC
GACGACAAAtaa (SEQ ID NO: 107)

The polynucleotide of SEQ ID NO: 107 was subcloned into vector pET28a, expressed host *E. coli* cells and the truncated *Chondrosia reniformis* non-fibrillar collagen 1 was purified as described herein. The purified non-fibrillar collagen produced a clear band on SDS-PAGE and an anti-FLAG western was observed at around 30 kilodaltons. There were no existing bands that appear at that location on the gel in the absence of expression of this protein.

### Truncated Chondrosia reniformis (Kidney sponge) Non Fibrillar Collagen 2 with DsbA secretion and FLAG tag

The amino acid sequence of truncated *Chondrosia reniform is* non-fibrillar collagen 2 with DsbA secretion and FLAG tag is disclosed in SEQ ID NO: 108. The DsbA secretion tag is encoded by nucleotides 1-57 of SEQ ID NO: 109 and the amino acid sequences are amino acids 1-19 of SEQ ID NO: 108. The non-fibrillar collagen nucleotide sequences are nucleotides 58-1509 of SEQ ID NO: 109 and the amino acid sequences are amino acids 20-503 of SEQ ID NO: 108. The FLAG nucleotide sequences are nucleotides 1510-1536 of SEQ ID NO: 109 and the amino acid sequences are amino acids 504-512 of SEQ ID NO: 108.

MKKIWLALAGLVLAFSASAGFPGAPGADGAPGQKGELGAVGPQGTPGLSGP
SGPTGPPGPKGVRGAPGSSGAKGDAGNPGDDGPVGPQGVPGVDGSPGQKGE
TGRVGPRGHDGINGTPGEDGATGFPGPDGAKGEKGTSGTAGLKGEKGDTCIP
DSNSTLGMPGTPGAGWSKGQKGESGIVGPKGEKGEIGTPGPPGFRGADGEPG
QRGEPGRAGAQGERGAPGNNGRDGFPGDPGADGAPGQKGELGAIGHPGFSG
PSGPSGPTGPPGPKGVRGAQGRPGDRGSPGDVGPIGAPGPPGADGVPGLTGV
QGRDGPKGESASSGAVYVRWGRTTCPSGADVVYSGRAAGAKYDHSGGTSD
HHCLPNNPQYLSEDDTNALGAQLYGVEYEIRDRSSPYNSLDQSDMPCVVCNA
NGRSQLLMVPARYTCPTGWSREYYGYMMSEGKAKNREGRKTTICMDFSAE
AVPGSGANTNPSPGIMMRANCNGLACPPYQSNTPLTCAVCTKGDYKDDDDK
(SEQ ID NO: 108)

The nucleic acid sequence of truncated *Chondrosia reniformis* non-fibrillar collagen 2 with DsbA secretion and FLAG tag is disclosed in SEQ ID NO: 109.

ATGAAAAAGATTTGGCTGGCGCTGGCTGGTTTAGTTTTAGCGTTTAGCGCA
TCGGCGGGTTTTCCTGGCGCTCCGGGTGCCGACGGTGCTCCGGGTCAAAA
AGGTGAACTGGGTGCCGTGGGTCCGCAGGGCACTCCGGGTCTGAGTGGTC

CTAGTGGTCCGACCGGTCCACCAGGTCCAAAAGGCGTGCGTGGTGCACCG
GGTAGCAGCGGAGCCAAAGGTGATGCAGGTAACCCTGGTGATGACGGTCC
GGTTGGTCCACAGGGCGTTCCAGGTGTTGATGGTAGCCCTGGCCAAAGG
GTGAAACCGGTCGTGTGGGTCCTCGTGGTCATGATGGTATTAATGGCACC
CCAGGTGAAGATGGTGCGACAGGCTTTCCAGGTCCGGATGGCGCAAAGGG
TGAGAAGGGCACCAGCGGTACAGCTGGCCTGAAGGGCGAAAGGGCGAT
ACATGCATCCCGGATTCAAATTCAACACTGGGCATGCCAGGTACGCCTGG
CGCAGGTTGGAGTAAAGGACAAAAAGGCGAATCAGGCATTGTGGACCT
AAAGGCGAGAAGGGTGAGATTGGTACTCCGGGACCGCCAGGCTTTCGCGG
TGCAGACGGCGAACCGGGTCAGCGTGGCGAACCTGGTCGTGCAGGCGCA
CAAGGTGAACGCGGAGCCCCTGGTAATAATGGACGTGATGGCTTTCCTGG
TGATCCAGGTGCAGATGGCGCACCTGGCCAGAAAGGCGAACTGGGAGCA
ATTGGTCATCCGGGATTTAGCGGTCCGTCAGGTCCGAGCGGACCGACAGG
TCCTCCTGGACCGAAAGGTGTACGTGGCGCACAGGGTCGTCCTGGCGATC
GTGGCAGTCCAGGTGATGTGGGTCCGATTGGTGCACCTGGTCCTCCAGGT
GCGGACGGCGTGCCTGGTTTAACAGGTGTGCAGGGTCGCGACGGTCCTAA
AGGTGAATCAGCAAGCAGCGGTGCAGTTTATGTTCGTTGGGGTCGTACCA
CCTGTCCTAGCGGAGCAGATGTTGTTTATAGCGGTCGCGCAGCCGGTGCA
AAATATGATCATTCAGGTGGCACCTCAGATCATCATTGTCTGCCGAATAAT
CCGCAGTATCTGAGCGAAGATGATACCAATGCACTGGGTGCACAGCTGTA
TGGTGTGGAATATGAAATTCGTGATCGTAGCAGCCCGTATAATAGCCTGG
ATCAGAGCGATATGCCGTGTGTTGTTTGTAATGCAAATGGTCGTAGCCAG
CTGCTGATGGTTCCGGCACGTTATACATGCCCGACCGGTTGGAGCCGTGA
ATATTATGGTTATATGATGAGCGAAGGCAAAGCCAAAAATCGCGAAGGTC
GTAAAACCACCATTTGTATGGATTTTAGCGCAGAAGCAGTTCCTGGTAGC
GGTGCAAATACCAATCCGAGTCCGGGTATTATGATGCGTGCAAATTGTAA
TGGTCTGGCATGTCCGCCTTATCAGAGCAATACACCGCTGACCTGTGCCGT
TTGTACCAAAGGTGACTACAAAGACGACGACGACAAAtaa (SEQ ID NO:
109)

The polynucleotide of SEQ ID NO: 109 was subcloned into vector pET28a, expressed host *E. coli* cells and the truncated *Chondrosia reniformis* non-fibrillar collagen 2 was purified as described herein. The purified fibrillary collagen produced a clear band on SDS-PAGE and an anti-FLAG western was observed at around 60 kilodaltons. There were no existing bands that appear at that location on the gel in the absence of expression of this protein.

### Example 14: Truncated Rhincodon typus (whale shark) Collagen

### Truncated Rhincodon typus (whale shark) Collagen Type 1 alpha 1 truncation 1 with DsbA secretion and FLAG tag

The amino acid sequence of truncated *Rhincodon typus* collagen type1 truncation 1 with DsbA secretion and FLAG tag is disclosed in SEQ ID NO: 110. The DsbA secretion tag is encoded by nucleotides 1-57 of SEQ ID NO: 111 and the amino acid sequences are amino acids 1-19 of SEQ ID NO: 110. The collagen nucleotide sequences are nucleotides 58-630 of SEQ ID NO: 111 and the amino acid sequences are amino acids 20-210 of SEQ ID NO: 110. The FLAG nucleotide sequences are nucleotides 631-657 of SEQ ID NO: 111 and the amino acid sequences are amino acids 211-219 of SEQ ID NO: 110.

MKKIWLALAGLVLAFSASAGPAGAKGPSGDIGRPGESGSPGARGHSGQPGRTGIAGN
QGLPGTAGEEGRTGPPGPAGLRGQAGMMGFPGPKGAAGLPGKPGDRGNVGLAGPR
GAPGKDGEVGAQGPPGVAGPTGPRGETGLAGSVGFQGMPGPSGAAGEPGKPGNQG
LRGDAGSPGMIGPRGERGLPGERGASGAQGLLGPRGTSGAPGLGDYKDDDDK (SEQ
ID NO: 110)

The nucleic acid sequence of truncated *Rhincodon typus* collagen type1 truncation 1 with DsbA secretion and FLAG tag is disclosed in SEQ ID NO: 111.

ATGAAAAAGATTTGGCTGGCGCTGGCTGGTTTAGTTTTAGCGTTTAGCGCATCGG
CGGGTCCGGCAGGCGCAAAAGGTCCGAGCGGTGATATTGGTCGTCCGGGTGAAA
GCGGTAGTCCGGGTGCACGTGGTCATAGCGGTCAGCCTGGTCGTACCGGTATTGC
AGGTAATCAGGGTCTGCCTGGTACAGCCGGTGAAGAAGGTCGCACCGGTCCGCC
AGGTCCTGCAGGTCTGCGTGGTCAGGCAGGTATGATGGGTTTTCCGGGTCCGAAA
GGTGCAGCGGGTCTGCCAGGCAAACCGGGTGATCGTGGTAATGTTGGTCTGGCT
GGTCCGCGTGGTGCACCGGGTAAAGATGGTGAAGTTGGTGCACAGGGTCCTCCG
GGTGTTGCAGGTCCGACCGGTCCTCGTGGTGAAACCGGTCTGGCAGGTAGCGTTG
GTTTTCAGGGTATGCCAGGTCCGTCAGGTGCAGCAGGCGAACCTGGTAAACCGG
GTAACCAGGGCCTGCGTGGTGATGCCGGTTCACCGGGTATGATTGGTCCACGCGG
TGAACGTGGCCTGCCTGGCGAACGTGGTGCAAGCGGTGCACAAGGTCTGCTGGG
TCCACGTGGCACCTCAGGCGCACCAGGTCTGGGTGACTACAAAGACGACGACGA
CAAAtaa (SEQ ID NO: 111)

The polynucleotide of SEQ ID NO: 111 was subcloned into vector pET28a, expressed host E. *coli* cells and the truncated *Rhincodon typus* collagen type1 truncation 1 was purified as described herein. The purified collagen produced a clear band on SDS-PAGE and an anti-FLAG western was observed at around 25 kilodaltons. There were no existing bands that appear at that location on the gel in the absence of expression of this protein.

### Truncated Rhincodon typus (whale shark) Collagen Type 6 alpha 1 truncation 2 with DsbA secretion and FLAG tag

The amino acid sequence of truncated *Rhincodon typus* collagen type 6 truncation 2 with DsbA secretion and FLAG tag is disclosed in SEQ ID NO: 112. The DsbA secretion tag is encoded by nucleotides 1-57 of SEQ ID NO: 113 and the amino acid sequences are amino acids 1-19 of SEQ ID NO: 112. The collagen nucleotide sequences are nucleotides 58-684 of SEQ ID NO: 113 and the amino acid sequences are amino acids 20-228 of SEQ ID NO: 112. The FLAG nucleotide sequences are nucleotides 685-711 of SEQ ID NO: 113 and the amino acid sequences are amino acids 229-237 of SEQ ID NO: 112.

MKKIWLALAGLVLAFSASAQGIPGSAGKEGGKGDPGPLGSPGKPGPDGLRGF
AGARGLPGAAGPPGLKGAEGPMGAPGLTGSTGERGPNGPAGAIGLPGRPGGP
GPPGPVGEKGDPGDKGLPGPAGDDGVQGAMGLPGPIGSQGPPGDYGDKGEL
GKPGQKGSKGDKGESGPPGPIGIQGPIGHPGPIGSDGSPGLRGYLGMRGQKGD
DGIRGLPGSAGPVGLQGLPGGDYKDDDDK (SEQ ID NO: 112)

The nucleic acid sequence of truncated *Rhincodon typus* collagen type 6 truncation 2 with DsbA secretion and FLAG tag is disclosed in SEQ ID NO: 113.

ATGAAAAAGATTTGGCTGGCGCTGGCTGGTTTAGTTTAGCGTTAGCGCA
TCGGCGCAGGGTATTCCGGGTAGCGCAGGTAAAGAAGGTGGTAAAGGCG
ATCCGGGTCCGCTGGGTTCACCGGGTAAACCGGGTCCTGATGGTCTGCGT
GGTTTTGCCGGTGCACGTGGTCTGCCTGGTGCAGCAGGTCCGCCTGGTCTG
AAAGGTGCCGAAGGTCCGATGGGTGCTCCGGGTCTGACCGGTAGCACCGG
TGAACGCGGTCCGAATGGTCCGGCAGGCGCAATTGGTCTGCCAGGTCGTC
CTGGTGGTCCGGGTCCTCCTGGTCCGGTTGGTGAAAAAGGTGATCCTGGT
GATAAAGGCCTGCCTGGTCCTGCCGGTGATGATGGTGTTCAGGGTGCCAT
GGGCTTACCGGGTCCGATTGGTAGCCAGGGTCCTCCGGGTGATTATGGCG
ATAAAGGTGAACTGGGTAAACCTGGCCAGAAAGGTAGCAAAGGTGACAA
AGGCGAAAGCGGTCCGCCAGGTCCGATCGGCATTCAGGGTCCTATTGGTC

ATCCAGGTCCAATTGGTTCAGATGGCTCACCGGGACTGCGTGGCTATCTG
GGTATGCGTGGACAGAAAGGTGATGACGGTATTCGTGGCCTGCCAGGTAG
TGCAGGTCCGGTGGGTCTGCAGGGACTGCCTGGTGGTGACTACAAAGACG
ACGACGACAAAtaa (SEQ ID NO: 113)

The polynucleotide of SEQ ID NO: 113 was subcloned into vector pET28a, expressed host *E. coli* cells and the truncated *Rhincodon typus* collagen type 6 truncation 2 was purified as described herein. The purified collagen produced a clear band on SDS-PAGE and an anti-FLAG western was observed at around 35 kilodaltons. There were no existing bands that appear at that location on the gel in the absence of expression of this protein.

### Truncated Rhincodon typus (whale shark) Collagen Type 6 alpha 1 truncation 3 with DsbA secretion and FLAG tag

The amino acid sequence of truncated *Rhincodon typus* collagen type 6 alpha 1 truncation 3 with DsbA secretion and FLAG tag is disclosed in SEQ ID NO: 114. The DsbA secretion tag is encoded by nucleotides 1-57 of SEQ ID NO: 115 and the amino acid sequences are amino acids 1-19 of SEQ ID NO: 114. The collagen nucleotide sequences are nucleotides 58-735 of SEQ ID NO: 115 and the amino acid sequences are amino acids 20-245 of SEQ ID NO: 114. The FLAG nucleotide sequences are nucleotides 736-762 of SEQ ID NO: 115 and the amino acid sequences are amino acids 246-254 of SEQ ID NO: 114.

MKKIWLALAGLVLAFSASAKGETGEAGDPGTPGEPGIAGPKGDVGDKGDAG
PPGAAGPAGVKGPPGEDGAKGDVGPAGFPGDPGPTGEPGVPGMDGGVGEKG
SLGDPGLTGPRGASGEPGPPGSPGKRGPPGPAGPEGREGLKGSKGSPGQEGPV
GRTGPIGPQGSPGNVGPKGLRGIPGPTGEQGLLGPPGQAGPPGPMGPPGMPGL
RGAQGLKGDKGHVGLIGLIGPPGEMGEKGDQGLPGIQGDYKDDDDK (SEQ
ID NO: 114)

The nucleic acid sequence of truncated *Rhincodon typus* collagen type 6 alpha 1 truncation 3 with DsbA secretion and FLAG tag is disclosed in SEQ ID NO: 115.

ATGAAAAAGATTTGGCTGGCGCTGGCTGGTTTAGTTTTAGCGTTTAGCGCA
TCGGCGAAAGGTGAAACCGGTGAAGCGGGTGATCCGGGTACACCGGGTG
AACCTGGTATTGCAGGTCCGAAAGGTGATGTTGGTGATAAAGGTGACGCA
GGTCCGCCTGGTGCAGCAGGTCCGGCAGGCGTTAAAGGTCCTCCGGGTGA
AGATGGTGCAAAAGGCGACGTTGGTCCTGCAGGTTTTCCTGGCGATCCGG

GTCCGACTGGTGAACCGGGTGTGCCAGGTATGGATGGTGGTGTGGGTGAA
AAAGGTAGCCTGGGTGATCCTGGTCTGACCGGTCCGCGTGGCGCAAGTGG
TGAACCAGGTCCACCGGGTAGTCCGGGTAAACGTGGTCCTCCTGGACCGG
CTGGTCCGGAAGGTCGTGAAGGTCTGAAAGGTAGCAAAGGTTCACCGGGT
CAAGAAGGTCCGGTTGGTCGTACCGGTCCGATTGGTCCGCAGGGCTCACC
GGGTAATGTTGGTCCTAAAGGTCTGCGTGGTATTCCGGGTCCTACAGGCG
AACAGGGTCTGCTGGGTCCGCCAGGCCAAGCAGGTCCTCCAGGTCCTATG
GGTCCACCTGGTATGCCTGGCCTGCGTGGTGCCCAGGGCCTGAAAGGCGA
TAAAGGCCATGTTGGTCTGATTGGCCTGATTGGTCCACCAGGTGAAATGG
GAGAAAAAGGCGATCAGGGCCTGCCTGGTATTCAGGGTGACTACAAAGA
CGACGACGACAAAtaa (SEQ ID NO: 115)

The polynucleotide of SEQ ID NO: 115 was subcloned into vector pET28a, expressed host *E. coli* cells and the truncated *Rhincodon typus* collagen type1 truncation 1 was purified as described herein. The purified collagen produced a clear band on SDS-PAGE and an anti-FLAG western was observed at around 25 kilodaltons. There were no existing bands that appear at that location on the gel in the absence of expression of this protein.

## Claims

1. A recombinant collagen polypeptide that comprises or consists of an amino acid sequence according to SEQ ID NO: 91.

2. The recombinant collagen polypeptide of claim 1, further comprising one or more selected from the group consisting of: a secretion tag, a histidine tag, a fluorescent protein, a protease cleavage site, and a beta-lactamase protein.

3. The recombinant collagen polypeptide of claim 2, wherein the secretion tag is DsbA.

4. A composition comprising between 0.005% w/w and 30% w/w of a recombinant collagen polypeptide according to any one of the preceding claims.

5. The composition of claim 4, wherein the composition is formulated for topical application.

6. The composition of claim 5, wherein the composition comprises a topical carrier, a preservative, or both.

7. The composition of claim 6, wherein the topical carrier is selected from the group consisting of: a liposome, a biodegradable microcapsule, a lotion, a spray, an aerosol, a dusting powder, a biodegradable polymer, a mineral oil, a triglyceride oil, a silicone oil, glycerin, glycerin monostearate, an alcohol, an emulsifying agents, a liquid petroleum, a white petrolatum, propylene glycol, polyoxyethylene, polyoxypropylene, wax, sorbitan monostearate, polysorbate, cetyl ester wax, cetearyl alcohol, 2-octyldodecanol, benzyl alcohol, cyclomethicone, cyclopentasiloxane, water, and any combination thereof.

8. The composition of claim 6 or 7, wherein the preservative is selected from the group consisting of: tocopherol, diiodomethyl-p-tolylsulfone, 2-bromo-2-nitropropane-1,3-diol, cis isomer 1-(3-chloroallyl)-3,5,7-triaza-1-azoniaadamantane chloride, glutaraldehyde, 4,4-dimethyl oxazolidine, 7-ethylbicyclooxazolidine, methyl paraben, sorbic acid, Germaben II, rosemary extract, EDTA, and any combination thereof.

9. A recombinant host cell comprising a polynucleotide encoding a recombinant collagen polypeptide according to any one of claims 1-3.

10. The recombinant host cell of claim 9, wherein the recombinant cell is a bacterial cell.

11. The recombinant host cell of claim 10, wherein the bacterial cell is of the species *Escherichia coli.*

12. The recombinant host cell of any one of claims 9-11, wherein the polynucleotide comprises or consists of a nucleotide sequence according to SEQ ID NO: 90.

13. The recombinant host cell of any one of claims 9-11, wherein the polynucleotide is codon optimized for expression in the recombinant host cell.

## Patentansprüche

1. Rekombinantes Kollagenpolypeptid, das eine Aminosäuresequenz gemäß SEQ ID NO: 91 umfasst oder daraus besteht.

2. Rekombinantes Kollagenpolypeptid nach Anspruch 1, das weiters eines oder mehrere umfasst, die aus der aus Folgenden bestehenden Gruppe ausgewählt sind: einer Sekretionsmarkierung, einer Histidinmarkierung, einem fluoreszierenden Protein, einer Proteasespaltstelle und einem beta-Lactamaseprotein.

3. Rekombinantes Kollagenpolypeptid nach Anspruch 2, wobei die Sekretionsmarkierung DsbA ist.

4. Zusammensetzung, umfassend zwischen 0,005 Gew.-% und 30 Gew.-% eines rekombinanten Kollagenpolypeptids nach einem der vorangegangenen Ansprüche.

5. Zusammensetzung nach Anspruch 4, wobei die Zusammensetzung zur topischen Anwendung formuliert ist.

6. Zusammensetzung nach Anspruch 5, wobei die Zusammensetzung einen topischen Träger, ein Konservierungsmittel oder beides umfasst.

7. Zusammensetzung nach Anspruch 6, wobei der topische Träger aus der aus Folgenden bestehenden Gruppe ausgewählt ist: einem Liposom, einer biologisch abbaubaren Mikrokapsel, einer Lotion, einem Spray, einem Aerosol, einem Talkpuder, einem biologisch abbaubaren Polymer, einem Mineralöl, einem Triglyzeridöl, einem Silikonöl, Glycerin, Glycerinmonostearat, einem Alkohol, Emulgatoren, einem flüssigen Rohöl, einer Vaseline, Propylenglykol, Polyoxyethylen, Polyoxypropylen, Wachs, Sorbitanmonostearat, Polysorbat, Cetylesterwachs, Cetylstearylalkohol, 2-Octyldodecanol, Benzylalkohol, Cyclomethicon, Cyclopentasiloxan, Wasser und beliebigen Kombinationen daraus.

8. Zusammensetzung nach Anspruch 6 oder 7, wobei das Konservierungsmittel aus der aus Folgenden bestehenden Gruppe ausgewählt ist: Tocopherol, Diiodmethyl-p-tolylsulfon, 2-Brom-2-nitropropan-1,3-diol, cis-Isomer von 1-(3-Chlorallyl)-3,5,7-triaza-1-azoniaadaman-tanchlorid, Glutaraldehyd, 4,4-Dimethyloxazolidin, 7-Ethylbicyclooxazolidin, Methylparaben, Sorbinsäure, Germaben II, Rosmarinextrakt, EDTA und einer beliebigen Kombination daraus.

9. Rekombinante Wirtszelle, umfassend ein Polynukleotid, das für ein rekombinantes Kollagenpolypeptid nach einem der Ansprüche 1 bis 3 kodiert.

10. Rekombinante Wirtszelle nach Anspruch 9, wobei die rekombinante Zelle eine Bakterienzelle ist.

11. Rekombinante Wirtszelle nach Anspruch 10, wobei die Bakterienzelle der Spezies Escherichia coli angehört.

12. Rekombinante Wirtszelle nach einem der Ansprüche 9 bis 11, wobei das Polynukleotid eine Nukleotidsequenz gemäß SEQ ID NO: 90 umfasst oder daraus besteht.

13. Rekombinante Wirtszelle nach einem der Ansprüche 9 bis 11, wobei das Polynukleotid für die Expression in der rekombinanten Wirtszelle Codon-optimiert ist.

## Revendications

1. Polypeptide de collagène recombinant qui comprend ou consiste en une séquence d'acides aminés selon SEQ ID NO : 91.

2. Polypeptide de collagène recombinant selon la revendication 1, comprenant en outre un ou plusieurs éléments choisis dans le groupe comprenant : une étiquette de sécrétion, une étiquette d'histidine, une protéine fluorescente, un site de clivage de protéase et une protéine bêta-lactamase.

3. Polypeptide de collagène recombinant selon la revendication 2, dans lequel l'étiquette de sécrétion est DsbA.

4. Composition comprenant entre 0,005 % p/p et 30 % p/p d'un polypeptide de collagène recombinant selon l'une quelconque des revendications précédentes.

5. Composition selon la revendication 4, dans laquelle la composition est formulée pour une application topique.

6. Composition selon la revendication 5, dans laquelle la composition comprend un support topique, un conservateur ou les deux.

7. Composition selon la revendication 6, dans laquelle le support topique est choisi dans le groupe constitué : d'un liposome, d'une microcapsule biodégradable, d'une lotion, d'un spray, d'un aérosol, d'une poudre à saupoudrer, d'un polymère biodégradable, d'une huile minérale, d'une huile de triglycéride, d'une huile de silicone, de glycérine, de monostéarate de glycérine, d'un alcool, d'agents émulsifiants, d'un pétrole liquide, d'un pétrolatum blanc, de propylène glycol, de polyoxyéthylène, de polyoxypropylène, de cire, de monostéarate de sorbitane, de polysorbate, de cire d'ester cétylique, d'alcool cétéarylique, de 2-octyldodécanol, d'alcool benzylique, de cyclométhicone, de cyclopentasiloxane, d'eau, et de toute combinaison de ceux-ci.

8. Composition selon la revendication 6 ou 7, dans laquelle le conservateur est choisi dans le groupe constitué de : tocophérol, diiodométhyl-p-tolylsulfone, 2-bromo-2-nitropropane-1,3-diol, isomère cis de chlorure de 1-(3-chloroallyl)-3,5,7-triaza-1-azoniaadamantane, glutaraldéhyde, 4,4-diméthyloxazolidine, 7-éthylbicyclooxazolidine, méthylparabène, acide sorbique, Germaben II, extrait de romarin, EDTA, et toute combinaison de ceux-ci.

9. Cellule hôte recombinante comprenant un polynucléotide codant pour un polypeptide de collagène recombinant selon l'une quelconque des revendications 1 à 3.

10. Cellule hôte recombinante selon la revendication 9, dans laquelle la cellule recombinante est une cellule bactérienne.

11. Cellule hôte recombinante selon la revendication 10, dans laquelle la cellule bactérienne est de l'espèce *Escherichia coli*.

12. Cellule hôte recombinante selon l'une quelconque des revendications 9 à 11, dans laquelle le polynucléotide comprend ou est constitué d'une séquence nucléotidique selon SEQ ID NO : 90.

13. Cellule hôte recombinante selon l'une quelconque des revendications 9 à 11, dans laquelle le polynucléotide est un codon optimisé pour expression dans la cellule hôte recombinante.
